(19) Europäisches Patentamt
European Patent Office
Office européen des brevets

(11) EP 3 548 501 B1

(12) # EUROPEAN PATENT SPECIFICATION

(45) Date of publication and mention of the grant of the patent:
**17.02.2021 Bulletin 2021/07**

(21) Application number: **17832091.7**

(22) Date of filing: **29.11.2017**

(51) Int Cl.:
***C07F 15/00*** (2006.01) ***B01J 31/22*** (2006.01)
***C07C 67/333*** (2006.01) ***C07C 67/475*** (2006.01)
***B01J 31/28*** (2006.01) ***C07C 1/213*** (2006.01)
***C07C 5/22*** (2006.01) ***C07C 6/04*** (2006.01)
***C07D 313/04*** (2006.01) ***C07D 319/06*** (2006.01)
***C07D 321/12*** (2006.01) ***C07D 239/22*** (2006.01)
***C07D 207/48*** (2006.01) ***C07D 307/06*** (2006.01)

(86) International application number:
**PCT/IB2017/057511**

(87) International publication number:
**WO 2018/100515 (07.06.2018 Gazette 2018/23)**

(54) **NOVEL RUTHENIUM COMPLEX, METHOD OF ITS PRODUCTION AND ITS USE IN REACTION OF OLEFINE METATHESIS**

NEUARTIGER RUTHENIUMKOMPLEX, VERFAHREN ZU DESSEN HERSTELLUNG UND DESSEN VERWENDUNG IN DER REAKTION DER OLEFINMETATHESE

NOUVEAU COMPLEXE DE RUTHÉNIUM, SON PROCÉDÉ DE PRODUCTION ET SON UTILISATION DANS LA RÉACTION DE MÉTATHÈSE D'OLÉFINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2016 PL 41963716**

(43) Date of publication of application:
**09.10.2019 Bulletin 2019/41**

(73) Proprietor: **Uniwersytet Warszawski**
**00-927 Warszawa (PL)**

(72) Inventors:
- **DABROWSKI, Michal**
  **18-200 Wysokie Mazowieckie (PL)**
- **GRELA, Karol**
  **01-471 Warszawa (PL)**

(74) Representative: **Dargiewicz, Joanna et al**
**JD&P Patent Attorneys**
**Joanna Dargiewicz & Partners**
**Ul. Mysliborska 93A/50**
**03-185 Warszawa (PL)**

(56) References cited:
**WO-A1-2016/092424 WO-A1-2018/197963**
**WO-A2-2008/010961**

- **YUKI KOBAYASHI ET AL: "Highly Activated Second-Generation Grubbs-Hoveyda Catalyst Driven by Intramolecular Steric Strain", SYNLETT, vol. 27, no. 16, 13 July 2016 (2016-07-13) , pages 2352-2356, XP055457668, DE ISSN: 0936-5214, DOI: 10.1055/s-0035-1562468**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 548 501 B1

## Description

### Introduction

The invention relates to a novel ruthenium complex, a method for its preparation and use as a catalyst and/or precatalyst in the olefin metathesis reaction. This invention is applicable to broadly understood organic synthesis utilising olefin reactions of Cross-Metathesis (CM), Ring-Closing Metathesis (RCM), Ring-Closing Enyne Metathesis (RCEYM), diastereoselective Ring Rearrangement Metathesis (dRRM), Ring-Opening Metathesis Polymerisation (ROMP) and Acyclic Diene Metathesis (ADMET).

### Background art

A number of ruthenium complexes is known in the art that allow to obtain internal olefins [R. H. Grubbs (Ed.), AG Wenzel (Ed.), D. J. O'Leary (Ed.), E. Khosravi (Ed.), Handbook of Olefin Metathesis, 2nd edition, 3 Volumes, 2015, John Wiley & Sons, Inc. 1608 pages], among which one should note the 1st, 2nd and 3rd generations, and complexes comprising two, identical or different, *N*-heterocyclic carbene ligands (NHCs). In ruthenium complexes, the active, 14-electron catalyst form comprises a neutral ligand that is a phosphine or NHC [Grubbs et al. Chem. Rev. 2010, 110, 1746-1787; Nolan et al. Chem. Commun. 2014, 50, 10355-10375]. The most versatile and effective complexes are those of the 2nd generation - the so-called Grubbs catalysts **(Gru-II),** Hoveydy-Grubbs **(Hov-II)** and Indenylidene (**Ind II**).

NHC ligands having various types, more or less complex steric hindrances and modified electronic properties are known from the literature [L. Benhamou, E. Chardon, Guy Lavigne, S. Bellemin-Laponnaz, V. César, Chem. Rev., 2011, 111, 2705-2733, DOI:10.1021/cr100328e]. In general, one may distinguish NHC imidazole and imidazoline carbene ligands, and some scant reports regarding ruthenium complexes containing NHC ligands with *N*-alkyl substituents can be found in the literature. Accordingly, we focus on ruthenium compounds containing *N*-aryl substituents in NHC ligands.

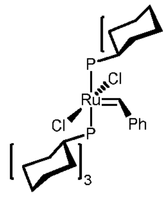
**Gru-I**

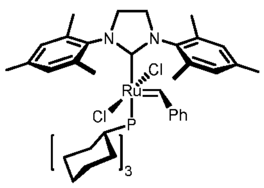
**Gru-II**

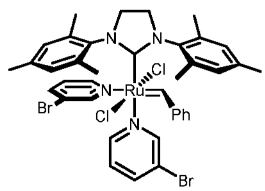
**Gru-III**

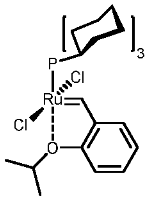
**Hov-I**

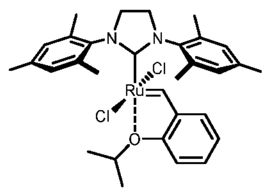
**Hov-II**

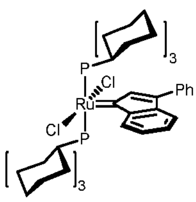
**M1**
**(Ind-I)**

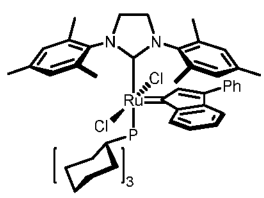
**M2**
**(Ind-II)**

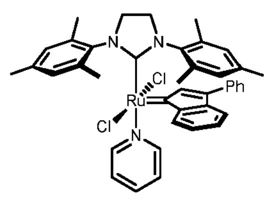
**M31**
**(Ind-III)**

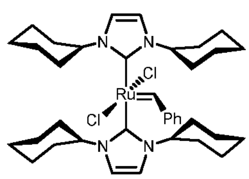
**bis-NHC**
**(Herrmann)**

When listing the least sterically hindered NHC ligands used in ruthenium complexes, one should start with bis-*ortho*-tolyl NHC ligand (such as ruthenium **A** and **B** complexes), used in both **Hov**, **Gru** and **Ind** complexes [(i) Grubbs et al., Org. Lett., 2007, 9, 1589-1592; (ii) Grubbs et al., Org. Lett., 2008, 10, 441-444; (iii) WO2014027040A1; (iv) Grela et al., Chem. Commun., 2013, 49, 3188-3190]. Indeed, these precatalysts are characterised by high activity, rapid initiation of metathesis reaction and high conversions/yields in reactions generating tri- and tetra-substituted C=C double bonds. Among symmetrical NHC ligands, less sterically hindered bis-*N*-phenyl (such as ruthenium **C** and **D** complexes) may be considered; unfortunately, ruthenium complexes containing this type of NHC ligands are rapidly degraded by C-H activation and insertion of Ru to the bond, where non-active in metathesis hydride ruthenium (Ru-H) complexes are generated. Ruthenium complexes containing NHC ligand with bis-*N*-2,6-difluorophenyl (such as ruthenium complex **E**) had very promising chemical properties in olefin metathesis reactions. The **Gru** complex exhibited significant activation versus **Hov**, which was explained by the formation, at the stage of initiation of the **Gru** complex, of intramolecular non-specific interactions between Ru···F atoms, which were observed in **Hov** complexes in solid state [Grubbs et al., J. Am Chem. Soc., 2006, 128, 11768-11769].

The opposite group of symmetrical NHC ligands includes the ligands with a substantial steric hinderence. The

first ligand in the order of the ligands will be the SIPr ligand (such as the ruthenium complex **F**), i.e. containing bis-*N*-2,6-diisopropylphenyl [(i) Mol et al., Adv. Synth. Catal., 2002, 344, 671-677; (ii) Grubbs et al., Organometallics, 2006, 25, 5740-5745]. In literature, SIPr ligand is abundant in **Gru, Hov** and **Ind** complexes, and ligand experiments using a hindered SIPr ligand have sometimes led to unintuitive results *[*Org. Lett., 2008, 10, 441-444]. Another, slightly more sterically crowded metathesis precatalyst is complex **G,** whose structure contains NHC ligand with bis-*N*-3,5-diterbutylphenyl [Grubbs et al., Org. Lett., 2007, 9, 1339-1342]. The **G** complex, despite its steric hinderence, unsubstituted positions 2,6 in the aromatic substituent make it an active catalyst. The last group of symmetrical NHC ligands are ligands having racemic, chiral or meso ligands (such as **H** complexes). These compounds are used for asymmetric olefin metathesis reactions conducted on substrates producing various optical isomers [Grubbs et al., J. Am. Chem. Soc., 2006, 128, 1840-1846].

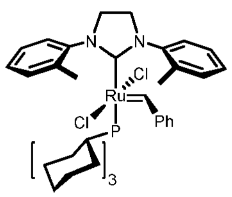
**A**

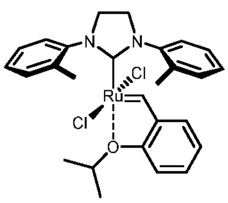
**B**

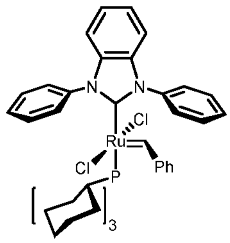
**C**

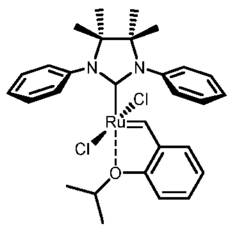
**D**

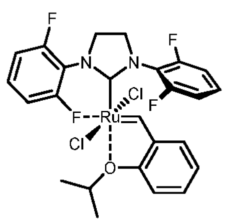
**E**

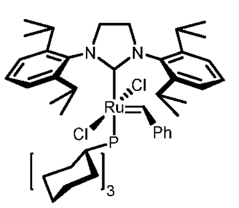
**F**

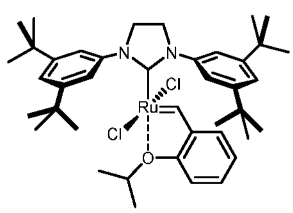
**G**

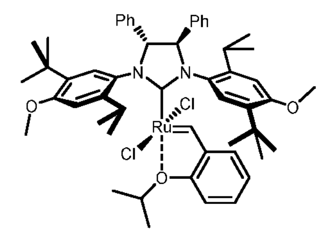
**H**

Another matter in regard of the structure of ruthenium complexes containing NHC ligands are compounds containing unsymmetrical *N,N'*-disubstituted NHC ligands. The literature regarding this topic is very abundant in terms of the number of new structures. Yet, in regards to exhaustive investigation of the potential uses of ruthenium complexes containing such unsymmetrical NHC ligands, literature doesn't offer much, as experiments that tested their usability are usually limited to simple RCM and CM reactions. Unsymmetrical NHC ligands containing two *N*-aryl substituents, such as **I** complex, were obtained by the Grubbs' team [Chem. Eur. J., 2008, 14, 7545-7556]. Other ruthenium complexes containing unsymmetrical NHC *N*-aryl-*N*-alkyl substituted ligands are e.g. **J** and **K** complexes [(i) Verpoort et al, Chem. Eur. J., 2006, 12, 4654-4661; (ii) Verpoort et al., Adv. Synth. Catal., 2007, 349, 1692-1700]. An interesting extension of this type of ruthenium complexes includes structures such as **M,** proposed by Mauduit et al. [ACS Catalysis, 2016, 6, 7970-7976]. These complexes contain a NHC ligand with one *N*-aryl, and one *N*-cycloalkyl substituents with varied ring sizes. Another modification of the NHC ligands in metathesis precatalysts was the synthesis of ligands containing one *N*-aryl, and one *N*-aralkyl or *N*-heteroaralkyl substituents. Catalysts of this type (having ligands such as in precatalysts N and O) exhibited promising properties in difficult olefin metathesis reactions (i) Grela et al., Organometallics, 2012, 31, 7316-7319; (ii) Grela et al., Organometallics, 2014, 33, 2160-2171; (iii) WO2016092424A1; (iv) Grela et al., RSC Adv., 2016, 6, 77013-77019].

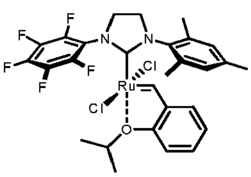
**I**

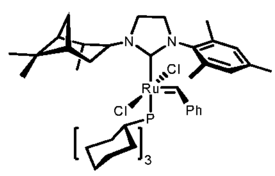
**J**

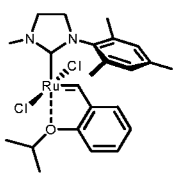
**K**

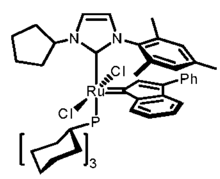
**M**

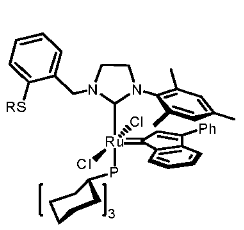 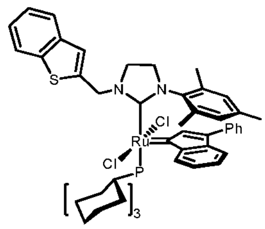 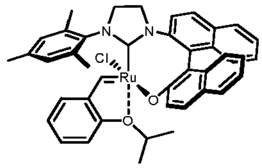

N O P

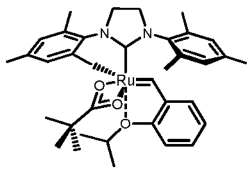 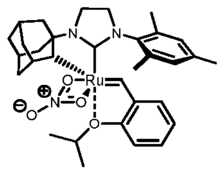

R S

The last group of olefin metathesis precatalysts containing unsymmetrical NHC ligands are bidentate complexes. The first report on the preparation and use of said complex in asymmetric olefin metathesis reactions was published by Hoveydy et al. [J. Am. Chem. Soc., 2002, 124, 4954-4955]. **P** ruthenium complexes, despite their low activity, exhibited high effectiveness in asymmetric reactions. The last group of unsymmetrical bidentate NHC ligands used in ruthenium complexes are **R** and **S** complexes containing adamantyl (or other) substituent that have been generated by C-H activation and insertion of Ru to this bond [(i) Grubbs et al., J. Am. Chem. Soc., 2011, 133, 8525-8527; (ii) Grubbs et al., J. Am. Chem. Soc., 2012, 134, 693-699; (iii) Grubbs et al., Organometallics 2015, 34, 2858-2869].

WO 2016/092424 A1 discloses ruthenium-imidazolidinylidene complexes having in their structure indenylidene alkylidene, bearing two neutral NHC carbene ligands lacking phosphine ligand type, or Hoveyda type having one NHC ligand and chelating benzylidene typical for olefin metathesis precatalyst, that exhibits a significantly higher thermal stability both in solution as well as in the solid state, and at the same time effectively catalyze metathesis reactions at the elevated temperatures. Additionally, these catalysts are activated in a slow and controlled manner at high temperatures, as compared to commercially available ruthenium complexes, thus combining features of the "latent catalyst" and high stability features of II-generation catalysts.

A paper by Y. Kobayashi et al. entitled Highly Activated Second-Generation Grubbs-Hoveyda Catalyst Driven by Intramolecular Steric Strain (Synlett, vol. 27, no. 16, 13 July 2016, pp. 2352-2356) discloses various Grubbs-Hoveyda second-generation catalysts, in particular a ruthenium-carbene metathesis catalyst, which features a phenanthrenyl-substituent. It also discloses the use of the 9-phenanthrenyl-substituent as a means of increasing the steric hindrance around the catalytic centrum.

WO 2008/010961 A2 related to the synthesis of terminal alkenes from internal alkenes using a cross-metathesis reaction catalyzed by a selected metathesis catalyst. In particular it discloses a ruthenium metathesis catalyst featuring a phenanthrenyl-substituent and the use of such complexes for catalysis of olefin metathesis reactions.

The complicated and costly synthesis pathways of ruthenium complexes containing NHC ligands that require the use of expensive reagents such as silver salts, strong bases, dry and deoxygenated solvents, impede the synthesis of ruthenium complexes and broad use of olefin metathesis in industry. Moreover, there are few known complexes that are durable and resistant to exposure to high temperatures, and few precatalysts whose activation to catalysts can be controlled during the reaction under high temperature conditions. Furthermore, controlling the activity and absence of isomerisation of the C=C double bond during (or after) the metathesis reaction under high temperature conditions is an important aspect of that issue.

Study of new applications of olefin metathesis reactions in industry is an important reason behind the continuous search for new ruthenium complexes with modified catalytic properties. These would be, in particular, ruthenium complexes with more universal catalytic properties in a broad spectrum of reaction conditions and high tolerance of functional groups of substrates.

**Solution to the problem**

Surprisingly, it has been found that olefin metathesis precatalysts and/or catalysts of the invention exhibit reduced activity in the C=C double bond isomerization side reaction relative to commercially available ruthenium complexes, which is a significant problem when conducting the cross-metathesis reaction at high temperatures (> 50°C). Olefin metathesis precatalysts and/or catalysts of the invention effectively catalyse medium size ring closure in the RCM

process (cyclic olefins having the ring size of 7, 8) relative to commercially available complexes, in particular substrates susceptible to migration of the double bond. Olefin metathesis precatalysts and/or catalysts of the invention catalyze at high yields preparation of chemical compounds through the process of biomass ethenolysis (e.g. ethyl oleate) that are desirable in the industry. In addition, olefin metathesis precatalysts and/or catalysts of the invention allow for obtaining previously unavailable chiral building blocks through the ethenolysis of naturally occuring terpenoids (exemplified by caryophyllene).

**Disclosure of the essence of the invention**

Thus, the present invention relates to a compound of the structure represented by formula 9

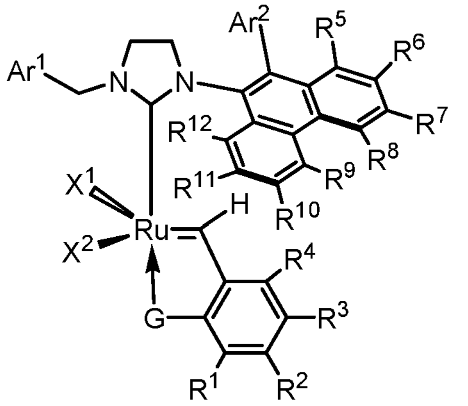

**9**

wherein:

$X^1$ and $X^2$ are each an anionic ligand; $X^1$ and $X^2$ may be interconnected to form a cyclic system;

G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;

$Ar^1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which are optionally substituted with hydrogen atoms or optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-SO$_2$NR'''$_2$), a sulfonamide group (-NR'''SO$_2$R'''), a sulpho group (-SO$_2$R'''), a sulphonium (-S$^+$R'''$_2$), a phosphonium group (-P(O)(OR''')$_2$), a phosphinium group (-P(O)R'''(OR''')), a phosphonous group (-P(OR''')$_2$), a phosphine group (-PR'''$_2$), a phosphine group (-P$^+$R'''$_3$) a nitro group (-NO$_2$), a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-N$^+$R'''$_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system.

Preferably, the compound of the present invention has the structure represented by formula **9a**

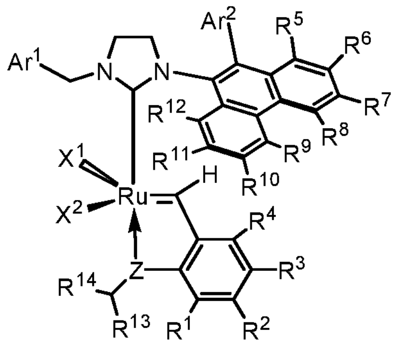

**9a**

wherein, $X^1$ and $X^2$ are each a halogen atom;

Z is O, S, NR' or PR', wherein groups R' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy;

$Ar_1$ and $Ar_2$ are each a $C_5$-$C_{20}$ aryland/or a $C_4$-$C_{20}$ heteroaryl group, which are substituted by hydrogen atoms, or optionally are substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl groups, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group ($-SO_2NR'''_2$), a sulpho group ($-SO_2R'''$), a sulphonium ($-S+R'''_2$), a phosphonium group ($-P(O)(OR''')_2$), a phosphinium group (-P(O)R'''(OR''')), a phosphonous group ($-P(OR''')_2$), a phosphine group ($-PR'''_2$), a nitro group ($-NO_2$), a nitroso group (-NO), a carboxy group (-COOH), an ester group (-COOR'''), a formyl group (-CHO), a ketone group, (-COR'''), an amide ($-CONR'''_2$), an imido (-CONR'''COR'''), an amino ($-NR'''_2$), an ammonium ($-N^+R'''_3$), an amido (-NR'''COR''') groups, in which groups R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

$R^{13}$ and $R^{14}$, are each a hydrogen atom or a $C_1$-$C_{25}$ alkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_5$-$C_{20}$ heteroaryloxy group, or which may be interconnected to form a substituted or non-substituted $C_4$-$C_{10}$ cyclic or $C_4$-$C_{12}$ polycyclic systems, it may also be an ester (-COOR'), amide ($-CONR'_2$), formyl (-CHO), ketone (-COR'), hydroxamic (-CON(OR')(R')) groups or a halogen atom, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy, or a halogen atom.

Preferably, the compound of the present invention has the structure represented by formula **9b**

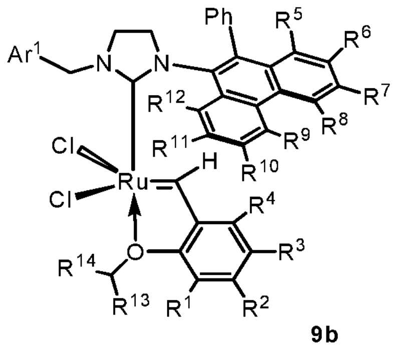

**9b**

wherein, $Ar_1$ is each a $C_5$-$C_{20}$ aryl, or a $C_4$-$C_{20}$ heteroaryl group, which is substituted by hydrogen atoms, or optionally is substituted by at least one a $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom; $R^1$, $R^2$, $R^3$, $R^4$ are each a hydrogen atom, an alkoxy group (-OR'), a sulfidic group (-SR'), a cyano group (-CN), a sulfoxide group (-S(O)R'), a sulfonamide group ($-SO_2NR'_2$), a sulpho group ($-SO_2R$), a sulphonium (-S+R'2), a phosphonium group (-P(O)(OR')2), a phosphinium group (-P(O)R'(OR')), a phosphonous group ($-P(OR')_2$), a phosphine group ($-PR'_2$), a nitro group ($-NO_2$), a nitroso group (-NO), a carboxy group (-COOH), an ester group (-COOR'), a formyl group (-CHO), a ketone group, (-COR'), an amide ($-CONR'_2$), an imido (-CONR'COR'), an amino ($-NR'_2$), an ammonium ($-N^+R'_3$), an amido (-NR'COR') groups, in which groups R' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

$R^{13}$ and $R^{14}$, are each a hydrogen atom or a $C_1$-$C_{25}$ alkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_5$-$C_{20}$ heteroaryloxy group, or which may be interconnected to form a substituted or non-substituted $C_4$-$C_{10}$ cyclic or $C_4$-$C_{12}$ polycyclic systems, it may also be an ester ($-COOR^a$), amide ($-CONR^a_2$), formyl (-CHO), ketone ($-COR^a$), hydroxamic ($-CON(OR^a)(R^a)$) groups or a halogen atom, wherein $R^a$ is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy, or a halogen atom.

Preferably, the compound of the present invention has the structure represented by formula **Ru-3**

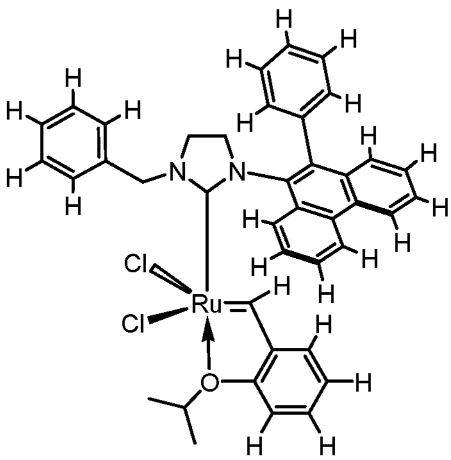

**Ru-3**

The present invention also relates to a method for preparing a compound of formula **9**

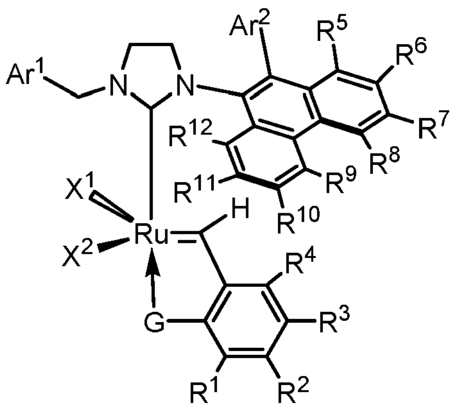

**9**

wherein:

$X^1$ and $X^2$ are each an anionic ligand; $X^1$ and $X^2$ may be interconnected to form a cyclic system;
G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;
$Ar^1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;
$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-$SO_2$NR'''$_2$), a sulfonamide group (-NR'''$SO_2$R'''), a sulpho group (-$SO_2$R'''), a sulphonium (-$S^+$R'''$_2$), a phosphonium group (-P(O)(OR''')$_2$), a phosphinium group (-P(O)R'''(OR''')), a phosphonous group (-P(OR''')$_2$), a phosphine group (-PR'''$_2$), a phosphine group (-$P^+$R'''$_3$) a nitro group (-$NO_2$), a a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-$N^+$R'''$_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system;
$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,
characterised in that the alkylidene ruthenium complex of formula **10**

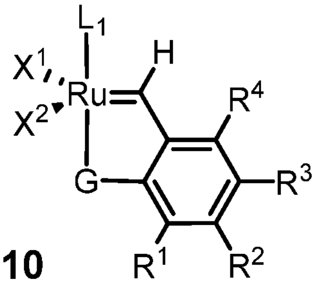

**10**

wherein:

$L^1$ are neutral ligands selected from the group comprising pyridine or substituted pyridine, $P(R^b)_3$, $P(OR^b)_3$, $O(R^b)_2$, $N(R^b)_3$, wherein $R^b$ is each a $C_1$-$C_{12}$ alkyl, a $C_3$-$C_{12}$ cycloalkyl, a $C_5$-$C_{20}$ aryl, a $C_7$-$C_{24}$ aralkyl, a $C_5$-$C_{24}$ perfluoroaryl, 5-12 membered heteroaryl;

$X^1$ and $X^2$ are each an anionic ligand; $X^1$ and $X^2$ may be interconnected to form a cyclic system;

G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide ($-CONR'_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group ($-SO_2NR'''_2$), a sulpho group ($-SO_2R'''$), a sulphonium (-S+R'''2), a phosphonium group ($-P(O)(OR''')_2$), a phosphinium group (-P(O)R'''(OR''')), a phosphonous group ($-P(OR''')_2$), a phosphine group ($-PR'''_2$), a nitro group ($-NO_2$), a nitroso group (-NO), a carboxy group (-COOH), an ester group (-COOR'''), a formyl group (-CHO), a ketone group, (-COR'''), an amide ($-CONR'''_2$), an imido (-CONR'''COR'''), an amino ($-NR'''_2$), an ammonium ($-N^+R'''_3$), an amido (-NR'''COR''') groups, in which groups R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, $C_5$-$C_{20}$ heteroaryloxy, and wherein $R^1$, $R^2$, $R^3$ and $R^4$ may be interconnected to form a cyclic system,

is reacted with carbene of formula **8aa**

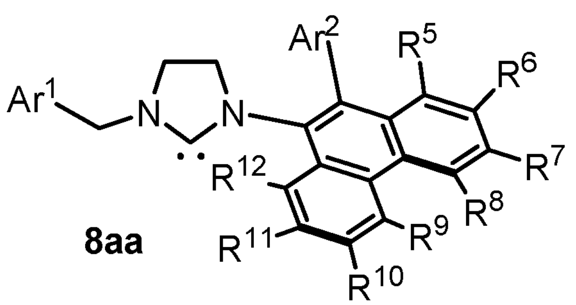

**8aa**

wherein:

$Ar_1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which are optionally substituted with hydrogen atoms or optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system.

Preferably, the alkylidene ruthenium complex of formula **10**

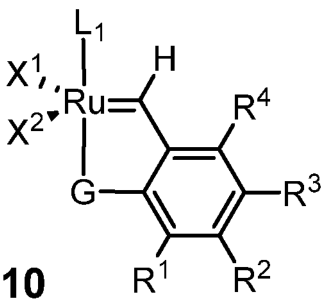

**10**

wherein:

$L^1$ are neutral ligands selected from the group comprising pyridine or substituted pyridine, $P(R^b)_3$, $P(OR^b)_3$, $O(R^b)_2$, $N(R^b)_3$, wherein $R^b$ is each a $C_1$-$C_{12}$ alkyl, a $C_3$-$C_{12}$ cycloalkyl, a $C_5$-$C_{20}$ aryl, a $C_7$-$C_{24}$ aralkyl, a $C_5$-$C_{24}$ perfluoroaryl, a 5-12 membered heteroaryl;

$X^1$ and $X^2$ are each an anionic ligand; $X^1$ and $X^2$ may be interconnected to form a cyclic system;

G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide ($-CONR'_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group ($-SO_2NR'''_2$), a sulpho group ($-SO_2R'''$), a sulphonium ($-S+R'''_2$), a phosphonium group ($-P(O)(OR''')_2$), a phosphinium group (-P(O)R'''(OR''')), a phosphonous group ($-P(OR''')_2$), a phosphine group ($-PR'''_2$), a nitro group ($-NO_2$), a nitroso group (-NO), a carboxy group (-COOH), an ester group (-COOR'''), a formyl group (-CHO), a ketone group, (-COR'''), an amide ($-CONR'''_2$), an imido (-CONR'''COR'''), an amino ($-NR'''_2$), an ammonium ($-N^+R'''_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,

is reacted with carbene produced *in situ* as a result of the effect of a base selected from those such as potassium *tert*-amylate, potassium *tert*-butoxide, potassium *N*,*N'*-bis (trimethylsilyl)amide, sodium hydride, on a carbene precursor of formula **8a**

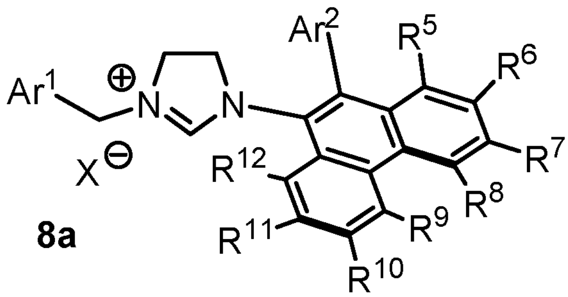

8a

wherein:

$Ar_1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system;

$X^-$ is a halide anion, or $BF_4^-$, $PF_6^-$, $ClO_4^-$.

Preferably, carbides of formula **8aa** are provided to the reaction medium by thermally generating them *in situ* from suitable carbene precursors of formula **8b** that are adducts with chloroform, carbon dioxide or alcohol,

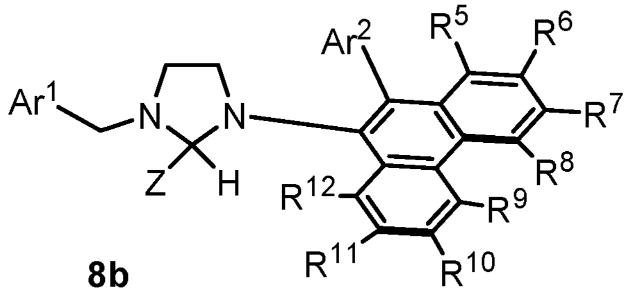

8b

wherein:

Z is $CCl_3$, $CO_2$ or OR', wherein R' is a $C_1$-$C_{12}$ alkyl, a $C_3$-$C_{12}$ cycloalkyl, a $C_2$-$C_{12}$ alkenyl, or a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy or a halogen atom;

$Ar_1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

Preferably, the alkylidene ruthenium complex of formula **10** is contacted with a compound of formula **8c,** which acts as a NHC carbene ligand donor of formula **8aa,**

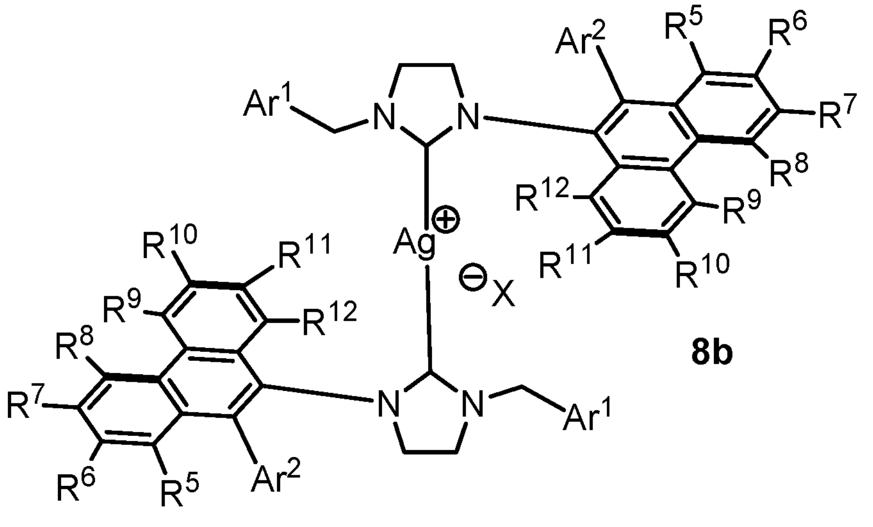

**8b**

wherein:

$Ar_1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system;

$X^-$ is a halide anion, or $BF_4^-$, $PF_6^-$, $ClO_4^-$.

The present invention also relates to the use of a compound of formula **9** as a precatalyst and/or catalyst in olefin metathesis reactions.

Preferably, the compound of formula **9** is used as a precatalyst and/or catalyst in the reactions of ring-closing metathesis (RCM), homomethatesis, cross-metathesis (CM), ethenolysis, isomerisation, in the diastereoselective ring rearrangement metathesis (DRRM) reaction, "alken-alkyn" (en-yn) metathesis or ROMP type polymerisation reactions.

Preferably, the compound of formula **9** is used as a precatalyst and/or catalyst in the ring-opening metathetic polymerisation (ROMP) reaction of dicyclopentadiene or norbornene.

Preferably, the compound of formula **9** is used as a precatalyst and/or catalyst in the reaction mixture for a period between 1 minute and 24 hours, and/or the reaction is conducted in non-polar solvents or without a solvent, and/or the reaction is conducted in an organic solvent such as toluene, benzene, mesitylene, dichloromethane, ethyl acetate, methyl acetate, tertbutyl methyl ether, cyclopentylmethyl ether, or with no solvent, and/or the reaction is conducted at a temperature of from 20 to 150°C.

Preferably, compound **9** is used in an amount of less than 1 mol%, and/or the compound **9** is added to the reaction mixture in portions and/or continuously using a pump, ans/or the compound **9** is added to the reaction mixture as a solid and/or as a solution in an organic solvent, and/or at least one olefin is added to the reaction mixture in portions and/or continuously using a pump.

Preferably, the gaseous by-product of the reaction (ethylene, propylene, butylene) is actively removed from the reaction mixture using an inert gas or vacuum.

Preferably, the compound of formula **9** is used as a substrate for the synthesis of other ruthenium complex compounds that are precatalysts and/or catalysts of olefin metathesis.

BRIEF DESCRIPTION OF FIGURES

The invention will be presented in greater detail in a preferred embodiment, with reference to the accompanying drawing, in which:

Fig. 1 shows the summary of olefin metathesis precatalysts and/or catalysts used according to the present invention.

Fig. 2 is the structure of the **Ru-3** compound obtained based on X-ray structural analysis.

Fig. 3 illustrates reaction profiles of the ring closing of diethyl diallylmalonate (DEDAM) in the RCM reaction catalysed by selected ruthenium complexes at 1 mol% at the temperature of 40°C in $CD_2Cl_2$ under argon.

Fig. 4 illustrates reaction profiles of the ring closing of diethyl diallylmalonate (DEDAM) in the RCM reaction catalysed by selected ruthenium complexes at 0.1 mol% at the temperature of 40°C in $CD_2Cl_2$ under argon.

Fig. 5 illustrates reaction profiles of the ring closing of diethyl allylmethylallylmalonate (DEAMAM) in the RCM reaction catalysed by selected ruthenium complexes at 1 mol% at the temperature of 40°C in $CD_2Cl_2$ under argon.

Fig. 6 illustrates reaction profiles of the ring closing of diethyl diallyltosylamide (DATA) in the RCM reaction catalysed by selected ruthenium complexes at 0.1 mol% at the temperature of 40°C in $CD_2Cl_2$ under argon.

Fig. 7 is a kinetic profile of the stability of selected precatalysts in $CD_2Cl_2$ under argon over time.

Fig. 8 is a kinetic profile of the stability (initiation speed) of selected precatalysts in $CD_2Cl_2$ under ethylene over time that reflects the decomposition of a 14-electron ruthenium olefin metathesis reaction catalyst.

Fig. 9a shows ethenolysis results of ethyl oleate under 10 bar of ethylene dynamic pressure (99.9% purity) at the temperature of 50°C for 3 hours.

Fig. 9b shows ethenolysis results of caryophyllene under various reaction conditions.

Fig. 10 is a kinetic profile of 1-octene homomethatesis reaction, and the accompanying isomerisation reaction, without solvent, at the temperature of 80°C, 500 ppm **Ru-3** (see Fig. 10a) or **Ru-1** (see Fig. 10b).

## Terms

The terms used in the present description have the meanings as follows. Non-defined terms in this document have the meaning given and understood by a person skilled in the art in the light of the best knowledge held, of the present disclosure, and of the context of the description of the patent application. Unless it is indicated otherwise, the following conventional chemistry terms are used the present description that have the meanings as defined below.

The term *"halogen atom"* or *"halogen"* refers to an element selected from F, Cl, Br, I.

The term *"carbene"* refers to a particle containing an neutral carbon atom with a valence number of two and having two unpaired (triplet state) or paired (singlet state) valence electrons. The term *"carbene"* also includes carbene analogs in which the carbon atom is substituted by another chemical element such as boron, silicon, germanium, tin, lead, nitrogen, phosphorus, sulphur, selenium and tellurium.

The term *"alkyl"* refers to a saturated, linear or branched hydrocarbon substituent having the indicated number of carbon atoms. Examples of alkyl substituents include -methyl, -ethyl, -*n*-propyl, -*n*-butyl, -*n*-pentyl, -*n*-hexyl, -*n*-heptyl, -*n*- octyl, *n*-nonyl, and -*n*-decyl. Representative branched -($C_1$-$C_{10}$)alkyls include -isopropyl, -sec-butyl, -isobutyl, -tert-butyl, -isopentyl, -neopentyl, -1-methylobutyl, -2-methylobutyl, -3-methylobutyl, -1,1-dimethylopropyl, -1,2-dimethylopropyl, -1-methylopentyl, -2-methylopentyl, -3-methylopentyl, -4-methylopentyl, -1-ethylobutyl, -2-ethylobutyl, -3-ethylobutyl, -1,1-dimethylobutyl, -1,2-dimethylobutyl, 1,3-dimethylobutyl, -2,2-dimethylobutyl, -2,3-dimethylobutyl, -3,3-dimethylobutyl, -1-methylohexyl, 2-methylohexyl, -3-methylohexyl, -4-methylohexyl, -5-methylohexyl, -1,2-dimethylopentyl, - 1,3-dimethylopentyl, -1,2-dimethylohexyl, -1,3-dimethylohexyl, -3,3-dimethylohexyl, 1,2-dimethyloheptyl, - 1,3-dimethyloheptyl, -3,3-dimethyloheptyl and the like.

The term *"alkoxy"* refers to an alkyl substituent as defined above bound by an oxygen atom.

The term *"perfluoroalkyl"* refers to an alkyl group as defined above in which all the hydrogen atoms have been substituted by the same or different halogen atoms.

The term *"cycloalkyl"* refers to a saturated mono- or polycyclic hydrocarbon substituent having the indicated number of carbon atoms. Examples of cycloalkyl substituents include -cyclopropyl, -cyclobutyl, - cyclopentyl, -cyclohexyl, -cycloheptyl, -cyclooctyl, -cyclononyl, -cyclodecyl and the like.

The term *"alkenyl"* refers to a saturated, linear or branched non-cyclic hydrocarbon substituent of the indicated number of carbon atoms and containing at least one double carbon-carbon bond. Examples of alkenyl substituents include -vinyl, -allyl, -1-butenyl, -2-butenyl, -isobutenyl, -1-pentenyl, -2-pentenyl, -3-methylo-1-butenyl, -2-methylo-2-butenyl, -2,3-dimethylo-2-butenyl, -1-hexenyl, -2-hexenyl, -3-hexenyl, -1-heptenyl, -2-heptenyl, -3-heptenyl, -1-octenyl, -2-octenyl, -3-octenyl, -1-nonenyl, -2-nonenyl, -3-nonenyl, - 1-decenyl, -2-decenyl, -3-decenyl and the like.

The term *"cycloalkenyl"* refers to a non-saturated mono- or polycyclic hydrocarbon substituent of the indicated number of carbon atoms and containing at least one double carbon-carbon bond. Examples of cycloalkenyl substituents include -cyclopentenyl, -cyclopentadienyl, -cyclohexenyl, -cyclohexadienyl, - cycloheptenyl, -cycloheptadienyl, -cycloheptatrienyl, -cyclooctenyl, -cyclooctadienyl, -cyclooctatrienyl, - cyclooctatetraenyl, -cyclononenyl, -cyclopentadienyl, -cyclodecenyl, -cyclodecadienyl and the like.

The term *"alkynyl"* refers to a saturated, linear or branched non-cyclic hydrocarbon substituent of the indicated number of carbon atoms and containing at least one triple carbon-carbon bond. Examples of alkynyl substituents include -acetylenyl, -propynyl, -1-butynyl, -2-butynyl, -1-pentynyl, -2-pentynyl, -3-methyl-1-butynyl, 4-pentynyl, -1-hexynyl, 2-hexynyl, -5-hexynyl and the like.

The term *"cycloalkynyl"* refers to a non-saturated mono- or polycyclic hydrocarbon substituent of the indicated number of carbon atoms and containing at least one triple carbon-carbon bond. Examples of cycloalkynyl substituents include -cyclohexnyl, -cycloheptynyl, -cyclooctynyl and the like.

The term *"aryl"* refers to an aromatic mono- or polycyclic hydrocarbon substituent having the indicated number of carbon atoms. Examples of aryl substituents include -phenyl, -tolyl, -xylyl, -naphthyl, -2,4,6-trimethylphenyl, -2-fluorophenyl, -4-fluorophenyl, -2,4,6-trifluorophenyl, -2,6-difluorophenyl, -4-nitrophenyl and the like.

The term *"aralkyl"* refers to an alkyl substituent as defined above substituted with at least one aryl as defined above. Examples of aralkyl substituents include -benzyl, -diphenylmethyl, -triphenylmethyl and the like.

The term *"heteroaryl"* refers to an aromatic mono- or polycyclic hydrocarbon substituent having the indicated number of carbon atoms, in which at least one carbon atom is substituted by a heteroatom selected from O, N and S atoms. Examples of heteroaryl substituents include -furyl, -thienyl, -imidazolyl, - oxazolyl, -thiazolyl, -isoxazolyl, -triazolyl, -oxadiazolyl, -thiadiazolyl, -tetrazolyl, -pyridyl, -pyrimidyl, - triazinyl, -indolyl, -benzo[b]furyl, -benzo[b]thienyl, -indazolyl, -benzoimidazolyl, -azaindolyl, -quinolyl, - isoquinolyl, -carbazolyl and the like.

The term *"heterocycle"* refers to a saturated or partially non-saturated, mono- or polycyclic hydrocarbon substituent having the indicated number of carbon atoms, in which at least one carbon atom is substituted by a heteroatom selected from O, N and S atoms. Examples of heterocyclic substituents include furyl, thiophenyl, pyrrolyl, oxazolyl, imidazolyl, thiazolyl, isoxazolyl, pyrazolyl, isothiazolyl, triazinyl, pyrrolidinonyl, pyrrolidinyl, hydantoinyl, oxiranyl, oxetanyl, tetrahydrofuranyl, tetrahydrothiophenyl, quinolinyl, isoquinolinyl, chromonyl, coumarinyl, indolyl, indolizinyl, benzo[b]furanyl, benzo[b]thiophenyl, indazolyl, purinyl, 4*H*-quinolizinyl, isoquinolyl, quinolyl, phthalazinyl, naphthyridinyl, carbazolyl, β-carbolinyl and the like.

The term *"neutral ligand"* refers to a non-charged substituent capable of coordinating with a metallic centre (the ruthenium atom). Examples of such ligands may include: amines, phosphines and oxides thereof, alkyl and aryl phosphites and phosphates, arsines and oxides thereof, ethers, alkyl and aryl sulphides, coordinated hydrocarbons, alkyl and aryl halides.

The term "anionic ligand" refers to a substituent capable of coordinating with a metallic centre (the ruthenium atom) with a charge capable of partially or completely compensating the charge of the metallic centre. Examples of such ligands may include fluoride, chloride, bromide, iodide, cyanide, cyanate and thiocyanate anions, carboxylic acid anions, alcohol anions, phenolic anions, thiol and thiophenol anions, delocalised charge hydrocarbon anions (e.g. cyclopentadiene), (organo)sulphuric and (organo)phosphoric acid anions and esters thereof (such as, for example, alkylsulphonic and aryl sulphonic acid anions, alkylphosphoric and arylphosphoric acid anions, sulphuric acid alkyl and aryl ester anions, phosphoric acid alkyl and aryl ester anions, alkylphosphoric and arylphosphoric alkyl and aryl ester anions). Optionally, the anionic ligand may have interconnected $L^1$, $L^2$ and $L^3$ groups, such as the catechol anion, the acetylacetone anion, the salicylaldehyde anion. Anionic ligands ($X^1$, $X^2$) and neutral ligands ($L^1$, $L^2$, $L^3$) may be interconnected to form multidentate ligands, such as a bidentate ligand ($X^1$-$X^2$), a tridentate ligand ($X^1$-$X^2$-$L^1$), a tetradentate ligand ($X^1$-$X^2$-$L^1$-$L^2$), a bidentate ligand ($X^1$-$L^1$), a tridentate ligand ($X^1$-$L^1$-$L^2$), a tetradentate ligand ($X^1$-$L^1$-$L^2$-$L^3$), a bidentate ligand ($L^1$-$L^2$), a tridentate ligand ($L^1$-$L^2$-$L^3$). Examples of such ligands include catechol anion, acetylacetone anion and salicylaldehyde anion.

The term *"heteroatom"* refers to an atom selected from the group comprising an atom of oxygen, sulphur, nitrogen, phosphorus and the like.

The term *"chlorinated solvent"* refers to a solvent, the structure of which comprises at least one atom of for example fluorine, chlorine, bromine and iodine; preferably more than one. Examples of such solvents include dichloromethane, chloroform, tetrachloromethane (carbon tetrachloride), 1,2-dichloroethane, chlorobenzene, perfluorobenzene, perfluorotoluene, freons and the like.

The term *"organic non-polar solvent"* refers to a solvent characterised by non-existent or very low dipole momentum. Examples of such solvents include pentane, hexane, octane, nonane, decane, benzene, toluene, xylene and the like.

The term *"organic polar solvent"* refers to a solvent characterised by a dipole momentum substantially greater

than zero. Examples of such solvents include dimethylformamide (DMF), tetrahydrofuran (THF) and its derivatives, diethyl ether, dichloromethane, ethyl acetate, chloroform, alcohols (MeOH, EtOH or *i*-PrOH) and the like.

The term "*GC*" refers to gas chromatography.

The term *"HPLC"* refers to high performance liquid chromatography, and solvents designated as "HPLC" solvents refer to solvents having sufficient purity for HPLC analysis.

The term *"NMR"* refers to nuclear magnetic resonance.

The term "*NHC*" refers to *N*-heterocyclic carbene.

The term *"DEDAM"* refers to diethyl diallylmalonate.

The term *"DEAMAM"* refers to diethyl allylmethylallylmalonate.

The term *"DATA"* refers to diallyltosylamide.

The term *"precatalyst'* refers to, in relation to ruthenium complexes, a 16-electron chemical compound which, after the step of dissociation of one ligand or reorganisation of the molecule, is converted to the 14-electron olefin metathesis catalyst as such, which is active in the catalytic cycle.

## Embodiments of the invention

The following examples are provided solely for the purpose of illustrating the invention and for clarifying the individual aspects thereof, and not with the intention to limit it, and should not be considered to be equivalent to the total scope thereof as defined in the appended claims. In the examples below, unless otherwise indicated, standard materials and methods were employed as used in the art or it was proceeded according to the manufacturer's recommendations for particular reagents and methods.

### Example I

Preparation of 9-bromo-10-nitrophenanthrene

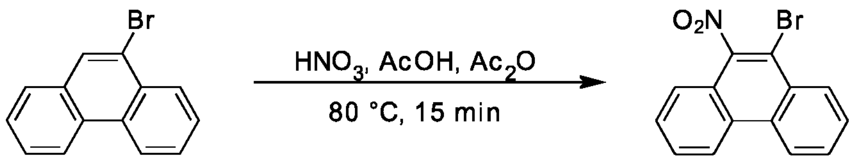


Modified reaction procedure: Ma et al., Org. Lett. 2003 5, 3317-3319, DOI: 10.1021/01035147k

To a vigorously stirred, hot (~ 80°C) solution of 9-bromophenanthrene (20.06 g, 0.075 mol) in a mixture of glacial acetic acid (22 mL, 0.38 mol) and acetic anhydride (8.2 mL, 0.086 mol), nitric acid (25 mL, 0.353 mol) was slowly added dropwise. An orange-yellow solid precipitated. The mixture was heated for another 15 minutes and then poured into cold water. Subsequently, in order to neutralise the nitric acid, a saturated solution of $NaHCO_3$ was added. The mixture was filtered off, and the precipitate was washed with water until the filtrate became colourless. The precipitate was air dried and then recrystallised from acetone/methanol mixture. A product was obtained in the form of a dark yellow solid (10.6 g, 47%).

Hint: In the next step, an incompletely purified product (over 70% purity) can be used. This facilitates the synthesis due to the low yield of the completely pure compound (~13% after several crystallisations). The product obtained according to the above procedure has a purity of more than 75% (up to ~90%).

$^1$H NMR (400 MHz, $CDCl_3$): $\delta$ = 8.64-8.71 (m, 2H), 8.45-8.40 (m, 1H), 7.73-7.83 (m, 3H). 7.61-7.71 (m, 2H).

$^{13}$C NMR (101 MHz, $CDCl_3$): $\delta$ = 130.7, 130.2, 129.5, 129.4, 128.9, 128.8, 128.6, 123.7, 123.2, 123.1, 122.4, 113.2.

HRMS was calculated for $C_{14}H_9BrNO_2$ $[M+H]^+$: 301.9811; measured: 301.9825, difference (ppm): 4.64.

Elemental analysis: calculated for $C_{14}H_8BrNO_2$: C 55.66; H 2.67; Br 26.45; N 4.64; found: C 55.85; H 2.71; Br 26.51; N 4.58

### Example II

Preparation of 9-nitro-10-phenylphenanthrene

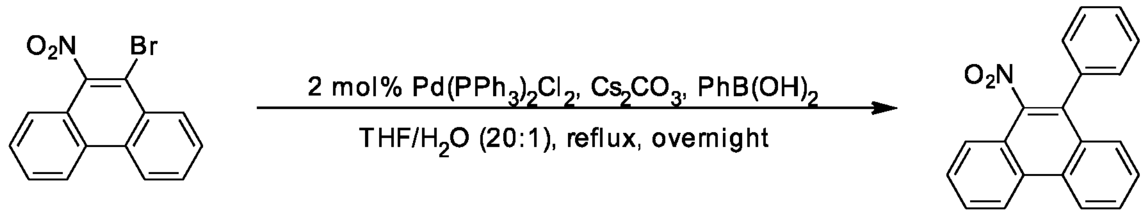


2 3

The following were sequentially placed in a 25 mL round-bottomed flask: $Pd(PPh_3)_2Cl_2$ (11.6 mg, 2 mol%, 0.02 eq.), $Cs_2CO_3$ (539 mg, 1.65 mmol, 2 eq.), $PhB(OH)_2$ (156 mg, 1.24 mmol, 1.5 eq.) i 9-bromo-10-nitrophenanthrene (250 mg, 0.83 mmol, 1 eq.). The flask with the solid content was placed under reduced pressure and then filled with argon, that step was repeated three times, subsequently a solvent (10 mL degassed THF and 0.6 mL degassed distilled water) was added to the flask. The reaction mixture was heated at reflux overnight.

The resulting dark reaction mixture was cooled to room temperature and THF was evaporated. A solid precipitated. Subsequently, the solid was filtered off, air dried and dissolved in the smallest possible volume of $CH_2Cl_2$. The solution was filtered through a silica pad, washing with 5% EtOAc in c-hexane. The filtrate was evaporated to give a yellow solid which was recrystallised from the mixture of $CH_2Cl_2$/heptane by means of a slow exchange of the solvent on a rotary evaporator to give a yellow microcrystalline solid (239 mg, 96%).

Hint: Since the purification of 9-bromo-10-nitrophenanthrene in crystallisation is cumbersome and inefficient, the second synthesis step can be carried out using a crude, untreated nitration product (if the mixture contains more than 70% of 9-bromo-10-nitrophenanthrene). In this case, the products of Suzuki coupling with other isomers are oiling out from the reaction mixture.

In a large-scale reaction $Pd(PPh_3)_2Cl_2$ (162.6 mg, 2 mol%, 0.02 eq.), $Cs_2CO_3$ (7.63 g, 23.17 mmol, 2 eq.), $PhB(OH)_2$ (2.18 g, 17.38 mmol, 1,5 eq.) and 9-bromo-10-nitrophenanthrene (3.5 g with 75% purity, 11.58 mmol, 1 eq.) were weighed in 125 mL degassed THF and 7 mL degassed and distilled water) with the same reaction procedure being followed. Purification: the reaction mixture was extracted with $CH_2Cl_2$, and after drying the solution with magnesium sulphate, SnatchCat (4.4 eq. to remove palladium) was used and subsequently filtered through a silica layer. After filtration and crystallisation, 2.42 g of a crystalline solid was obtained (which represents 70% of the yield, based on the mixture mass, 94% yield based on the substrate).

$^1H$ NMR (400 MHz, $CDCl_3$): $\delta$ = 8.83-8.76 (m, 2H), 7.83-7.70 (m, 4H), 7.64-7.50 (m, 5H), 7.46-7.42 (m, 2H).

$^{13}C$ NMR (101 MHz, $CDCl_3$): $\delta$ = 147.3, 133.9, 130.6, 130.5, 130.4, 130.2, 130.1, 129.0, 128.8, 128.7, 128.6, 128.5, 128.4, 127.8, 123.1, 123.0, 122.9, 122.8.

IR (diamond tip): ν = 3061, 6028, 2881, 1642, 1520, 1490, 1441, 1377, 1238 $cm^{-1}$.

HRMS was calculated for $C_{20}H_{13}NNaO_2$ $[M+Na]^+$: 322.0839; found: 322.0850, difference (ppm): 3.42.

Melting point: 213.7-214.6 °C.

Elemental analysis: calculated for $C_{20}H_{13}NO_2$: C, 80.25; H, 4.38; N, 4.68. Found: C, 79.98; H, 4.35; N, 4.67.

**Example III**

Preparation of 10-phenylphenanthrene-9-amine

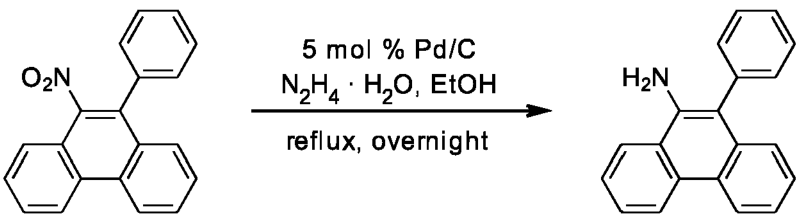


Modified preparation procedure according to Org. Synth. 1960, 40, 5, DOI:10.15227/orgsyn.040.0005 Reaction proceed without argon atmosphere on air

In a 500 mL round bottom flask 9-nitro-10-phenylphenanthrene (3 g, 10.02 mmol, 1 eq.) was dissolved in 200 mL ethanol. The solution was heated to 50°C. Pd/C (513 mg, 482 mmol, 5 mol%) was added to the hot mixture, and subsequently $N_2H_4 \cdot H_2O$ (2 mL, 41.07 mmol, 4 eq.) was added portionwise. The mixture was refluxed for 16 hours, filtered through Celite, and then evaporated. The crude residue was dissolved in a small volume (approx. 20 mL) of hot

methanol and allowed to slowly cool to room temperature, after which the mixture was placed in a refrigerator overnight. Next day, white needles of the product were filtered off and dried under reduced pressure (2.62 g, 96%).
$^1$H NMR (400 MHz, $CDCl_3$): δ = 8.79-8.74 (m, 1H),, 8.66-8.62 (m, 1H), 7.98-7.94 (m, 1H), 7.72-7.63 (m, 2H), 7.61-7.55 (m, 2H), 7.52-7.33 (m, 5H), 7.29-7.25 (m, 1H), 3.98 (s, 2H, $NH_2$).
$^{13}$C NMR (101 MHz, $CDCl_3$): δ = 137.9, 136.7, 133.2, 131.3, 130.8, 129.5, 127.8, 126.8, 126.7, 125.9, 125.3, 125.2, 123.4, 123.2, 122.5, 121.7, 118.3.
IR (diamond tip): ν = 3348, 3312, 3065, 3023, 1621, 1587, 1492, 1440, 1422, 1395, 1330 $cm^{-1}$.

HRMS was calculated for $C_{20}H_{16}N[M+H]^+$: 270.1277; found: 270.1285, difference (ppm): 2.96.

Elemental analysis: calculated for $C_{20}H_{15}N$: C, 89.19; H, 5.61; N, 5.20; found: C, 89.38; H, 5.64; N, 5.08.

**Example IV**

Preparation of 2-chloro-*N*-(10-phenyl-9-phenanthryl)acetamide

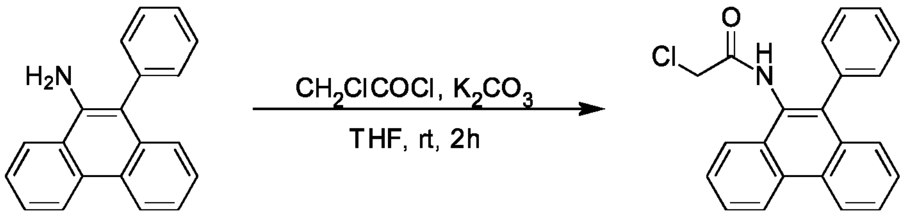


Modified procedure: Powell et al., Org. Lett. 2004 6, 4069-4072, DOI: 10.1021/ol048235t
To a suspension of $K_2CO_3$ (1.95 g, 13.96 mmol, 2.5 eq.) in 100 mL of THF 10-phenylphenanthrene-9-amine (1.5 g, 5.57 mmol, 1 eq.) was added, and the resulting mixture was stirred for 15 minutes. Then, chloroacetyl chloride (500 μL, 6.15 mmol, 1.1 eq.) was added dropwise. The progress of the reaction was monitored by TLC. At the end of the reaction (about 1-2 h), the reaction mixture was filtered through a pad of celite, washed with THF and evaporated. The solid residue was dissolved in $CH_2Cl_2$/hexane and filtered through a silica gel layer (washing with 15% EtOAc in hexane). The filtrate was evaporated, dissolved in a minimum volume of $CH_2Cl_2$, heptane was added, and the product was crystallised by slow exchange of the solvent on a rotary evaporator to give 1.82 g of a white solid (95%).

Hint: the amine is luminous blue under the UV lamp. If after 1-2 hours the solution is luminous under UV light, add more chloroacetyl chloride in small portions until the colour under UV is no longer visible.
$^1$H NMR (400 MHz, $CDCl_3$): δ = 8.82 - 8.74 (m, 2H), 8.00 - 7.93 (m, 1H), 7.90 (s, 1H, NH), 7.77 - 7.63 (m, 3H), 7.58 - 7.44 (m, 5H), 7.39 - 7.29 (m, 2H), 4.04 (s, 2H, $CH_2Cl$).
$^{13}$C NMR (101 MHz, $CDCl_3$): δ = 166.0, 137.1, 136.8, 131.6, 130.9, 130.3, 129.6, 128.7, 128.7, 128.1, 128.0, 127.8, 127.4, 127.3, 127.2, 126.9, 124.0, 123.1, 122.7, 42.6.
IR (diamond tip): ν = 3238, 3065, 3032, 1683, 1663, 1564, 1532, 1489, 1444, 1431, 1327, 1311, 1248 $cm^{-1}$.

HRMS was calculated for $C_{22}H_{17}ClNO [M+H]^+$: 346.0993; found 346.0994, difference (ppm): 0.29.

Melting point: 223.5-223.7 °C.

Elemental analysis: calculated for $C_{22}H_{16}ClNO$: C 76.41; H 4.66; Cl 10.25; N 4.05; found: C 76.30; H 4.72; Cl 10.31; N 3.94

**Example V**

Preparation of *N*$^2$-benzvl-*N*$^1$-(10-phenyl-9-phenanthryl)glycynamide

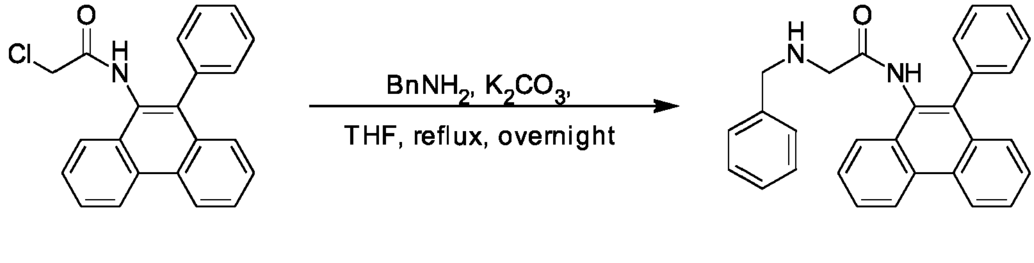

To a hot, vigorously stirred solution of 2-chloro-*N*-(10-phenyl-9-phenantryl)acetamide (1.1 g, 3.03 mmol, 1 eq.) and $K_2CO_3$ (1 g, 7.22 mmol, 2.3 eq.) in THF, benzylamine (0.67 mL, 6.06 mmol, 2 eq.) was added. The mixture was refluxed for 16 hours. After cooling it to room temperature, the mixture was filtered through Celite and evaporated. The resulting oil was purified by chromatography (35% EtOAc in hexane) to give 1.08 g of a white solid (86% yield). $^1$H NMR (400 MHz, $CDCl_3$): δ = 8.90 (s, 1H, NHCO), 8.80 - 8.74 (m, 2H), 7.97 - 7.91 (m, 1H), 7.75 - 7.59 (m, 3H), 7.51 - 7.27 (m, 10H), 7.18 - 7.11 (m, 2H), 3.38 (s, 2H, $CH_2NHCO$), 3.35 (s, 2H, $CH_2NH_2$), 1.70 (bs, 2H, $NH_2$).
$^{13}$C NMR (101 MHz, $CDCl_3$): δ = 171.5, 139.1, 138.0, 136.2, 131.9, 130.9, 130.1, 129.8, 129.3, 129.2, 128.6, 128.6, 128.1, 127.8, 127.6, 127.4, 127.2, 127.1, 126.8, 126.7, 124.6, 122.9, 122.7, 53.4, 51.8.
IR (diamond tip): ν = 3332, 3061, 2877, 2829, 1677, 1594, 1476, 1449, 1424, 1377, 1363, 1324 cm$^{-1}$.

HRMS was calculated for $C_{29}H_{25}N_2O$ [M+H]$^+$: 417.1961; found 417.1954, difference (ppm): 1.68

Melting point: 139.7-140.1 °C.

Elemental analysis: calculated for $C_{29}H_{24}N_2O$: C, 83.63; H, 5.81; N, 6.73; found: C, 83.56; H, 5.74; N, 6.60.

**Example VI**

Preparation of *N*-benzyl-*N'*-(10-phenyl-9-phenanthryl)ethylene-1,2-diamine

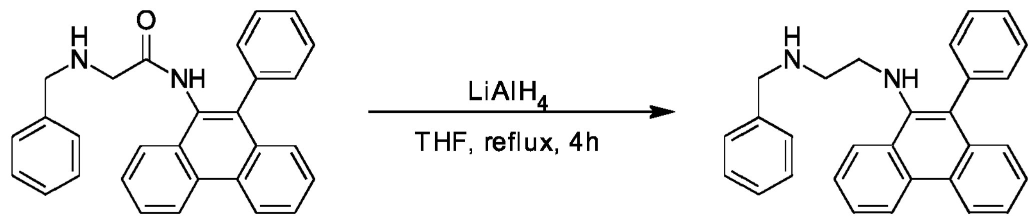


To a round bottom flask containing 1 g *N*$^2$-benzyl-*N*$^1$-(10-phenyl-9-phenanthryl)glycinamide (1 eq., 2.4 mmol) 60 mL of dry THF was added. The solution was cooled to 0°C and 549 mg LAH (6 eq., 14.5 mmol) was slowly added portionwise. The vigorously stirred suspension was warmed to room temperature and subsequently kept at boiling point temperature for 4 hours, the reaction progress was monitored by TLC. At the end of the reaction, the mixture was diluted with $Et_2O$ and cooled to 0°C. Then, 0.6 mL of water, 0.6 mL of 15% NaOH and a further 1.8 mL of water were slowly added dropwise. The mixture was warmed to room temperature, $MgSO_4$ was added, and it was stirred for 15 minutes. The mixture was stirred for another 15 minutes and then filtered through a pad of celite, evaporated to dryness and washed with $Et_2O$ to give 0.96 g of a white solid (99% yield) which was used in the subsequent steps of synthesis without further purification.
$^1$H NMR (400 MHz, $CDCl_3$): δ = 8.76 - 8.71 (m, 1H), 8.67 (ddt, J = 8.3, 1.3, 0.6 Hz, 1H), 8.37 - 8.28 (m, 1H), 7.74-7.57 (m, 2H), 7.56-7.21 (m, 11H), 7.25-7.10 (m, 2H), 3.94 (s, 1H), 3.65 (s, 2H), 3.17 (dd, J = 6.5, 5.1 Hz, 2H), 2.75 - 2.67 (m, 2H), 1.57 (s, 2H).
$^{13}$C NMR (101 MHz, $CDCl_3$): δ = 141.1, 140.4, 137.8, 132.9, 131.4, 130.9, 130.9, 129.2, 129.2, 128.4, 128.1, 127.9, 127.9, 127.7, 127.5, 127.0, 126.7, 126.5, 125.1, 124.6, 124.6, 123.1, 122.5, 122.5, 53.6, 50.2, 49.4.
IR (diamond tip): ν = 3298, 3025, 2948, 2855, 1587, 1450, 1379, 1325, 1234, 1110, 1091 cm$^{-1}$.

HRMS was calculated for $C_{29}H_{27}N_2$ [M+H]$^+$ : 403.2169; found 403.2164, difference (ppm): 1.24.

Elemental analysis: calculated for $C_{29}H_{26}N_2$: C, 86.53; H, 6.51; N, 6.96; found: C, 86.70; H, 6.57; N, 6.84.

**Example VII**

Preparation of 3-benzyl-1-(10-phenyl-9-phenanthryl)-4,5-dihydro-1*H*-imidazol-3-yl chloride

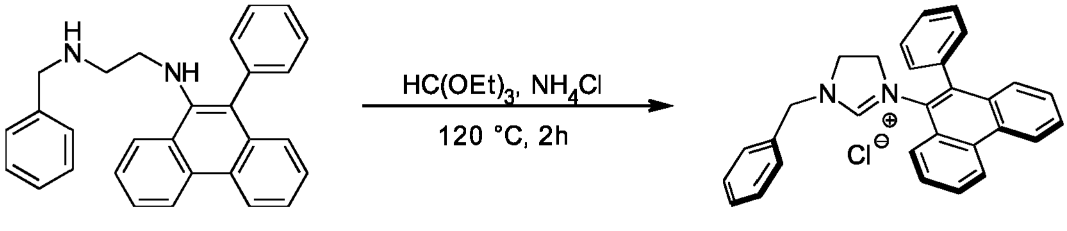

To a flask containing *N*-benzyl-*N'*-(10-phenyl-9-phenanthryl)ethan-1,2-diamine (1.21 g, 3.0 mmol, 1 eq.) and ammonium chloride (0.35 g, 6.6 mmol, 2.2 eq.), triethyl orthoformate (10.2 mL, 60 mmol, 20 eq.) was added. The mixture was heated to 120°C under gentle argon flow (to remove released ethanol). After 3 hours, the mixture was cooled to room temperature, and then $Et_2O$ was added. The precipitate was filtered off and washed three times with $Et_2O$. The precipitate was dried under reduced pressure. 1.32 g of the expected product (yield 98%) was obtained.
$^1$H NMR (400 MHz, $CDCl_3$): $\delta$ = 10.28 (s, 1H), 8.73 (dd, J = 15.5, 8.1 Hz, 2H), 8.29 (d, J = 7.7 Hz, 1H), 7.89 (d, J = 7.5 Hz, 1H), 7.77 (tdd, J = 8.3, 7.0, 1.1 Hz, 2H), 7.70 - 7.41 (m, 6H), 7.34 (dd, J = 4.9, 1.8 Hz, 3H), 7.21 (d, J = 7.4 Hz, 1H), 7.09 (dd, J = 6.6, 2.9 Hz, 2H), 5.58 (d, J = 14.6 Hz, 1H), 4.57 (d, J = 14.6 Hz, 1H), 4.49 (ddd, J = 12.3, 10.8, 7.4 Hz, 1H), 4.17 (q, J = 11.5, 11.1 Hz, 1H), 3.61 (q, J = 11.0, 10.4 Hz, 1H), 3.44 (td, J = 11.7, 7.4 Hz, 1H).
$^{13}$C NMR (101 MHz, $CDCl_3$): $\delta$ = 160.2, 138.3, 135.5, 132.9, 132.0, 131.2, 130.8, 130.8, 130.0, 128.9, 128.8, 128.8, 128.7, 128.6, 128.5, 128.5, 128.4, 128.4, 128.3, 128.0, 127.4, 127.2, 124.0, 122.9, 122.8, 52.5, 52.0, 48.0.
IR (diamond tip): ν = 3026, 2856, 1642, 1626, 1491, 1449, 1378, 1361, 1324, 1272, 1249, 1233, 1210, 1174, 1116, 1083, 1029 $cm^{-1}$.

HRMS was calculated for $C_{30}H_{25}N_2$ $[M-Cl]^+$ : 413.2012; found 413.2007, difference (ppm): 1.21.

Elemental analysis: calculated for $C_{30}H_{25}ClN_2$: C 80.25; H 5.61; Cl 7.90; N 6.24; found: C, 80.01; H, 5.66; Cl, 7.85; N, 6.19.

**Example VIII**

Preparation of dichloro(3-benzvl-1-(1 0-phenyl-9-phenanthryl))-2-imidazolidinvlidene)(*o*-isopropoxyphenylmethylene)ruthenium

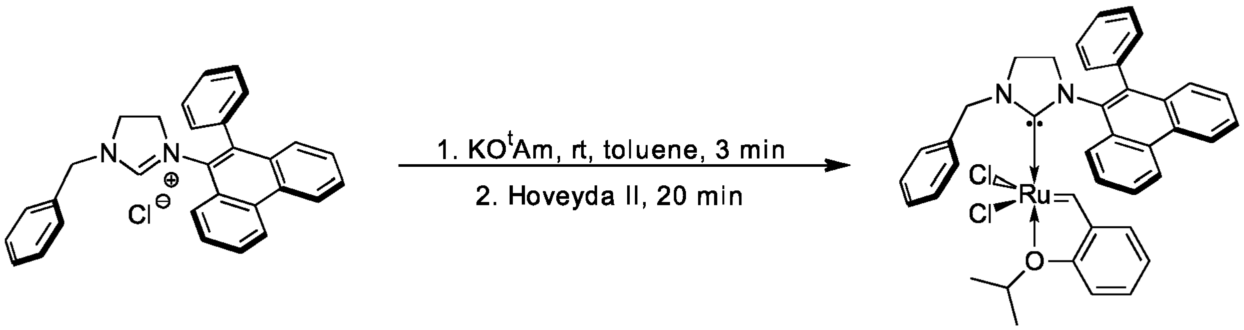


213.4 mg imidazolinium salt (0.47 mmol, 1.1 eq.) was placed in a Schlenk vessel and dried under reduced pressure at the temperature of 70°C for 30 minutes. The reaction vessel was then cooled to room temperature and toluene (20 mL) was added. 0.3 mL 25% KO$^t$Am (1.09 eq.) was added to the resulting suspension, and then, after a clear solution was formed (after about 1 minute), 259 mg of **Hov-I** complex (0.43 mmol, 1 eq.) was added, and the Schlenk vessel was placed in a heated oil bath. The progress of the reaction was monitored by TLC. After approx. 15 minutes, the Schlenk vessel with the reaction mixture was placed in an ice bath. After 5 minutes, 20 mL of n-hexane was added, and the mixture was purified by chromatography using eluent 0→10→20% EtOAc/hexane. The green fraction was collected and, after evaporation, the solid was recrystallised from the $CH_2Cl_2$/MeOH mixture to give 166 mg as a brown-green solid (52% yield).
$^1$H NMR (400 MHz, $CDCl_3$): $\delta$ = 16.60 (s, 1H), 8.92 (d, J = 8.3 Hz, 1H), 8.80 (d, J = 8.3 Hz, 1H), 8.13 (d, J = 8.2 Hz, 1H), 7.79 (dt, J = 16.0, 8.1 Hz, 3H), 7.71 - 7.62 (m, 3H), 7.56 (dt, J = 16.7, 7.5 Hz, 3H), 7.44 (t, J = 6.6 Hz, 4H), 7.40 - 7.29 (m, 3H), 6.86 (d, J = 8.3 Hz, 1H), 6.64 (t, J = 7.5 Hz, 1H), 6.24 (d, J = 7.5 Hz, 1H), 5.81 - 5.50 (m, 2H), 5.21 - 5.04 (m, 1H), 3.80 (q, J = 11.2, 9.9 Hz, 1H), 3.47 - 3.33 (m, 2H), 3.10 (q, J = 12.8, 11.1 Hz, 1H), 1.75 (d, J = 6.1 Hz, 6H).
$^{13}$C NMR (101 MHz, $CDCl_3$): $\delta$ = 289.8, 210.6, 152.8, 143.4, 138.2, 136.2, 135.9, 135.0, 133.3, 131.8, 130.9, 130.7, 129.8, 129.7, 129.5, 129.3, 129.2, 128.8, 128.5, 128.3, 127.9, 127.9, 127.8, 127.4, 127.1, 125.8, 122.8, 122.5, 122.3, 122.3, 112.8, 75.3, 56.2, 53.0, 47.6, 22.3, 22.2.
IR (diamond tip): ν = 3059, 2987, 2889, 1587, 1572, 1472, 1436, 1419, 1381, 1263, 1214, 1110 $cm^{-1}$.

HRMS was calculated for $C_{40}H_{35}N_2ORu$ $[M-HCl-Cl]^+$: 661.1798; found 661.1795, difference (ppm): 0.45.

Melting point: 230.5°C (decomposition).

Elemental analysis: calculated for $C_{40}H_{36}Cl_2N_2ORu$: C 65.57; H 4.95; Cl 9.68; N 3.82; found: C, 65.53; H, 5.06; Cl, 9.66; N, 3.85.

**Example IX**

RCM reaction profiles closing DEDAM, DEAMAM and DATA

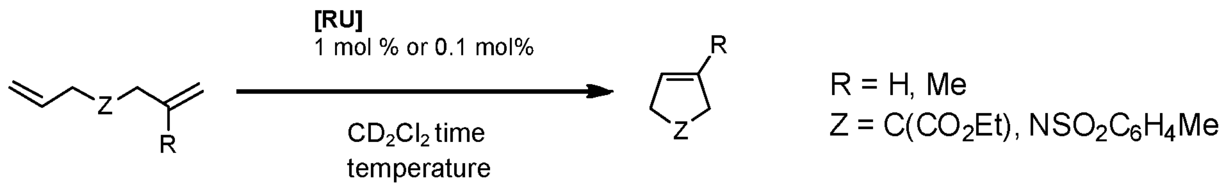


General procedure for preparing RCM reaction profiles monitored by $^1$H-NMR.

All stock solutions and the test samples were prepared in a glove box under inert gas (argon). The diene stock solution was prepared as follows: 1.166 mol of diene was weighed into a 10 mL volumetric flask. Then distilled, degassed dry $CD_2Cl_2$ was added to a volume of 10 mL. The solution was mixed.

600 μL of the diene stock solution was placed in a NMR tube with Young's tap. The stock solution of the precatalyst was prepared by weighing 7 μmol of the complex in a 1 mL volumetric flask and dissolving it in distilled, degassed dry $CD_2Cl_2$ to a volume of 1 mL. After mixing, the stock solution of the precatalyst (100 μL, 0.7 μmol) was added to the NMR tube, mixed and placed in an NMR device.

Measurement points were recorded during the reaction using Agilent's software (vnmj) "Array" function. The conversion was calculated by comparing the integration ratio of the methylene proton signals of the substrate and those of the product in line with the following equation:

$$Conversion(\%) = \frac{[P] \times 100\%}{[P] + [S]}$$

where:

[P] → integration of the methylene proton signal of the product;
[S] → integration of the methylene proton signal of the substrate;

**Example X**

Diastereoselective Ring Rearrangement Metathesis (dRRM) reactions

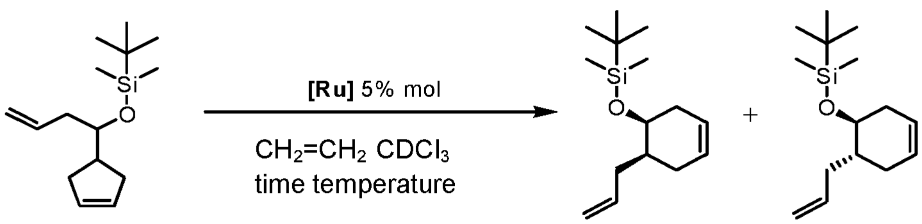


500 μL of durene solution and 100 μL of substrate solution in $CDCl_3$ were inserted to an NMR tube equipped with a rubber septum (from which air was previously removed and which was filled with argon, the operation was repeated three times). The NMR tube was cooled to the temperature of 0°C, and ethylene gas was passed through the solution through a long needle over 5 minutes. Subsequently, the tube was shaken, re-cooled and the ethylene bubbling was repeated. The NMR tube was warmed to room temperature and the initial (P0) $^1$H-NMR spectrum was recorded. Subsequently, 100 μL of the precatalyst solution (5 mol%) was injected, the tube was shaken and thermostated at the temperature indicated in table 1. After the reaction time indicated in table 1, $^1$H-NMR spectrum (isomer ratio) was recorded, then the reaction was quenched (SnatchCat, 5 mg/mL, and then 500 μL $CH_2Cl_2$ to full volume of a 1.5 mL vial), after which GC analysis was conducted (simultaneously monitoring the conversion and *trans*/*cis* isomer ratio).

***Table 1. Summary of dRRM reaction results***

| Time [h] | Temperature [°C] | Precatalyst [Ru] | Conversion[1] | Isomer ratio[2] |
|---|---|---|---|---|
| 1 | 50 | **Ru-3** | >99% | 1.52:1 |
| | | **Ru-1** | >99% | 1.13:1 |
| | | **Ru-2** | 91% | 3.37:1 |
| 10 | 50 | **Ru-3** | >99% | 1.52:1 |
| | | **Ru-1** | >99% | 1.16:1 |
| | | **Ru-2** | >99% | 4.10:1 |
| 17 | 22 | **Ru-3** | >99% | 1.24:1 |
| | | **Ru-1** | >99% | 1.20:1 |
| | | **Ru-2** | 96% | 3.88:1 |

Determined by GC using an internal standard -durene (1,2,4,5--tetramethylbenzene).
[2]Determined by $^1$H-NMR and GC, *trans*/*cis* ratio

**Example XI**

Cross-metathesis (CM) reaction of allylbenzene and (*Z*)-1,4-diacetoxy-2-butendiol

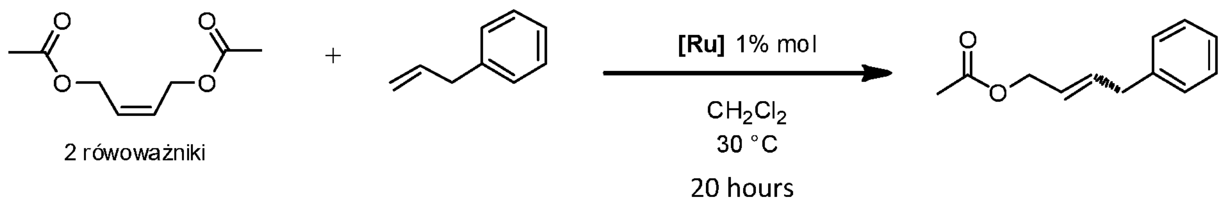


mLTo a solution of allylbenzene (97.9 mg, 0.812 mmol, 110 mL) and cis-1,4-diacetoxy-2-butendiol (297 mg, 1.638 mmol, 275 mL) in methylene chloride (7 mL) the precatalyst solution (1 mol%, 8.12 μmol) in 1 mL of methylene chloride was added. The resulting reaction mixture was stirred at the temperature of 30°C for 20 hours. Then, the solution was evaporated, and the crude residue was purified by column chromatography (10% ethyl acetate in hexane). The product was obtained as a colourless oil (yields and isomer ratios are summarised in Table 2). The reaction was conducted according to the procedure [Organometallics, 2006, 25, 5740-5745].

***Table 2. Summary of CM reaction results***

| Item | Precatalyst [Ru] | Yield [%] | *E*/*Z* ratio |
|---|---|---|---|
| 1 | **Ru-1** | 87 | 10.9:1 |
| 2 | **Ru-2** | 88 | 10.1:1 |
| 3 | **Ru-3** | 91 | 9.9:1 |

**Example XII**

Reaction of ring-closing metathesis in alkenyne reaction

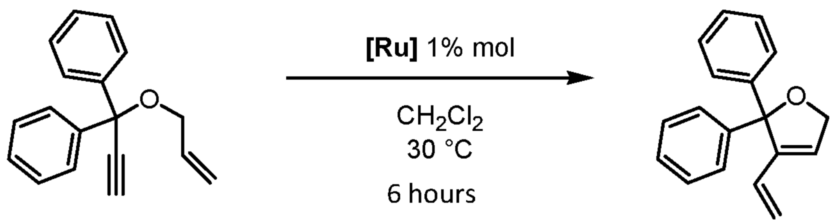


To a solution of alkenyne substrate (205.71 mg, 0.828 mmol) in dry $CH_2Cl_2$ (7 mL) the solution of the appropriate precatalyst (1 mol%) in dry $CH_2Cl_2$ (1 mL) was added. The reaction mixture was stirred at 30°C. At the end of the reaction (TLC monitoring, 6 hours) the solvent was evaporated, and the crude reaction product was purified by column chromatography (hexane/ethyl acetate, 39:1) to give the product as a colourless oil (yields are summarised in Table 3).

***Table 3. Summary of the alkenyne reaction results***

| Item | Precatalyst [Ru] | Yield [%] |
|---|---|---|
| 1 | **Ru-1** | 97 |
| 2 | **Ru-2** | 97 |
| 3 | **Ru-3** | 98 |

**Example XIII**

Preparative-scale RCM reactions

mLTo a thermostated (at a temperature of 40°C) substrate solution (0.4 mmol) in methylene chloride (3.5 mL) the solution of the precatalyst (0.5 mol%, RCM) in methylene chloride (0.5 mL) was added. After 6 hours, the reaction was quenched with a 10 mg/mL SnatchCat solution (0.5 mL per 0.5 mol% of the precatalyst). The mixture was evaporated on silica gel, and the product-containing powder was purified by chromatography using an automated Combiflash® system.

***Table 4. Results of preparative-scale RCM reactions***

| Item | Substrate | Product | Precatalyst [Ru] | Yield [%] (Conversion [%]) |
|---|---|---|---|---|
| 1 | 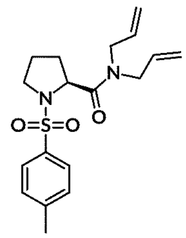 | 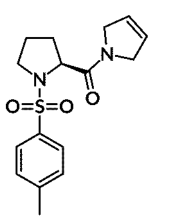 | **Ru-1**<br>**Ru-2**<br>**Ru-3** | 99<br>89<br>99 |
| 2 | 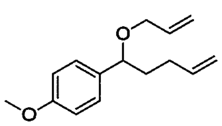 | 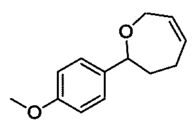 | **Ru-1**<br>**Ru-2**<br>**Ru-3** | 49<br>75<br>80 |
| 3 | 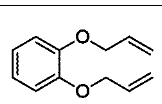 | 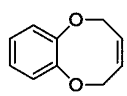 | **Ru-1**<br>**Ru-2**<br>**Ru-3** | 53<br>-<br>82 |
| 4 | 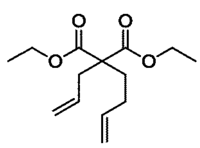 | 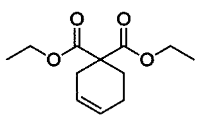 | **Ru-1**<br>**Ru-2**<br>**Ru-3** | 99<br>99<br>99 |

(continued)

| Item | Substrate | Product | Precatalyst [Ru] | Yield [%] (Conversion [%]) |
|---|---|---|---|---|
| 5 | 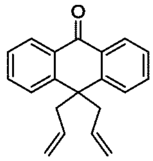 | 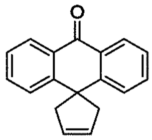 | **Ru-1**<br>**Ru-2**<br>**Ru-3** | 95<br>95<br>95 |
| 6 | 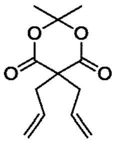 | 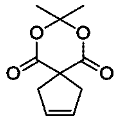 | **Ru-1**<br>**Ru-2**<br>**Ru-3** | 98<br>-<br>70 |
| 7 | 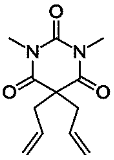 | 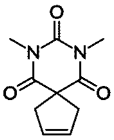 | **Ru-1**<br>**Ru-2**<br>**Ru-3** | 99<br>4<br>56 |
| 8 | 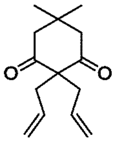 | 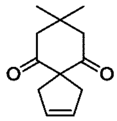 | **Ru-1**<br>**Ru-2**<br>**Ru-3** | 96<br>2<br>22 |
| 9 | 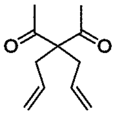 | 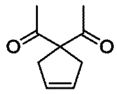 | **Ru-1**<br>**Ru-2**<br>**Ru-3** | 95<br>-<br>92 |
| 10 | 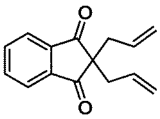 | 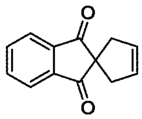 | **Ru-1**<br>**Ru-2**<br>**Ru-3** | (96)<br>(0)<br>(5) |
| 11 | 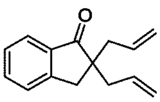 | 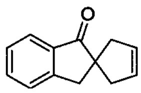 | **Ru-1**<br>**Ru-2**<br>**Ru-3** | (93)<br>(0)<br>(4) |

**Example XIV**

Preparative-scale CM reactions

mLTo a thermostated (at a temperature of 40°C) substrate solution (0.8 mmol) in methylene chloride (7 mL) the solution of the precatalyst (1 mol%, CM) in methylene chloride (1 mL) was added. After 20 hours, the reaction was quenched with a 10 mg/mL SnatchCat solution (1 mL per 0.5 mol% of the precatalyst). The mixture was evaporated on silica gel, and the product-containing powder was purified by chromatography using an automated Combiflash® system.

***Table 5. Results of preparative-scale CM reactions***

| Item | Substrates | Product | [Ru] | Yield [%] | *E*/*Z ratio* |
|---|---|---|---|---|---|
| 1 | 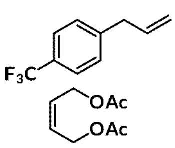 | 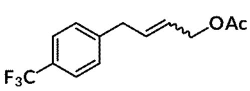 | **Ru-1**<br>**Ru-2**<br>**Ru-3** | 84<br>75<br>85 | 9:1<br>6.5:1<br>8.6:1 |

(continued)

| Item | Substrates | Product | [Ru] | Yield [%] | *E/Z ratio* |
|---|---|---|---|---|---|
| 2 | 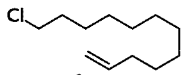 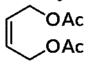 | 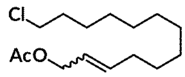 | **Ru-1**<br>**Ru-2**<br>**Ru-3** | 86<br>87<br>87 | 8:1<br>6.3:1<br>7.5:1 |
| 3 | 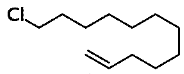 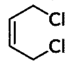 | 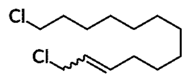 | **Ru-1**<br>**Ru-2**<br>**Ru-3** | 81<br>75<br>89 | 7.32:1<br>7.19:1<br>7.26:1 |
| 4 | 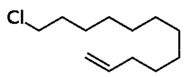 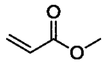 | 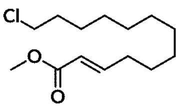 | **Ru-1**<br>**Ru-2**<br>**Ru-3** | 95<br>-<br>87 | only E |
| 5 | 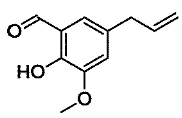 | 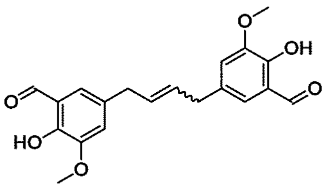 | **Ru-1**<br>**Ru-2**<br>**Ru-3** | 59<br>49<br>63 | 6.8:1<br>6.6:1<br>9:1 |
| 6 | 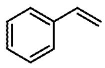 | 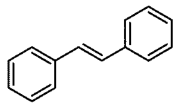 | **Ru-1**<br>**Ru-2**<br>**Ru-3** | 99<br>-<br>36 | only E |
| 7 | 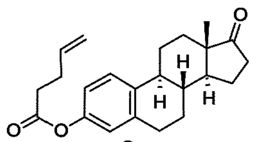 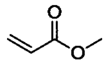 | 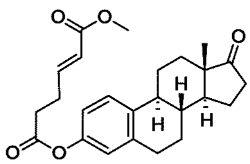 | **Ru-1**<br>**Ru-2**<br>**Ru-3** | 91<br>49<br>30 | only E |

**Example XV**

Stability tests of ruthenium complexes in solution in an inert gas atmosphere

In a glove box, 12.8 mmol of ruthenium complex was placed in an NMR tube, dissolved in 0.7 mL $CD_2Cl_2$, after which 50 mL of 1,3,5-trimethoxybenzene solution (internal standard, stock solution prepared from 128 μmol of 1,3.5 -trimethoxybenzene and 1 mL of $CD_2Cl_2$) was added. The NMR tube was sealed with a stopper and the contents were shaken. After recording the $^1H$ NMR spectrum at zero point, the NMR tube was heated for one month in a water bath (at the temperature of 40°C). Further spectra were recorded at appropriate time intervals (1-2 days from the beginning). The results of this experiment are presented in Fig. 7.

**Example XVI**

Stability/onset rapidity tests of ruthenium complexes in solution in an ethylene atmosphere

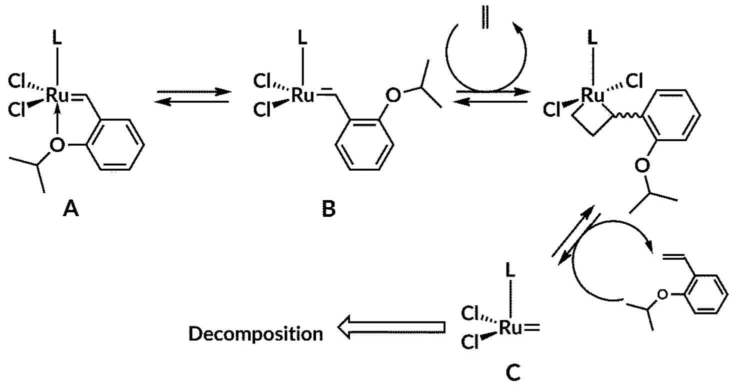


The sample was prepared according to the protocol of Example XV. The $^1H$ NMR spectrum was recorded at "0", at the temperature of 40°C. Then, a NMR tube with open Young's tap was placed in an autoclave. The autoclave was blown three times with inert gas (cycle: argon 2 bar pressure, releasing excess gas to atmospheric pressure), and then three times with ethylene (up to 20 bar pressure). The chamber was then filled with ethylene (20 bar) and allowed to stand for 20 minutes under dynamic gas pressure. Afterwards, excess gas was released, and the tap on the NMR tube was closed. The contents were mixed.

$^1H$ NMR were recorded during the reaction using Agilent's software (vnmj) "Array" function at the temperature of 40°C over 11 hours. The disappearance of the benzylidene precatalyst signal ($\delta$ = approx. 16 to 17 ppm) relative to the signal of the internal standard (1,3,5-trimethoxybenzene, aromatic signals $\delta$ = 6.08 ppm) was recorded.

The results of this experiment are presented in Fig. 8.

**Example XVII**

Ethenolysis of ethyl oleate

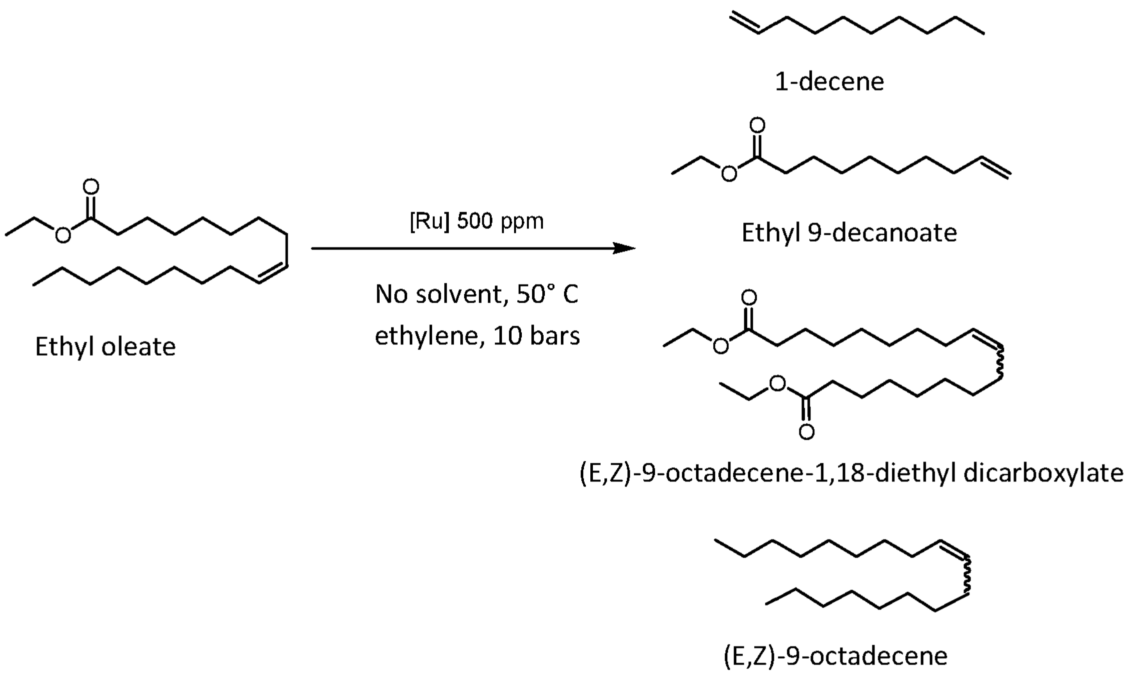


Ethyl oleate was passed through a thin layer (2cm) of neutral alumina (Alfa Aesar, alumina, activated, neutral, Brockmann Grade I, 58 A, 60 Mesh Powder, S.A. 150 $m^2/g$) and placed in a glass reactor. Subsequently, tetradecane (0.8 mL, Sigma Aldrich) was added. The mixture was well mixed and a point zero sample (1-2 drops at the tip of the Pasteur pipette) was taken. The solution of the precatalyst (in dry $CH_2Cl_2$) previously prepared in another vessel was

added in one portion to the reactor (0.1 mL, 500 ppm). A previously heated autoclave (to 50°C) was closed and connected to the ethylene line. The reactor was washed with 3 × 10 bar ethylene gas (ethylene with a purity of 3.0 → 99.9%, Linde), and then the pressure was raised to 10 bar ethylene dynamic pressure. After 3 hours of stirring, the reactor was cooled in an ice bath (over 2 min), the pressure was reduced, the reactor was opened and the sample (1 mL) of the reaction mixture was quenched with 4 mL of ethylvinyl ether solution in $CH_2Cl_2$ (C = 2 M). 0.2 mL sample of quenched mixture 0.8 mL of $CH_2Cl_2$ was added and it was analysed by GC. For experimental results, see fig. 9a.

**Example XVIII**

Ethenolysis of carophyllene

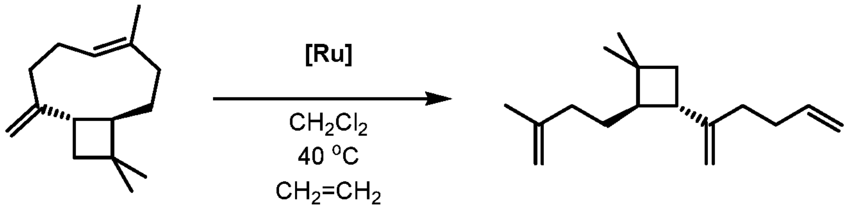


µLTo a solution of caryophyllene (90 mg, 0.1 mL, 0.434 mmol) in methylene chloride (4 mL) 100 µL of the precatalyst solution in methylene chloride (complex volume: see table) was added. The autoclave was blown three times with ethylene, and then the reaction mixture was heated to the temperature of 40°C under 10 bar dynamic ethylene pressure over 1 hour. After an hour, the autoclave was cooled to the temperature of 0°C on ice (5 minutes). The cooled reaction mixture was quenched with SnatchCat (Apeiron) solution. After 15 minutes of stirring, 10 mL of hexane was added. The solution was filtered through a layer of silica gel, which was then washed with a solution of 25% (v/v) methylene chloride in hexane. The filtrate was evaporated. A colourless oil was obtained. The composition of the sample (product to substrate ratio) was calculated based on the ratio of the signals in the $^1$H NMR spectrum.

***Table 6. Results of the caryophyllene ethenolysis reaction***

| Precat. [Ru] | Conditions complex volume, ethylene pressure (purity), temperature, concentration, time | Conversion [%] | TON | TOF [1/h] |
|---|---|---|---|---|
| **Ru-3** | 0.5 mol%, 10 bar (3.0) 40°C, 0.1 M, 1 h | 100 | 200 | 200 |
| **Ru-1** | 0.5 mol%, 10 bar (3.0) 40°C, 0.1 M, 1 h | 55 | 92 | 92 |
| **Ru-4** | 0.5 mol%, 10 bar (3.0) 40°C, 0.1 M, 1 h | 79 | 154 | 154 |
| **Ru-3** | 0.5 mol%, 10 bar (3.0) room temp., 0.1 M, 1 h | 100 | 188 | 188 |
| **Ru-3** | 0.1 mol%, 10 bar (3.0) 40°C, 0.1 M, 1 h | 100 | 953 | 953 |
| **Ru-3** | 0.1 mol%, 10 bar (3.0) room temp., 0.1 M, 1 h | 72 | 686 | 686 |
| **Ru-3** | 0,05% mol, 10 bar (3.0) 40°C, 0.1 M, 1 h | 100 | 1797 | 1797 |
| **Ru-1** | 0,05% mol, 10 bar (3.0) 40°C, 0.1 M, 1 h | 4 | 81 | 81 |

(continued)

| Precat. [Ru] | Conditions complex volume, ethylene pressure (purity), temperature, concentration, time | Conversion [%] | TON | TOF [1/h] |
|---|---|---|---|---|
| **Ru-4** | 0,05% mol, 10 bar (3.0) 40°C, 0.1 M, 1 h | 20 | 404 | 404 |
| **Ru-3** | 0,05% mol, 10 bar (3.0) room temp., 0.1 M, 1 h | 47 | 845 | 845 |
| **Ru-3** | 0,01% mol, 10 bar (3.0) 40°C, 0.1 M, 1 h | 32 | 2876 | 2876 |
| **Ru-3** | 0,025% mol, 10 bar (3.0) | SM, | - | - |
| | 40°C, no solvent, 1 h | polymerisation products, no full conversion | | |
| **Ru-3** | 0,01% mol, 10 bar (3.0) 40°C, no solvent, 1 h | SM, polymerisation products, no full conversion | - | - |
| **Ru-3** | 0,025% mol, 10 bar (4.5) 40°C, 0.4 M, 3 h | 68 | 2715 | 905 |
| **Ru-1** | 0,025% mol, 10 bar (4.5) 40°C, 0.4 M, 3 h | 33 | 1316 | 439 |
| **Ru-4** | 0,025% mol, 10 bar (4.5) 40°C, 0.4 M, 3 h | 49 | 1965 | 655 |

**Example XIX**

1-octene homometathesis selectivity, analysis of the isomerisation versus olefin metathesis processes

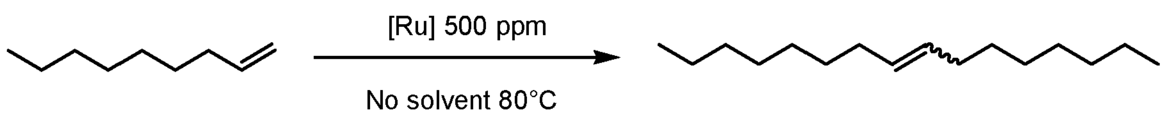


1.5 mL (9.37 mmol, 1 eq.) of 1-octene and 0.5 mL (1.9 mmol, 0.2 eq) of tetradecane as the internal standard were placed in a Schlenk vessel. The zero point sample (2-3 drops at the tip of the Pasteur pipette) was taken. The mixture was heated to the temperature of 80°C and then 500 ppm (0.0005 eq., 0.05 mol%) of the precatalyst was added. Samples were taken at different times (10 min, 30 min, 1 h, 2 h, 24 h), and quenched using SnatchCat solution in methylene chloride. The samples thus obtained were examined using a gas chromatograph with a flame detector (FID). For results, see fig. 10a and 10b.

## Claims

1. A compound of the formula **9**

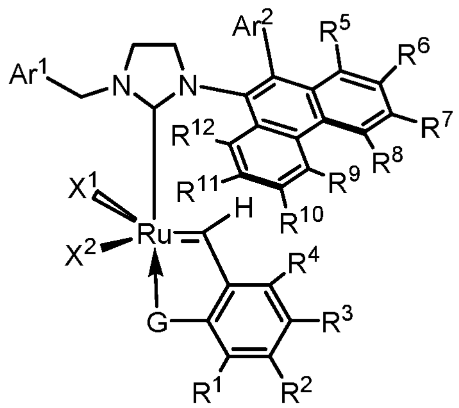

**9**

wherein:

$X^1$ and $X^2$ are each an anionic ligand; $X^1$ and $X^2$ may be interconnected to form a cyclic system;
G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;
$Ar^1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which are optionally substituted with hydrogen atoms or optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;
$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-SO$_2$NR'''$_2$), a sulfonamide group (-NR'''SO$_2$R'''), a sulpho group (-SO$_2$R'''), a sulphonium (-S$^+$R'''$_2$), a phosphonium group (-P(O)(OR''')$_2$), a phosphinium group (-P(O)R'''(OR''')), a phosphonous group (-P(OR''')$_2$), a phosphine group (-PR'''$_2$), a phosphine group (-P$^+$R'''$_3$) a nitro group (-NO$_2$), a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-N$^+$R'''$_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system;
$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system.

**2.** The compound according to claim 1 of the formula 9a

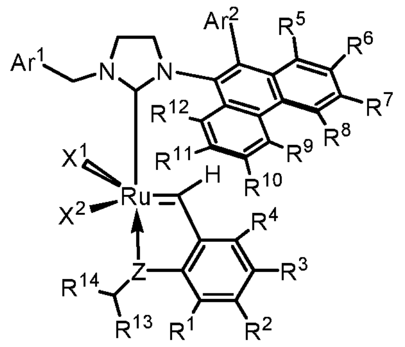

**9a**

wherein, $X^1$ and $X^2$ are each a halogen atom;

Z is O, S, NR' or PR', wherein groups R' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy;

$Ar_1$ and $Ar^2$ are each a $C_5$-$C_{20}$ aryland/or a $C_4$-$C_{20}$ heteroaryl group, which are substituted by hydrogen atoms, or optionally are substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl groups, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group ($-SO_2NR'''_2$), a sulpho group ($-SO_2R'''$), a sulphonium ($-S^+R'''_2$), a phosphonium group ($-P(O)(OR''')_2$), a phosphinium group (-P(O)R'''(OR''')), a phosphonous group ($-P(OR''')_2$), a phosphine group ($-PR'''_2$), a nitro group ($-NO_2$), a nitroso group (-NO), a carboxy group (-COOH), an ester group (-COOR'''), a formyl group (-CHO), a ketone group, (-COR'''), an amide ($-CONR'''_2$), an imido (-CONR'''COR'''), an amino ($-NR'''_2$), an ammonium ($-N^+R'''_3$), an amido (-NR'''COR''') groups, in which groups R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

$R^{13}$ and $R^{14}$, are each a hydrogen atom or a $C_1$-$C_{25}$ alkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_5$-$C_{20}$ heteroaryloxy group, or which may be interconnected to form a substituted or non-substituted $C_4$-$C_{10}$ cyclic or $C_4$-$C_{12}$ polycyclic systems, it may also be an ester (-COOR'), amide ($-CONR'_2$), formyl (-CHO), ketone (-COR'), hydroxamic (-CON(OR')(R')) groups or a halogen atom, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy, or a halogen atom.

**3.** The compound according to claim 1 of the formula **9b**

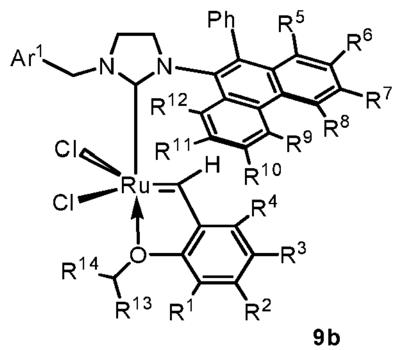

**9b**

wherein, $Ar_1$ is each a $C_5$-$C_{20}$ aryl, or a $C_4$-$C_{20}$ heteroaryl group, which is substituted by hydrogen atoms, or

optionally is substituted by at least one a $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^1$, $R^2$, $R^3$, $R^4$ are each a hydrogen atom, an alkoxy group (-OR'), a sulfidic group (-SR'), a cyano group (-CN), a sulfoxide group (-S(O)R'), a sulfonamide group (-$SO_2NR'_2$), a sulpho group (-$SO_2R$), a sulphonium (-S+R'2), a phosphonium group (-P(O)(OR')2), a phosphinium group (-P(O)R'(OR')), a phosphonous group (-$P(OR')_2$), a phosphine group (-$PR'_2$), a nitro group (-$NO_2$), a nitroso group (-NO), a carboxy group (-COOH), an ester group (-COOR'), a formyl group (-CHO), a ketone group, (-COR'), an amide (-$CONR'_2$), an imido (-CONR'COR'), an amino (-$NR'_2$), an ammonium (-$N^+R'_3$), an amido (-NR'COR') groups, in which groups R' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

$R^{13}$ and $R^{14}$, are each a hydrogen atom or a $C_1$-$C_{25}$ alkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_5$-$C_{20}$ heteroaryloxy group, or which may be interconnected to form a substituted or non-substituted $C_4$-$C_{10}$ cyclic or $C_4$-$C_{12}$ polycyclic systems, it may also be an ester (-$COOR^a$), amide (-$CONR^a_2$), formyl (-CHO), ketone (-$COR^a$), hydroxamic (-$CON(OR^a)(R^a)$) groups or a halogen atom, wherein $R^a$ is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy, or a halogen atom.

4. The compound according to claim 1, having a structure represented by a formula such as Ru-3:

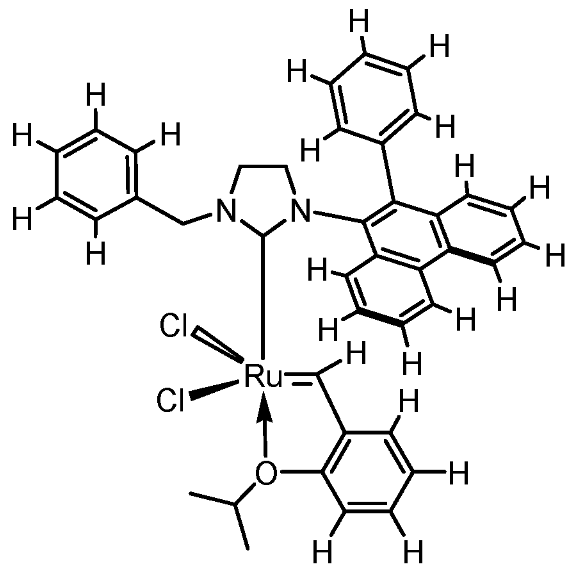

**Ru-3**

5. Method for preparing a compound of formula **9**

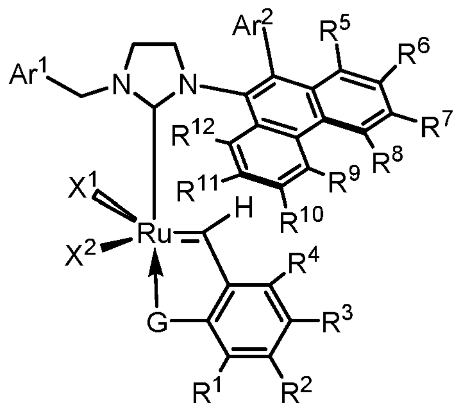

**9**

wherein:

$X^1$ and $X^2$ are each an anionic ligand; $X^1$ and $X^2$ may be interconnected to form a cyclic system;

G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;

$Ar^1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-SO$_2$NR'''$_2$), a sulfonamide group (-NR'''SO$_2$R'''), a sulpho group (-SO$_2$R'''), a sulphonium (-S$^+$R'''$_2$), a phosphonium group (-P(O)(OR''')$_2$), a phosphinium group (-P(O)R'''(OR''')), a phosphonous group (-P(OR''')$_2$), a phosphine group (-PR'''$_2$), a phosphine group (-P$^+$R'''$_3$) a nitro group (-NO$_2$), a a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-N$^+$R'''$_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system.

**characterised in that** the alkylidene ruthenium complex of formula **10**

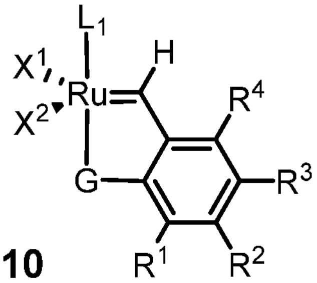

**10**

wherein:

$L^1$ are neutral ligands selected from the group comprising pyridine or substituted pyridine, $P(R^b)_3$, $P(OR^b)_3$, $O(R^b)_2$, $N(R^b)_3$, wherein $R^b$ is each a $C_1$-$C_{12}$ alkyl, a $C_3$-$C_{12}$ cycloalkyl, a $C_5$-$C_{20}$ aryl, a $C_7$-$C_{24}$ aralkyl, a $C_5$-$C_{24}$ perfluoroaryl, 5-12 membered heteroaryl;

$X^1$ and $X^2$ are each an anionic ligand; $X^1$ and $X^2$ may be interconnected to form a cyclic system;

G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;

$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-SO$_2$NR'''$_2$), a sulpho group (-SO$_2$R'''), a sulphonium (-S+R'''2), a phosphonium group (-P(O)(OR''')$_2$), a phosphinium group (-P(O)R'''(OR''')), a phosphonous group (-P(OR''')$_2$), a phosphine group (-PR'''$_2$), a nitro group (-NO$_2$), a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-N$^+$R'''$_3$), an amido (-NR'''COR''') groups, in which groups R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, $C_5$-$C_{20}$ heteroaryloxy, and wherein $R^1$, $R^2$, $R^3$ and $R^4$ may be interconnected to form a cyclic system, is reacted with carbene of formula **8aa**

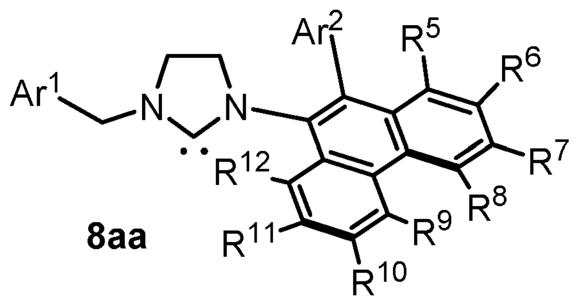

**8aa**

wherein:

$Ar_1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which are optionally substituted with hydrogen atoms or optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least

one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

**6.** Method for preparing a compound of formula 9 according to claim 5, **characterised in that** the alkylidene ruthenium complex of formula **10**

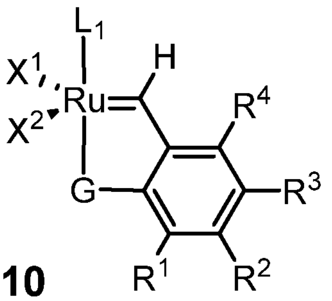

**10**

wherein

$L^1$ are neutral ligands selected from the group comprising pyridine or substituted pyridine, $P(R^b)_3$, $P(OR^b)_3$, $O(R^b)_2$, $N(R^b)_3$, wherein $R^b$ is each a $C_1$-$C_{12}$ alkyl, a $C_3$-$C_{12}$ cycloalkyl, a $C_5$-$C_{20}$ aryl, a $C_7$-$C_{24}$ aralkyl, a $C_5$-$C_{24}$ perfluoroaryl, a 5-12 membered heteroaryl;
$X^1$ and $X^2$ are each an anionic ligand; $X^1$ and $X^2$ may be interconnected to form a cyclic system;
G is a halogen atom or a substituent selected from the group of OR', SR', S(O)R', $S(O)_2R'$ N(R')(R"), P(R')(R"), where R' and R" are the same or different $C_1$-$C_{25}$ alkyl group, a $C_3$-$C_{12}$ cycloalkyl group, a $C_1$-$C_{25}$ alkoxy group, a $C_2$-$C_{25}$ alkenyl group, a $C_1$-$C_{12}$ perfluoroalkyl, a $C_5$-$C_{20}$ aryl, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, or which may be interconnected to form a substituted or non-substituted $C_4$-$C_{10}$ cyclic or $C_4$-$C_{12}$ polycyclic systems, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, may also be substituted by ester (-COOR'), amide (-CONR'$_2$), formyl (-CHO), ketone (-COR'), hydroxamic (-CON(OR')(R')) group, wherein R' is a $C_1$-$C_{12}$ alkyl, a $C_3$-$C_{12}$ cycloalkyl, a $C_2$-$C_{12}$ alkenyl, a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{20}$ aryl, a $C_5$-$C_{24}$ aryloxy, a $C_7$-$C_{24}$ aralkyl, a $C_2$-$C_{20}$ heterocyclyl, a $C_4$-$C_{20}$ heteroaryl, a $C_5$-$C_{20}$ heteroaryloxy, or a halogen atom;
$R^1$, $R^2$, $R^3$, $R^4$ are each a hydrogen atom, an alkoxy group (-OR'''), a sulfidic group (-SR'''), a cyano group (-CN), a sulfoxide group (-S(O)R'''), a sulfonamide group (-SO$_2$NR'''$_2$), a sulpho group (-SO$_2$R'''), a sulphonium (-S+R'''$_2$), a phosphonium group (-P(O)(OR''')$_2$), a phosphinium group (-P(O)R'''(OR''')), a phosphonous group (-P(OR''')$_2$), a phosphine group (-PR'''$_2$), a nitro group (-NO$_2$), a nitroso group (-NO), a carboxy group (-COOH), an ester group (-COOR'''), a formyl group (-CHO), a ketone group, (-COR'''), an amide (-CONR'''$_2$), an imido (-CONR'''COR'''), an amino (-NR'''$_2$), an ammonium (-N$^+$R'''$_3$), an amido (-NR'''COR''') groups, in which group R''' is a $C_1$-$C_5$ alkyl, a $C_1$-$C_5$ perfluoroalkyl, a $C_5$-$C_{24}$ aryl, a $C_7$-$C_{24}$ aralkyl, a $C_5$-$C_{24}$ perfluoroaryl, a $C_4$-$C_{20}$ heteroaryl, a $C_5$-$C_{20}$ heteroaryloxy, and wherein $R^1$, $R^2$, $R^3$ and $R^4$ may be interconnected to form a cyclic system,
is reacted with carbene produced *in situ* as a result of the effect of a base selected from those such as potassium *tert*-amylate, potassium *tert*-butoxide, potassium *N*, *N'*-bis (trimethylsilyl) amide, sodium hydride, on a carbene precursor of formula **8a**

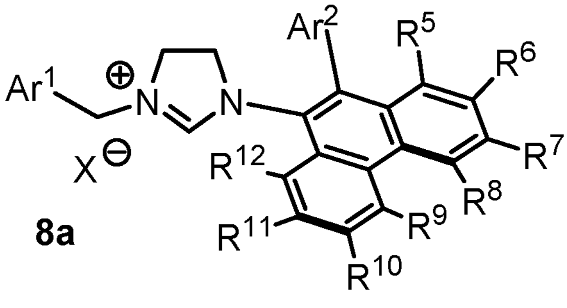

**8a**

wherein:

$Ar_1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;
$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system;
$X^-$ is a halide anion, or $BF_4^-$, $PF_6^-$, $ClO_4^-$.

7. Method for preparing a compound of formula **9** according to claim 5, **characterised in that** the carbides of formula **8aa** are provided to the reaction medium by thermally generating them *in situ* from suitable carbene precursors of formula **8b** that are adducts with chloroform, carbon dioxide or alcohol,

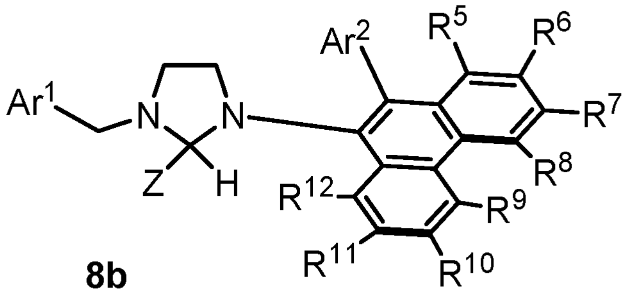

**8b**

wherein:

Z is $CCl_3$, $CO_2$ or OR', wherein R' is a $C_1$-$C_{12}$ alkyl, a $C_3$-$C_{12}$ cycloalkyl, a $C_2$-$C_{12}$ alkenyl, or a $C_5$-$C_{20}$ aryl, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_5$-$C_{20}$ heteroaryloxy or a halogen atom;
$Ar_1$ and $Ar^2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;
$R^5$, $R^6$, $R^7$, $R^8$, $R^9$, $R^{10}$, $R^{11}$ and $R^{12}$ are each a hydrogen atom or a $C_1$-$C_{25}$ alkyl, $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system,

8. Method for preparing a compound of formula 9 according to claim 5, **characterised in that** the alkylidene ruthenium complex of formula **10** is contacted with a compound of formula **8c,** which acts as a NHC carbene ligand donor of formula **8aa,**

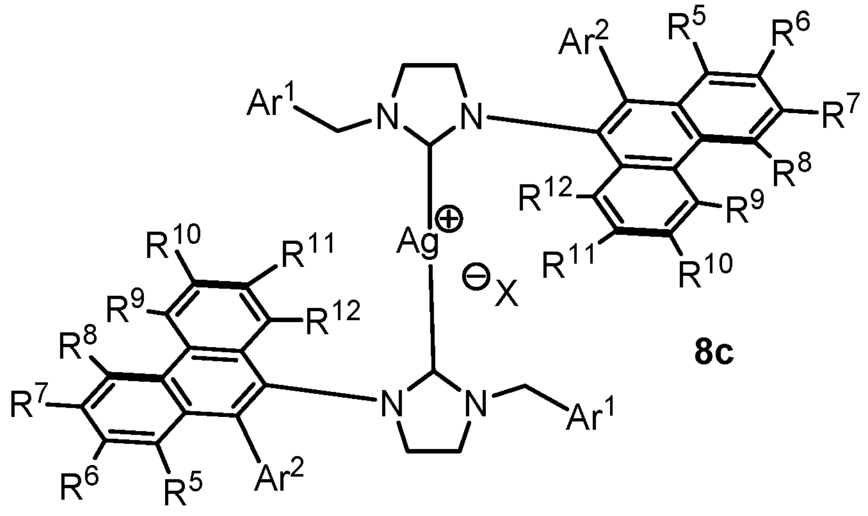

**8c**

wherein:

$Ar_1$ and $Ar_2$ are each a $C_5$-$C_{20}$ aryl, a $C_5$-$C_{24}$ aryloxy, a $C_4$-$C_{20}$ heteroaryl, a $C_5$-$C_{20}$ heteroaryloxy, a $C_5$-$C_{24}$ perfluoroaryl group, which is optionally substituted with hydrogen atoms or it is optionally substituted with at least one $C_1$-$C_{12}$ alkyl, a $C_1$-$C_{12}$ perfluoroalkyl, a $C_1$-$C_{12}$ alkoxy, a $C_5$-$C_{24}$ aryloxy, a $C_2$-$C_{20}$ heterocyclyl, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl, or a halogen atom;

$R^5$, $R^6$, $R^7$, $R^8$, $R^9$, $R^{10}$, $R^{11}$ and $R^{12}$ are each a hydrogen atom or a $C_1$-$C_{25}$ alkyl, a $C_3$-$C_{12}$ cycloalkyl, a $C_1$-$C_5$ perfluoroalkyl, a $C_2$-$C_{12}$ alkenyl, a $C_5$-$C_{20}$ aryl, a $C_5$-$C_{24}$ aryloxy, a $C_2$-$C_{20}$ heterocyclic, a $C_4$-$C_{20}$ heteroaryl, a $C_5$-$C_{20}$ heteroaryloxy, a $C_7$-$C_{24}$ aralkyl, a $C_5$-$C_{24}$ perfluoroaryl group, which are optionally substituted by at least one $C_1$-$C_{12}$ alkyl, Ca $_1$-$C_{12}$ perfluoroalkyl, a $C_1$-$C_{12}$ alkoxy, a $C_5$-$C_{24}$ aryloxy, a $C_4$-$C_{20}$ heteroaryl, a $C_5$-$C_{20}$ heteroaryloxy, or a halogen atom; and wherein $R^5$ and $R^6$ and/or $R^7$ and $R^8$ may be interconnected to form a cyclic system;

$X^-$ is a halide anion, or $BF_4^-$, $PF_6^-$, $ClO_4^-$.

**9.** The use of compound of formula 9 according to any of the claims 1 to 4 as a precatalyst and/or catalyst in olefin metathesis reactions.

**10.** The use according to claim 9, wherein the compound of formula 9 is used as a precatalyst and/or catalyst in the reactions of ring-closing metathesis (RCM), homomethatesis, cross-metathesis (CM), ethenolysis, isomerisation, in the diastereoselective ring rearrangement metathesis (DRRM) reaction, "alken-alkyn" (enyn) metathesis or ROMP type polymerisation reactions.

**11.** The use according to claim 9 or 10, wherein the compound of formula 9 is used as a precatalyst and/or catalyst in the ring opening metathesis polymerisation (ROMP) reaction of dicyclopentadiene or norbornene.

**12.** The use according to any of the claims 9 to 11, wherein the compound of formula 9 is used as a precatalyst and/or catalyst in the reaction mixture for a period between 1 minute and 24 hours, and/or the reaction is conducted in non-polar solvents or without a solvent, and/or the reaction is conducted in an organic solvent such as toluene, benzene, mesitylene, dichloromethane, ethyl acetate, methyl acetate, tertbutyl methyl ether, cyclopentylmethyl ether, or with no solvent, and/or the reaction is conducted at a temperature of from 20 to 150°C.

**13.** The use according to any of the claims 9 to 12, wherein compound 9 is used in an amount of less than 1 mol%, and/or the compound 9 is added to the reaction mixture in portions and/or continuously using a pump, and/or the compound 9 is added to the reaction mixture as a solid and/or as a solution in an organic solvent, and/or at least one olefin is added to the reaction mixture in portions and/or continuously using a pump.

**14.** The use according to any of the claims 9 to 19, wherein the gaseous by-product of the reaction (ethylene, propylene, butylene) is actively removed from the reaction mixture using an inert gas or vacuum.

**15.** The use of the compound of formula **9** as a substrate for the synthesis of other ruthenium complex compounds that

are precatalysts and/or catalysts of olefin metathesis.

## Patentansprüche

**1.** Verbindung mit der Formel 9

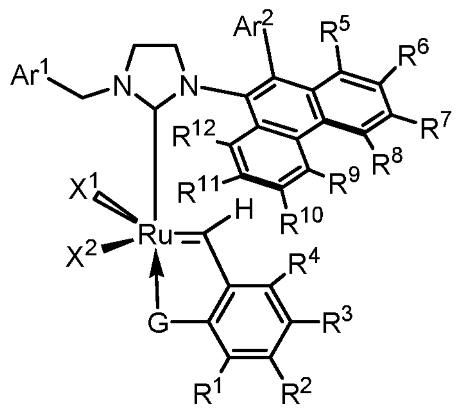

**9**

wobei:

X1 und X2 unabhängig voneinander anionische Liganden sind, X1 und X2 miteinander verbunden werden können, um ein zyklisches System zu bilden;

G ist das Halogenatom oder ein Substituent, ausgewählt aus der Gruppe OR', SR', S(O)R', $S(O)_2R'$ N(R')(R"), P(R')(R"), wobei R' und R" die gleiche oder unterschiedliche $C_1$-$C_{25}$-Alkylgruppe, $C_3$-$C_{12}$-Cycloalkylgruppe, $C_1$-$C_{25}$-Alkoxygruppe, $C_2$-$C_{25}$-Alkenylgruppe, $C_1$-$C_{12}$ Perfluoralkylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe bedeuten, oder die mit der Herstellung von substituierten oder unsubstituierten zyklischen $C_4$-$C_{10}$ der polyzyklischen $C_4$-$C_{12}$-Systemen kombiniert werden können, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_2$-$C_{20}$-Heterozyklus, $C_4$-$C_{20}$-Heteroaryl, $C_5$-$C_{20}$-Heteroaryloxyl substituiert sein können, sie können auch eine substituierte Ester- (-COOR'), Amid- (-CONR'$_2$), Formyl- (-CHO), Keto- (-COR'), Hydroxamgruppe (-CON(OR')(R')) sein, worin R' $C_1$-$C_{12}$-Alkyl, $C_3$-$C_{12}$-Zykloalkyl, $C_2$-$C_{12}$-Alkenyl, $C_5$-$C_{20}$-Aryl bedeutet, die mit mindestens einem $C_1$-$C_{12}$-Alkyl, $C_1$-$C_{12}$-Perfluoralkyl, $C_1$-$C_{12}$-Alkoxyl, $C_5$-$C_{20}$-Aryl, $C_5$-$C_{24}$-Aryloxyl, $C_7$-$C_{24}$-Aralkyl, $C_2$-$C_{20}$-Heterozyklus, $C_4$-$C_{20}$-Heteroaryl, $C_5$-$C_{20}$-Heteroaryloxyl oder einem Halogenatom substituiert sein können;

$Ar^1$ i $Ar^2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;

$R^1$, $R^2$, $R^3$, $R^4$ unabhängig voneinander ein Wasserstoffatom, eine Alkoxygruppe (-OR'''), eine Sulfidgruppe (-SR'''), eine Cyangruppe (-CN), eine Sulfoxidgruppe (-S(O)R'''), eine Sulfonamidgruppe (-SO$_2$NR'''$_2$), eine Sulfonamidgruppe (-NR'''SO$_2$R'''), eine Sulfongruppe (-SO$_2$R'''), eine Sulfoniumgruppe (-S$^+$R'''$_2$), eine Phosphitgruppe (-P(O)(OR''')$_2$), eine Phosphinatgruppe (-P(O)R'''(OR''')), eine Phosphonitgruppe (-P(OR''')$_2$), eine Phosphingruppe (-PR'''$_2$), eine Phosphongruppe (-P$^+$R'''$_3$) eine Nitrogruppe (-NO$_2$), eine Nitrosogruppe (-NO), eine Carboxylgruppe (-COOH), eine Estergruppe (-COOR'''), eine Formylgruppe (-CHO), eine Ketogruppe (-COR'''), eine Amidgruppe (-CONR'''$_2$), eine Imidgruppe (-CONR'''COR'''), eine Amingruppe (-NR'''$_2$), eine Ammoniumgruppe (-N$^+$R'''$_3$), eine Amidgruppe (-NR'''COR''') sind, wobei R''' $C_1$-$C_5$-Alkyl, $C_1$-$C_5$-Perfluoralkyl, $C_5$-$C_{24}$-Aryl, $C_7$-$C_{24}$-Aralkyl, $C_5$-$C_{24}$-Perfluoraryl, $C_4$-$C_{20}$-Heteroaryl, $C_5$-$C_{20}$-Heteroaryloksyl bedeutet, und $R^1$, $R^2$, $R^3$ und $R^4$ kombiniert werden können, um ein zyklisches System zu bilden;

$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, $C_3$-$C_{12}$-Cycloalkylgruppe, $C_1$-$C_5$-Perfluoralkylgruppe, $C_2$-$C_{12}$-Alkenylgruppe, $C_5$-$C_{20}$-Arylgruppe, $C_5$-$C_{24}$-Aryloxygruppe, $C_2$-$C_{20}$-heterozyklischeGruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe sind, die mit mindestens einem $C_1$-$C_{12}$-Alkyl, $C_1$-$C_{12}$-Perflu-

oralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.

**2.** Verbindung nach Anspruch 1 mit der Formel **9a**

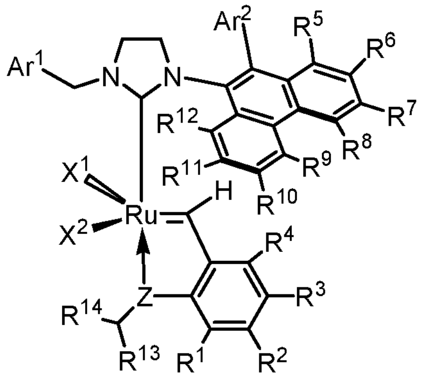

**9a**

X1 und X2 sind unabhängig voneinander ein Halogenatom;
Z bedeutet O, S, NR' oder PR', wobei R' C1-C5-Alkyl, C1-C5-Perfluoralkyl, C5-C24-Aryl, C7-C24-Aralkyl, C5-C24-Perfluoraryl, C4-C20-Heteroaryl, C5-C20-Heteroaryloxyl bedeutet;
$Ar_1$ i $Ar_2$ unabhängig voneinander die $C_5$-$C_{20}$-Arylgruppe, $C_4$-$C_{20}$-Heteroarylgruppe sind, die mit Wasserstoffatomen oder alternativ mit mindestens einer C1-C12-Alkylgruppe, $C_1$-$C_{12}$-Perfluoralkylgruppe, $C_1$-$C_{12}$-Alkoxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;
R1, R2, R3, R4 unabhängig voneinander ein Wasserstoffatom, eine Alkoxygruppe (-OR'''), eine Sulfidgruppe (-SR'''), eine Cyangruppe (-CN), eine Sulfoxidgruppe (-S(O)R'''), eine Sulfonamidgruppe (-SO2NR'''2), eine Sulfongruppe (-$SO_2$R'''), eine Phosphitgruppe (-P(O)(OR''')2), eine Phosphinatgruppe (-P(O)R'''(OR''')), eine Phosphonitgruppe (-P(OR''')$_2$), eine Phosphingruppe (-PR'''2), eine Nitrogruppe (-$NO_2$), eine Nitrosogruppe (-NO), eine Carboxylgruppe (-COOH), eine Estergruppe (-COOR'''), eine Formylgruppe (-CHO), eine Ketogruppe (-COR'''), eine Amidgruppe (-CONR'''2), eine Imidgruppe (-CONR'''COR'''), eine Amingruppe (-NR'''2), eine Ammoniumgruppe (-N+R'''$_3$), eine Amidgruppe (-NR'''COR''') sind, wobei R''' C1-C5-Alkyl, C1-C5-Perfluoralkyl, C5-C24-Aryl, C7-C24-Aralkyl, C5-C24-Perfluoraryl, C4-C20-Heteroaryl, C5-C20-Heteroaryloksyl bedeutet, und R1, R2, R3 und R4 kombiniert werden können, um ein zyklisches System zu bilden;
$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, eine $C_3$-$C_{12}$-Cycloalkylgruppe, eine $C_1$-$C_5$-Perfluoralkylgruppe, eine $C_2$-$C_{12}$-Alkenylgruppe, eine $C_5$-$C_{20}$-Arylgruppe, eine $C_5$-$C_{24}$-Aryloxygruppe, eine $C_2$-$C_{20}$-heterozyklische Gruppe, eine $C_4$-$C_{20}$-Heteroarylgruppe, eine $C_5$-$C_{20}$-Heteroaryloxylgruppe, eine $C_7$-$C_{24}$ -Aralkylgruppe, eine $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.
$R^{13}$ und $R^{14}$, unabhängig voneinander ein Wasserstoffatom, eine $C_1$-$C_{25}$-Alkylgruppe, eine $C_1$-$C_{25}$-Alkoxygruppe, eine $C_2$-$C_{25}$-Alkenylgruppe, eine $C_1$-$C_{12}$-Perfluoralkylgruppe, eine $C_5$-$C_{20}$-Arylgruppe, eine $C_5$-$C_{24}$-Aryloxygruppe, eine $C_5$-$C_{20}$-Heteroaryloxylgruppe sind, oder die mit der Herstellung von substituierten oder unsubstituierten zyklischen C4-C10 der polyzyklischen C4-C12-Systemen kombiniert werden können, sie können auch eine substituierte Ester- (-COOR'), Amid- (-CONR'$_2$), Formyl- (-CHO), Keto- (-COR'), Hydroxamgruppe (-CON(OR')(R')) oder einem Halogenatom sein, worin R' $C_1$-$C_{12}$-Alkyl, $C_3$-$C_{12}$-Zykloalkyl, $C_2$-$C_{12}$-Alkenyl, $C_5$-$C_{20}$-Aryl bedeutet, die gegebenenfalls mit mindestens einem C1-C12-Alkyl, C1-C12-Perfluoralkyl, C1-C12-Alkoxyl, C5-C24-Aryoxyl, C5-C20-Heteroaryloxyl oder einem Halogenatom substituiert sind;

**3.** Verbindung nach Anspruch 1 mit der Formel 9b

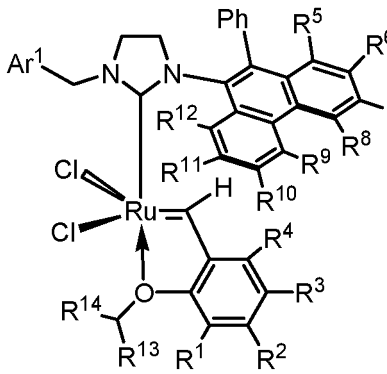

**9b**

$Ar_1$ unabhängig die $C_5$-$C_{20}$-Arylgruppe, $C_4$-$C_{20}$-Heteroarylgruppe ist, die mit Wasserstoffatomen oder alternativ mit mindestens einer C1-C12-Alkylgruppe, $C_1$-$C_{12}$-Perfluoralkylgruppe, $C_1$-$C_{12}$-Alkoxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;

R1, R2, R3, R4 unabhängig voneinander ein Wasserstoffatom, eine Alkoxygruppe (-OR'), eine Sulfidgruppe (-SR'), eine Cyangruppe (-CN), eine Sulfoxidgruppe (-S(O)R'), eine Sulfonamidgruppe (-SO2NR'2), eine Sulfongruppe (-$SO_2$R), eine Phosphitgruppe (-P(O)(OR')2), eine Phosphinatgruppe (-P(O)R'(OR')), eine Phosphonitgruppe (-P(OR')2), eine Phosphingruppe (-PR'2), eine Nitrogruppe (-$NO_2$), eine Nitrosogruppe (-NO), eine Carboxylgruppe (-COOH), eine Estergruppe (-COOR'), eine Formylgruppe (-CHO), eine Ketogruppe (-COR'), eine Amidgruppe (-CONR'2), eine Imidgruppe (-CONR'COR'), eine Amingruppe (-NR'2), eine Ammoniumgruppe (-N+R'3), eine Amidgruppe (-NR'COR') sind, wobei R' C1-C5-Alkyl, C1-C5-Perfluoralkyl, C5-C24-Aryl, C7-C24-Aralkyl, C5-C24-Perfluoraryl, C4-C20-Heteroaryl, C5-C20-Heteroaryloksyl bedeutet, und R1, R2, R3 und R4 kombiniert werden können, um ein zyklisches System zu bilden;

$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, eine $C_3$-$C_{12}$-Cycloalkylgruppe, eine $C_1$-$C_5$-Perfluoralkylgruppe, eine $C_2$-$C_{12}$-Alkenylgruppe, eine $C_5$-$C_{20}$-Arylgruppe, eine $C_5$-$C_{24}$-Aryloxygruppe, eine $C_2$-$C_{20}$-heterozyklische Gruppe, eine $C_4$-$C_{20}$-Heteroarylgruppe, eine $C_5$-$C_{20}$-Heteroaryloxylgruppe, eine $C_7$-$C_{24}$-Aralkylgruppe, eine $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.

$R^{13}$ und $R^{14}$, unabhängig voneinander ein Wasserstoffatom, eine $C_1$-$C_{25}$-Alkylgruppe, eine $C_1$-$C_{25}$-Alkoxygruppe, eine $C_2$-$C_{25}$-Alkenylgruppe, eine $C_1$-$C_{12}$-Perfluoralkylgruppe, eine $C_5$-$C_{20}$-Arylgruppe, eine $C_5$-$C_{24}$-Aryloxygruppe, eine $C_5$-$C_{20}$-Heteroaryloxylgruppe sind, oder die mit der Herstellung von substituierten oder unsubstituierten zyklischen C4-C10 der polyzyklischen C4-C12-Systemen kombiniert werden können, sie können auch eine substituierte Ester- (-COOR'), Amid- (-CONR'$_2$), Formyl- (-CHO), Keto- (-COR'), Hydroxamgruppe (-CON(OR')(R')) oder einem Halogenatom sein, worin R' $C_1$-$C_{12}$-Alkyl, $C_3$-$C_{12}$-Zykloalkyl, $C_2$-$C_{12}$-Alkenyl, $C_5$-$C_{20}$-Aryl bedeutet, die gegebenenfalls mit mindestens einem C1-C12-Alkyl, C1-C12-Perfluoralkyl, C1-C12-Alkoxyl, C5-C24-Aryoxyl, C5-C20-Heteroaryloxyl oder einem Halogenatom substituiert sind.

**4.** Verbindung nach Anspruch 1, mit einer Struktur, die durch Formel wie Ru-3 dargestellt wird:

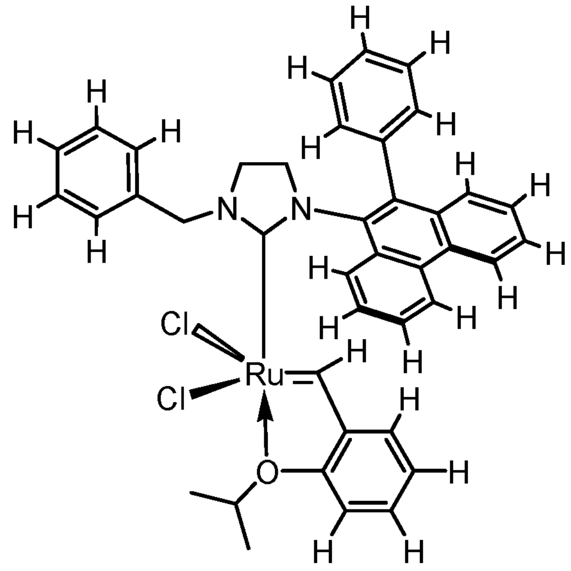

**Ru-3**

**5.** Verfahren zur Herstellung der Verbindung mit der Formel 9

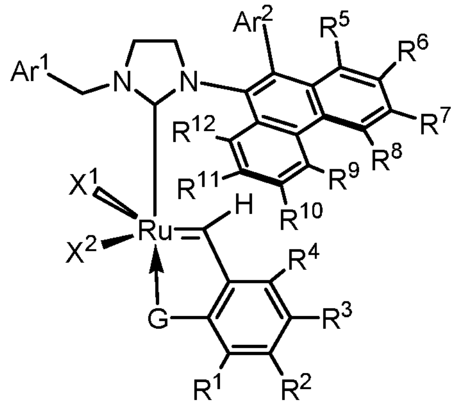

**9**

wobei:

X1 und X2 unabhängig voneinander anionische Liganden sind, X1 und X2 miteinander verbunden werden können, um ein zyklisches System zu bilden;

G ist das Halogenatom oder ein Substituent, ausgewählt aus der Gruppe OR', SR', S(O)R', $S(O)_2R'$ N(R')(R"), P(R')(R"), wobei R' und R" die gleiche oder unterschiedliche $C_1$-$C_{25}$-Alkylgruppe, $C_3$-$C_{12}$-Cycloalkylgruppe, $C_1$-$C_{25}$-Alkoxygruppe, $C_2$-$C_{25}$-Alkenylgruppe, $C_1$-$C_{12}$-Perfluoralkylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe bedeuten, oder die mit der Herstellung von substituierten oder unsubstituierten zyklischen $C_4$-$C_{10}$ der polyzyklischen $C_4$-$C_{12}$-Systemen kombiniert werden können, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_2$-$C_{20}$-Heterozyklus, $C_4$-$C_{20}$-Heteroaryl, $C_5$-$C_{20}$-Heteroaryloxyl substituiert sein können, sie können auch eine substituierte Ester- (-COOR'), Amid- (-CONR'$_2$), Formyl- (-CHO), Keto- (-COR'), Hydroxamgruppe (-CON(OR')(R')) sein, worin R' $C_1$-$C_{12}$-Alkyl, $C_3$-$C_{12}$-Zykloalkyl, $C_2$-$C_{12}$-Alkenyl, $C_5$-$C_{20}$-Aryl bedeutet, die mit mindestens einem $C_1$-$C_{12}$-Alkyl, $C_1$-$C_{12}$-Perfluoralkyl, $C_1$-$C_{12}$-Alkoxyl, $C_5$-$C_{20}$-Aryl, $C_5$-$C_{24}$-Aryloxyl, $C_7$-$C_{24}$-Aralkyl, $C_2$-$C_{20}$-Heterozyklus, $C_4$-$C_{20}$-Heteroaryl, $C_5$-$C_{20}$-Heteroaryloxyl oder einem Halogenatom substituiert sein können;

$Ar^1$ i $Ar^2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxy-

gruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;

$R^1$, $R^2$, $R^3$, $R^4$ unabhängig voneinander ein Wasserstoffatom, eine Alkoxygruppe (-OR'''), eine Sulfidgruppe (-SR'''), eine Cyangruppe (-CN), eine Sulfoxidgruppe (-S(O)R'''), eine Sulfonamidgruppe ($-SO_2NR'''_2$), eine Sulfonamidgruppe ($-NR'''SO_2R'''$), eine Sulfongruppe ($-SO_2R'''$), eine Sulfoniumgruppe ($-S^+R'''_2$), eine Phosphitgruppe ($-P(O)(OR''')_2$), eine Phosphinatgruppe (-P(O)R'''(OR''')), eine Phosphonitgruppe ($-P(OR''')_2$), eine Phosphingruppe ($-PR'''_2$), eine Phosphongruppe ($-P^+R'''_3$) eine Nitrogruppe ($-NO_2$), eine Nitrosogruppe (-NO), eine Carboxylgruppe (-COOH), eine Estergruppe (-COOR'''), eine Formylgruppe (-CHO), eine Ketogruppe (-COR'''), eine Amidgruppe ($-CONR'''_2$), eine Imidgruppe (-CONR'''COR'''), eine Amingruppe ($-NR'''_2$), eine Ammoniumgruppe ($-N^+R'''_3$), eine Amidgruppe (-NR'''COR''') sind, wobei R''' $C_1$-$C_5$-Alkyl, $C_1$-$C_5$-Perfluoralkyl, $C_5$-$C_{24}$-Aryl, $C_7$-$C_{24}$-Aralkyl, $C_5$-$C_{24}$-Perfluoraryl, $C_4$-$C_{20}$-Heteroaryl, $C_5$-$C_{20}$-Heteroaryloksyl bedeutet, und $R^1$, $R^2$, $R^3$ und $R^4$ kombiniert werden können, um ein zyklisches System zu bilden;

$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, $C_3$-$C_{12}$-Cycloalkylgruppe, $C_1$-$C_5$-Perfluoralkylgruppe, $C_2$-$C_{12}$-Alkenylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden,

**dadurch gekennzeichnet, dass** der Alkyliden-Rutheniumkomplex mit der Formel 10

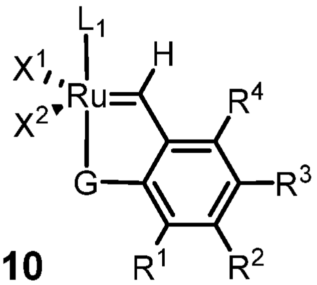

**10**

wobei:

L1 einen inerten Liganden bedeutet, der aus einer Gruppe ausgewählt ist, die Pyridin oder substituiertes Pyridin, P(R')3, P(OR')3, O(R')2, N(R')3 einschließt, wobei jedes R' unabhängig voneinander C1-C12-Alkyl, $C_3$-$C_{12}$-Zykloalkyl, C5-C20-Aryl, C7-C24-Aralkyl, C5-C24-Perfluoraryl, 5-12-gliedriges Heteroaryl bedeutet;

X1 und X2 unabhängig voneinander anionische Liganden sind, X1 und X2 miteinander verbunden werden können, um ein zyklisches System zu bilden;

G ist das Halogenatom oder ein Substituent, ausgewählt aus der Gruppe OR', SR', S(O)R', $S(O)_2R'$ N(R')(R"), P(R')(R"), wobei R' und R" die gleiche oder unterschiedliche $C_1$-$C_{25}$-Alkylgruppe, $C_3$-$C_{12}$-Cycloalkylgruppe, $C_1$-$C_{25}$-Alkoxygruppe, $C_2$-$C_{25}$-Alkenylgruppe, $C_1$-$C_{12}$-Perfluoralkylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe bedeuten, oder die mit der Herstellung von substituierten oder unsubstituierten zyklischen $C_4$-$C_{10}$ der polyzyklischen $C_4$-$C_{12}$-Systemen kombiniert werden können, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_2$-$C_{20}$-Heterozyklus, $C_4$-$C_{20}$-Heteroaryl, $C_5$-$C_{20}$-Heteroaryloxyl substituiert sein können, sie können auch eine substituierte Ester- (-COOR'), Amid- ($-CONR'_2$), Formyl- (-CHO), Keto- (-COR'), Hydroxamgruppe (-CON(OR')(R')) sein, worin R' $C_1$-$C_{12}$-Alkyl, $C_3$-$C_{12}$-Zykloalkyl, $C_2$-$C_{12}$-Alkenyl, $C_5$-$C_{20}$-Aryl bedeutet, die mit mindestens einem $C_1$-$C_{12}$-Alkyl, $C_1$-$C_{12}$-Perfluoralkyl, $C_1$-$C_{12}$-Alkoxyl, $C_5$-$C_{20}$-Aryl, $C_5$-$C_{24}$-Aryloxyl, $C_7$-$C_{24}$-Aralkyl, $C_2$-$C_{20}$-Heterozyklus, $C_4$-$C_{20}$-Heteroaryl, $C_5$-$C_{20}$-Heteroaryloxyl oder einem Halogenatom substituiert sein können;

R1, R2, R3, R4 unabhängig voneinander ein Wasserstoffatom, eine Alkoxygruppe (-OR'''), eine Sulfidgruppe (-SR'''), eine Cyangruppe (-CN), eine Sulfoxidgruppe (-S(O)R'''), eine Sulfonamidgruppe ($-SO_2NR'''_2$), eine Sulfongruppe (-SO2R'''), eine Phosphitgruppe (-P(O)(OR''')2), eine Phosphinatgruppe (-P(O)R'''(OR''')), eine Phosphonitgruppe (-P(OR''')2), eine Phosphingruppe (-PR'''2), eine Nitrogruppe ($-NO_2$), eine Nitrosogruppe (-NO), eine Carboxylgruppe (-COOH), eine Estergruppe (-COOR'''), eine Formylgruppe (-CHO), eine Ketogruppe (-COR'''), eine Amidgruppe (-CONR'''2), eine Imidgruppe (-CONR'''COR'''), eine Amingruppe (-NR'''2), eine Ammoniumgruppe ($-N+R'''_3$), eine Amidgruppe (-NR'''COR''') sind, wobei R''' C1-C5-Alkyl, C1-C5-Perfluoralkyl,

C5-C24-Aryl, $C_7$-$C_{24}$-Aralkyl, C5-C24-Perfluoraryl, C4-C20-Heteroaryl, C5-C20-Heteroaryloksyl bedeutet, und R1, R2, R3 und R4 kombiniert werden können, um ein zyklisches System zu bilden; einer Reaktion mit dem Carben mit der Formel **8aa** unterzogen wird

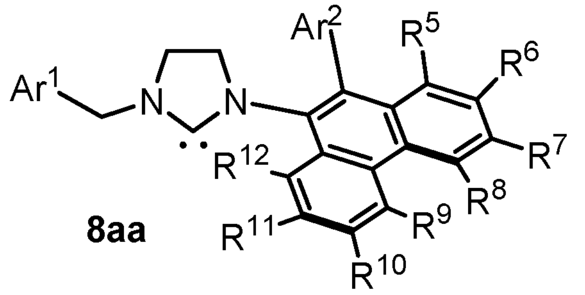

**8aa**

wobei:

$Ar_1$ i $Ar_2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;

$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, $C_3$-$C_{12}$-Cycloalkylgruppe, $C_1$-$C_5$-Perfluoralkylgruppe, $C_2$-$C_{12}$-Alkenylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.

6. Verfahren zur Herstellung der Verbindung mit der Formel 9 nach Anspruch 5, **dadurch gekennzeichnet, dass** der Alkyliden-Rutheniumkomplex mit der Formel 10

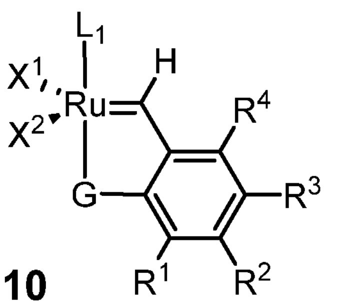

**10**

L1 einen inerten Liganden bedeutet, der aus einer Gruppe ausgewählt ist, die Pyridin oder substituiertes Pyridin, $P(R^b)_3$, $P(OR^b)_3$, $O(R^b)_2$, $N(R^b)_3$ einschließt, wobei jedes $R^b$ unabhängig voneinander C1-C12-Alkyl, $C_3$-$C_{12}$-Zykloalkyl, C5-C20-Aryl, C7-C24-Aralkyl, C5-C24-Perfluoraryl, 5-12-gliedriges Heteroaryl bedeutet;

X1 und X2 unabhängig voneinander anionische Liganden sind, X1 und X2 miteinander verbunden werden können, um ein zyklisches System zu bilden;

G ist das Halogenatom oder ein Substituent, ausgewählt aus der Gruppe OR', SR', S(O)R', $S(O)_2R'$ N(R')(R''), P(R')(R'')(R'''), wobei R', R'' und R''' die gleiche oder unterschiedliche C1-$C_{25}$-Alkylgruppe, C3-C12-Cycloalkylgruppe, C1-C25-Alkoxygruppe, $C_2$-$C_{25}$-Alkenylgruppe, C1-C12-Perfluoralkylgruppe, C5-C20-Arylgruppe, $C_5$-$C_{24}$-Aryloxygruppe, C2-C20-heterozyklische Gruppe, C4-C20-Heteroarylgruppe, C5-C20-Heteroaryloxylgruppe bedeuten,oder die mit der Herstellung von substituierten oder unsubstituierten zyklischen C4-C10 der polyzyklischen C4-C12-Systemen kombiniert werden können, die mit mindestens einem C1-C12-Alkyl, C1-C12-Perfluoralkyl, C1-C12-Alkoxyl, $C_5$-$C_{24}$-Aryloxyl, C2-C20-Heterozyklus, C4-C20-Heteroaryl, C5-C20-Heteroaryloxyl substituiert sein können, sie können auch eine substituierte Ester- (-COOR'), Amid- (-CONR'2), Formyl- (-CHO), Keto-(-COR'), Hydroxamgruppe (-CON(OR')(R')) sein, worin R' $C_1$-$C_{12}$-Alkyl, $C_3$-$C_{12}$-Zykloal-

kyl, $C_2$-$C_{12}$-Alkenyl, $C_5$-$C_{20}$-Aryl bedeutet, die mit mindestens einem C1-C12-Alkyl, C1-C12-Perfluoralkyl, C1-C12-Alkoxyl, C5-C20-Aryl, $C_5$-$C_{24}$-Aryloxyl, $C_7$-$C_{24}$-Aralkyl, C2-C20-Heterozyklus, C4-C20-Heteroaryl, C5-C20-Heteroaryloxyl oder einem Halogenatom substituiert sein können;

R1, R2, R3, R4 unabhängig voneinander ein Wasserstoffatom, eine Alkoxygruppe (-OR'''), eine Sulfidgruppe (-SR'''), eine Cyangruppe (-CN), eine Sulfoxidgruppe (-S(O)R'''), eine Sulfonamidgruppe (-SO2NR'''2), eine Sulfongruppe (-$SO_2$R'''), eine Phosphitgruppe (-P(O)(OR''')2), eine Phosphinatgruppe (-P(O)R'''(OR''')), eine Phosphonitgruppe (-P(OR''')2), eine Phosphingruppe (-PR'''2), eine Nitrogruppe (-NO2), eine Nitrosogruppe (-NO), eine Carboxylgruppe (-COOH), eine Estergruppe (-COOR'''), eine Formylgruppe (-CHO), eine Ketogruppe (-COR'''), eine Amidgruppe (-CONR'''2), eine Imidgruppe (-CONR'''COR'''), eine Amingruppe (-NR'''2), eine Ammoniumgruppe (-N+R'''$_3$), eine Amidgruppe (-NR'''COR''') sind, wobei R''' C1-C5-Alkyl, C1-C5-Perfluoralkyl, C5-C24-Aryl, C7-C24-Aralkyl, C5-C24-Perfluoraryl, C4-C20-Heteroaryl, C5-C20-Heteroaryloksyl bedeutet, und R1, R2, R3 und R4 kombiniert werden können, um ein zyklisches System zu bilden;

mit dem In-situ entstandenen Carben, das sich aus der Reaktion der Base ergibt, die aus Kalium- tert -amylat, Kalium-tert-butanolat, Kalium-N,N'-bis(trimethylsilyl)amid und Natriumhydrid ausgewählt ist, mit einem Carben-Ausgangsstoff der Formel 8a

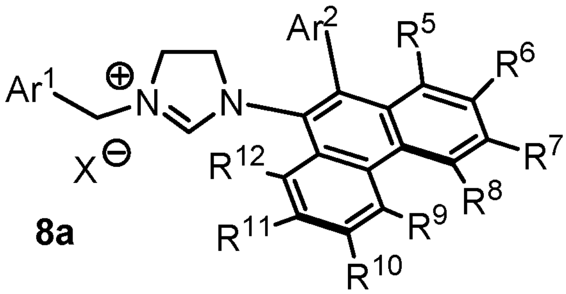

**8a**

wobei:

Ar$_1$ i Ar$_2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;

$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, eine $C_3$-$C_{12}$-Cycloalkylgruppe, eine $C_1$-$C_5$-Perfluoralkylgruppe, eine $C_2$-$C_{12}$-Alkenylgruppe, eine $C_5$-$C_{20}$-Arylgruppe, eine $C_5$-$C_{24}$-Aryloxygruppe, eine $C_2$-$C_{20}$-heterozyklische Gruppe, eine $C_4$-$C_{20}$-Heteroarylgruppe, eine $C_5$-$C_{20}$-Heteroaryloxylgruppe, eine $C_7$-$C_{24}$-Aralkylgruppe, eine $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.

X- steht für Halogenidanion, oder BF4-, PF6-, ClO4-.

7. Verfahren zur Herstellung der Verbindung mit der Formel 9 nach Anspruch 5, **dadurch gekennzeichnet, dass** die Carbene der Formel 8aa durch ihre thermische in-situ-Erzeugung aus geeigneten Carben-Ausgangsstoffen der Formel 8b als Addukte mit Chloroform, Kohlendioxid oder Alkohol der Reaktionsumgebung zugeführt werden,

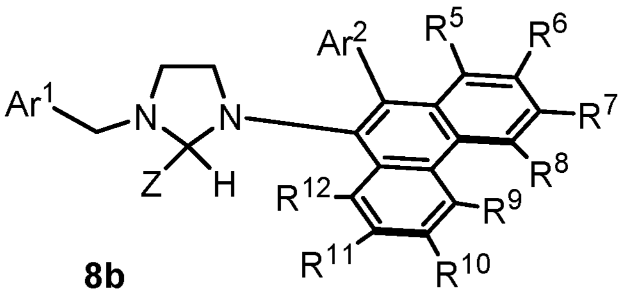

**8b**

wobei:

Z bedeutet CCl3, CO2 oder OR', worin R' C1-C12-Alkyl, $C_3$-$C_{12}$-Zykloalkyl, C2-C12-Alkyl oder $C_5$-$C_{20}$-Aryl bedeutet, das mit mindestens einem C1-C12-Alkyl, C1-C12-Perfluoralkyl, C1-C12-Alkoxyl, C5-C24-Aryoxyl, C5-C20-Heteroaryl oder einem Halogenatom substituiert sein kann;
$Ar_1$ i $Ar_2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;
$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, $C_3$-$C_{12}$-Cycloalkylgruppe, $C_1$-$C_5$-Perfluoralkylgruppe, $C_2$-$C_{12}$-Alkenylgruppe, $C_5$-$C_{20}$-Arylgruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.

**8.** Verfahren zur Herstellung der Verbindung mit der Formel 9 nach Anspruch 5, **dadurch gekennzeichnet, dass** der Alkyliden-Rutheniumkomplex der Formel 10 eine Verbindung der Formel 8c kontaktiert, die als NHC-Carbenliganden-Elektronendonor der Formel 8aa wirkt,

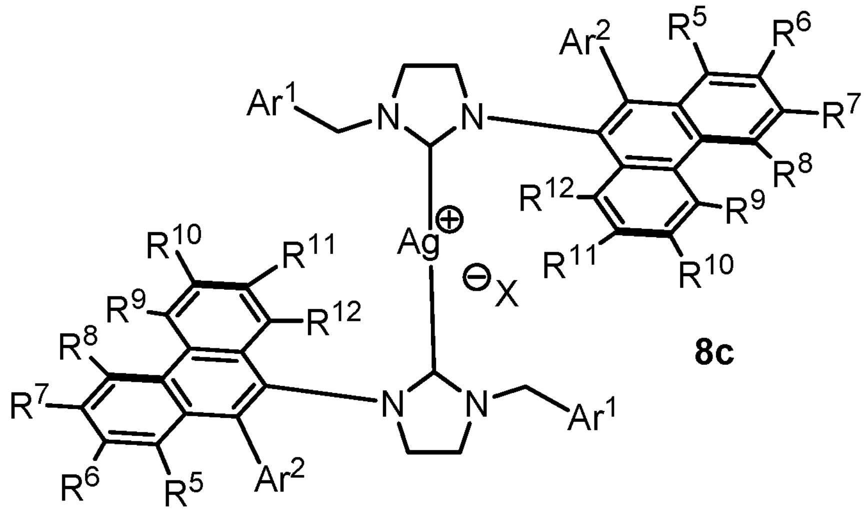

**8c**

wobei:

$Ar_1$ i $Ar_2$ unabhängig voneinander die $C_5$-$C_{20}$-Arylgruppe, $C_5$-$C_{24}$-Aryloxygruppe, $C_4$-$C_{20}$-Heteroarylgruppe, $C_5$-$C_{20}$-Heteroaryloxylgruppe, $C_5$-$C_{24}$-Perfluorarylgruppe sind, die mit Wasserstoffatomen oder alternativ mit mindestens einer $C_1$-$C_{12}$-Alkylgruppe, $C_1$-$C_{12}$-Perfluoralkylgruppe, $C_1$-$C_{12}$-Aryloxygruppe, $C_5$-$C_{24}$-Aryloxygruppe, $C_2$-$C_{20}$-heterozyklische Gruppe, $C_4$-$C_{20}$-Heteroarylgruppe, $C_5$-$C_{20}$-Heteroaryloxylgruppe, $C_7$-$C_{24}$-Aralkylgruppe, $C_5$-$C_{24}$-Perfluorarylgruppe oder einem Halogenatom substituiert sein kann;
$R^5$, $R^6$, $R^7$, $R^8$, $R^9$, $R^{10}$, $R^{11}$ i $R^{12}$ unabhängig voneinander ein Wasserstoffatom oder eine $C_1$-$C_{25}$-Alkylgruppe, eine $C_3$-$C_{12}$-Cycloalkylgruppe, eine $C_1$-$C_5$-Perfluoralkylgruppe, eine $C_2$-$C_{12}$-Alkenylgruppe, eine $C_5$-$C_{20}$-Arylgruppe, eine $C_5$-$C_{24}$-Aryloxygruppe, eine $C_2$-$C_{20}$-heterozyklische Gruppe, eine $C_4$-$C_{20}$-Heteroarylgruppe, eine $C_5$-$C_{20}$-Heteroaryloxylgruppe, eine $C_7$-$C_{24}$-Aralkylgruppe, eine $C_5$-$C_{24}$-Perfluorarylgruppe sind, die mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryloxyl, $C_4$-$C_{20}$-Heteroaryl, $C_5$-$C_{20}$-Heteroaryloxyl, oder einem Halogenatom substituiert sein können; und $R^5$ i $R^6$ und/oder $R^7$ und $R^8$ kombiniert werden können, um ein zyklisches System zu bilden.
X- steht für Halogenidanion, oder BF4-, PF6-, CI04-.

**9.** Verwendung einer Verbindung gemäß einem der Ansprüche 1-4 mit Formel 9 als Vorkatalysator und/oder Katalysator in der Olefinmetathese-Reaktion.

**10.** Verwendung gemäß dem Anspruch 9, wobei die Verbindung mit Formel 9 als Vorkatalysator und/oder Katalysator in der Ringschlussmetathese (RCM), Homometathese, Kreuzmetathese (CM), Ethenolyse, Isomerisierung, in der Metathese vom diastereoselektiven Umgruppierungstyp, der Alken-Alkin-Metathese (en-yn) oder in ROMP-Polymerisationsreaktionen verwendet wird.

**11.** Verwendung gemäß dem Anspruch 9 oder 10, wobei die Verbindung mit Formel 9 als Vorkatalysator und/oder Katalysator in einer metathetischen Ringöffnungspolymerisationsreaktion (ROMP) von Dicyclopentadien oder Norbornen verwendet wird.

**12.** Verwendung gemäß einem der Ansprüche 9-11, wobei die Verbindung mit Formel 9 als Vorkatalysator und/oder Katalysator in einem Reaktionsgemisch von 1 Minute bis 24 Stunden verwendet wird, und/oder die Reaktion in unpolaren oder nichtlösenden Lösungsmitteln durchgeführt wird, und/oder die Reaktion in einem organischen Lösungsmittel wie Toluol, Benzol, Mezytylen, Dichlormethan, Ethylacetat, Methylacetat, Tertbutylmethylether, Cyclopentylmethylether oder ohne Lösungsmittel durchgeführt wird, und/oder die Reaktion bei 20 bis 150 °C durchgeführt wird.

**13.** Verwendung gemäß einem der Ansprüche 9-12, obei Verbindung 9 in einer Menge von nicht mehr als 1 molar verwendet wird, und/oder Verbindung 9 dem Reaktionsgemisch portionsweise und/oder kontinuierlich unter Verwendung der Pumpe zugesetzt wird, und/oder Verbindung 9 dem Reaktionsgemisch in fester Form und/oder als Lösung in einem organischen Lösungsmittel zugesetzt wird, und/oder mindestens ein Olefin dem Reaktionsgemisch portionsweise und/oder kontinuierlich unter Verwendung der Pumpe zugesetzt wird.

**14.** Verwendung gemäß einem der Ansprüche 9 bis 13, bei der ein gasförmiges Nebenprodukt der Reaktion (Ethylen, Propylen, Butylen) aktiv aus dem Reaktionsgemisch durch Inertgas oder Vakuum entfernt wird.

**15.** Verwendung der Verbindung der Formel 9 als Substrat für die Synthese von anderen Rutheniumkomplexverbindungen, die Vorkatalysatoren und/oder Katalysatoren für die Olefinmetathese sind.

## Revendications

**1.** Le composé de formule 9

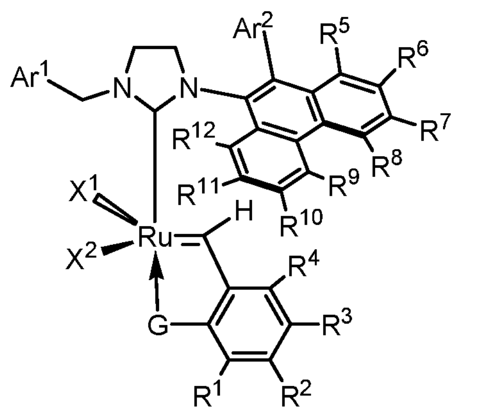

**9**

où:

$X^1$ et $X^2$ signifient indépendamment un ligand anionique, $X^1$ et $X^2$ peuvent être liés entre eux en formant un système cyclique;
G est un atome d'halogène ou un substituant choisi dans le groupe OR', SR', S(O)R', $S(O)_2R'$ N(R')(R"), P(R')(R"), où R' et R" signifient un groupe alkyle identique ou différent en $C_1$-$C_{25}$, groupe cycloalkyle en $C_3$-$C_{12}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, ou qui peuvent être liés entre eux en formant un système cyclique en $C_4$-$C_{10}$ substitué ou non substitué ou un système polycyclique en

$C_4$-$C_{12}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, peuvent être également substitués par un groupe ester (-COOR'), amide (-CONR'$_2$), formyle (-CHO), cétone (-COR'), hydroxamique (-CON(OR')(R')) où R' signifie alkyle en $C_1$-$C_{12}$, cycloalkyle en $C_3$-$C_{12}$, acényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryle en $C_5$-$C_{20}$, aryloxy en $C_5$-$C_{24}$, aralkyle en $C_7$-$C_{24}$, hétérocycle en $C_2$-$C_{20}$, hétéroaryle en $C_4$-$C_{20}$, hétéroaryloxy en $C_5$-$C_{20}$, ou un atome d'halogène;

$Ar^1$ et $Ar^2$ signifient indépendamment groupe aryle en $C_5$-$C_{20}$, aryloxy en $C_5$-$C_{24}$, hétéroaryle en $C_4$-$C_{20}$, hétéroaryloxy en $C_5$-$C_{20}$, perfluoroaryle en $C_5$-$C_{24}$, qui est substitué par les atomes d'hydrogène ou éventuellement est substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;

$R^1$, $R^2$, $R^3$, $R^4$ signifient indépendamment un atome d'hydrogène, groupe alcoxy (-OR'''), groupe sulfide (-SR'''), groupe cyano (-CN), groupe sulfoxyde (-S(O)R'''), groupe sulfonamide (-SO2NR'''2), groupe sulfonamide (-NR'''$SO_2$R'''), groupe sulfone (-$SO_2$R'''), sulfonium (-$S^+$R'''$_2$), groupe phosphonite (-P(O)(OR''')$_2$), groupe phosphinite (-P(O)R'''(OR''')), groupe phosphinate (-P(OR''')$_2$), groupe phosphine (-PR'''2), groupe phosphonine (-$P^+$R'''$_3$) grupe nitro (-$NO_2$), groupe nitroso (-NO), groupe carboxyle (-COOH), groupe ester (-COOR'''), groupe formyle (-CHO), groupe cétone (-COR'''), amide (-CONR'''$_2$), imide (-CONR'''COR'''), amine (-NR'''$_2$), ammonium (-$N^+$R'''$_3$), amide (-NR'''COR'''), où R''' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$, et en plus $R^1$, $R^2$, $R^3$ et $R^4$ peuvent être liés entre eux en formant un système cyclique;

$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$,hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique.

2. Le composé selon la revendication 1 de formule **9a**

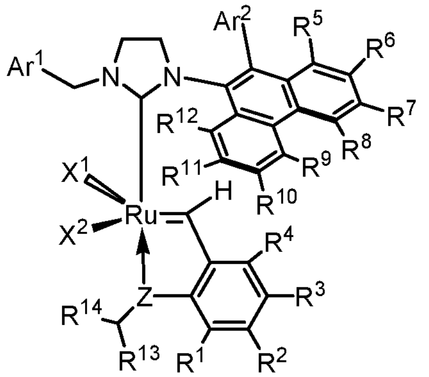

**9a**

$X^1$ et $X^2$ signifient indépendamment un atome d'halogène;

Z signifie O, S, NR' ou PR', où R' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$;

$Ar_1$ i $Ar_2$ signifient indépendamment un groupe aryle en $C_5$-$C_{20}$, hétéroaryle en $C_4$-$C_{20}$, qui est substitué ipar les atomes d'hydrogène ou est éventuellement substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;

$R^1$, $R^2$, $R^3$, $R^4$ signifient indépendamment un atome d'hydrogène, groupe alcoxy (-OR'''), groupe sulfide (-SR'''), groupe cyano (-CN), groupe sulfoxyde (-S(O)R'''), groupe sulfonamide (-$SO_2$NR'''$_2$), groupe sulfone (-$SO_2$R'''), groupe phosphonite (-P(O)(OR''')$_2$), groupe phosphinite (-P(O)R'''(OR''')), groupe phosphinate (-P(OR''')$_2$), groupe phosphine (-PR'''$_2$), grupe nitro (-$NO_2$), groupe nitroso (-NO), groupe carboxyle (-COOH), groupe ester (-COOR'''), groupe formyle (-CHO), groupe cétone (-COR'''), amide (-CONR'''$_2$), imide (-CONR'''COR'''), amine (-NR'''$_2$), ammonium (-$N^+$R'''$_3$), amide (-NR'''COR'''), où R''' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$,

et en plus $R^1$, $R^2$, $R^3$ et $R^4$ peuvent être liés entre eux en formant un système cyclique;
$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique;
$R^{13}$ et $R^{14}$, signifient indépendamment un atome d'hydrogène, un groupe alkyle en $C_1$-$C_{25}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en $C_5$-$C_{24}$, hétéroaryloxy en $C_5$-$C_{20}$, ou qui peuvent être liés entre eux en formant un système cyclique $C_4$-$C_{10}$ subsitué ou non substitué ou un système polycyclique $C_4$-$C_{12}$, peut également signifier un groupe ester (-COOR'), amide (-CONR'$_2$), formyle (-CHO), cétone (-COR'), hydroxamique (-CON(OR')(R')) ou un atome d'halogène, où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryloxy en $C_5$-$C_{20}$, ou un atome d'halogène;

**3.** Le composé selon la revendication 1 de formule **9b**

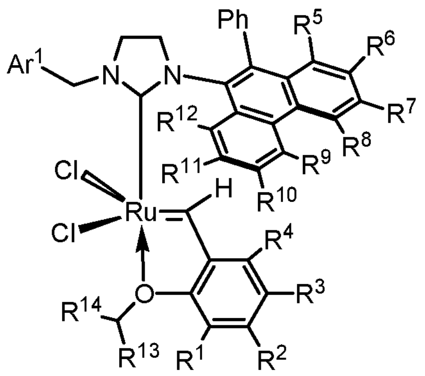

**9b**

$Ar_1$ signifie indépendamment un groupe aryle en $C_5$-$C_{20}$, hétéroaryle en $C_4$-$C_{20}$, qui est substitué par les atomes d'hydrogène ou est éventuellement substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;
$R^1$, $R^2$, $R^3$, $R^4$ signifient indépendamment un atome d'hydrogène, un groupe alcoxy (-OR'), groupe sulfide (-SR'), groupe cyano (-CN), groupe sulfoxyde (-S(O)R'), groupe sulfonamide (-SO$_2$NR'$_2$), groupe sulfone (-SO$_2$R), groupe phosphonite (-P(O)(OR')$_2$), groupe phosphinite (-P(O)R'(OR')), groupe phosphinate (-P(OR')$_2$), groupe phosphine (-PR'$_2$), grupe nitro (-NO$_2$), groupe nitroso (-NO), groupe carboxyle (-COOH), groupe ester (-COOR'), groupe formyle (-CHO), groupe cétone (-COR'), amide (-CONR'$_2$), imide (-CONR'COR'), amine (-NR'$_2$), ammonium (-N$^+$R'$_3$), amide (-NR'COR'), où R' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$, et en plus $R^1$, $R^2$, $R^3$ et $R^4$ peuvent être liés entre eux en formant un système cyclique;
$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$,cycolalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique;
$R^{13}$ et $R^{14}$, signifient indépendamment un atome d'hydrogène ou un groupe alkyle en $C_1$-$C_{25}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en -$C_{24}$, hétéroaryloxy en $C_5$-$C_{20}$, ou qui peuvent être liés entre eux en formant un système cyclique $C_4$-$C_{10}$ subsitué ou non substitué ou un système polycyclique $C_4$-$C_{12}$, peut également signifier un groupe ester (-COOR'), amide (-CONR'$_2$), formyle (-CHO), cétone (-COR'), hydroxamique (-CON(OR')(R')) ou un atome d'halogène, où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryloxy en $C_5$-$C_{20}$, ou un atome d'halogène;

4. Le composé selon la revendication 1 avec une structure représentée par une formule telle que **Ru-3:**

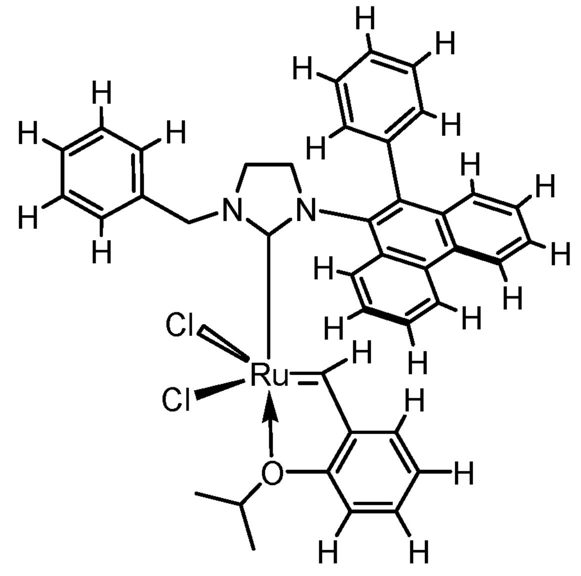

**Ru-3**

5. Le procédé de préparation d'un composé de formule 9

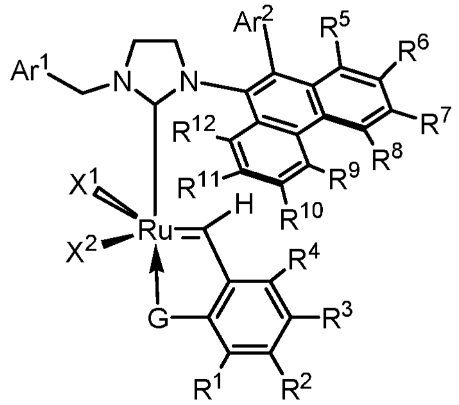

**9**

où:

$X^1$ et $X^2$ signifient indépendamment un ligand anionique, $X^1$ et $X^2$ peuvent être liés entre eux en formant un système cyclique;

G est un atome d'halogène ou un substituant choisi dans le groupe OR', SR', S(O)R', $S(O)_2R'$ N(R')(R"), P(R')(R"), où R' et R" signifient le groupe alkyle en $C_1$-$C_{25}$ identique ou différent, groupe cycloalkyle en $C_3$-$C_{12}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, ou qui peuvent être liés entre eux en formant un système cyclique $C_4$-$C_{10}$ substitué ou non substitué ou un système polycyclique en $C_4$-$C_{12}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, peuvent être également substitués par un groupe ester (-COOR'), amide (-$CONR'_2$), formyle (-CHO), cétone (-COR'), hydroxamique (-CON(OR')(R')) où R' signifie alkyle en $C_1$-$C_{12}$, cycloalkyle en $C_3$-$C_{12}$, acényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryle en $C_5$-$C_{20}$, aryloxy en $C_5$-$C_{24}$, aralkyle en $C_7$-$C_{24}$, hétérocycle en $C_2$-$C_{20}$, hétéroaryle en $C_4$-$C_{20}$, hétéroaryloxy en $C_5$-$C_{20}$, ou un atome d'halogène;

$Ar^1$ et $Ar^2$ signifient indépendamment groupe aryle en $C_5$-$C_{20}$, aryloxy en $C_5$-$C_{24}$, hétéroaryle en $C_4$-$C_{20}$, hété-

roaryloxy en $C_5$-$C_{20}$, perfluoroaryle en $C_5$-$C_{24}$, qui est substitué par les atomes d'hydrogène ou éventuellement est substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;

$R^1$, $R^2$, $R^3$, $R^4$ signifient indépendamment un atome d'hydrogène, groupe alcoxy (-OR'''), groupe sulfide (-SR'''), groupe cyano (-CN), groupe sulfoxyde (-S(O)R'''), groupe sulfonamide (-$SO_2NR'''_2$), groupe sulfonamide (-NR'''$SO_2$R'''), groupe sulfone (-$SO_2$R'''), sulfonium (-$S^+R'''_2$), groupe phosphonite (-P(O)$(OR''')_2$), groupe phosphinite (-P(O)R'''(OR''')), groupe phosphinate (-P$(OR''')_2$), groupe phosphine (-$PR'''_2$), groupe phosphonine (-$P^+R'''_3$) groupe nitro (-$NO_2$), groupe nitroso (-NO), groupe carboxyle (-COOH), groupe ester (-COOR'''), groupe formyle (-CHO), groupe cétone (-COR'''), amide (-$CONR'''_2$), imide (-CONR'''COR'''), amine (-$NR'''_2$), ammonium (-$N^+R'''_3$), amide (-NR'''COR'''), où R''' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$, et en plus $R^1$, $R^2$, $R^3$ et $R^4$ peuvent être liés entre eux en formant un système cyclique;

$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique.

**caractérisé en ce que** le complexe alkylidène ruthénium de formule **10**

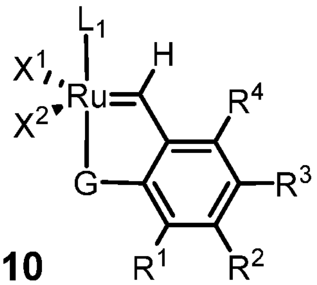

**10**

où:

$L^1$ signifie un ligand neutre choisi dans le groupe constitant en pyridine ou pyridine substituée, $P(R')_3$, $P(OR')_3$, $O(R')_2$, $N(R')_3$, où chaque R' signifie indépendamment alkyle en $C_1$-$C_{12}$, cycloalkyle en $C_3$-$C_{12}$, aryle en $C_5$-$C_{20}$, aralkyle en $C_7$-$C_{24}$, perfluoroaryle en $C_5$-$C_{24}$, hétéroaryle à 5-12 chaînons;

$X^1$ et $X^2$ signifient indépendamment un ligand anionique, $X^1$ et $X^2$ peuvent être liés entre eux en formant un système cyclique;

G est un atome d'halogène ou un substituant choisi dans le groupe OR', SR', S(O)R', $S(O)_2R'$ N(R')(R"), P(R')(R"), où R' et R" signifient le groupe alkyle en $C_1$-$C_{25}$ identique ou différent, groupe cycloalkyle en $C_3$-$C_{12}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, ou qui peuvent être liés entre eux en formant un système cyclique $C_4$-$C_{10}$ substitué ou non substitué ou un système polycyclique en $C_4$-$C_{12}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, peuvent être également substitués par un groupe ester (-COOR'), amide (-$CONR'_2$), formyle (-CHO), cétone (-COR'), hydroxamique (-CON(OR')(R')) où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryle en $C_5$-$C_{20}$, aryloxy en $C_5$-$C_{24}$, aralkyle en $C_7$-$C_{24}$, hétérocycle en $C_2$-$C_{20}$, hétéroaryle en $C_4$-$C_{20}$, hétéroaryloxy en $C_5$-$C_{20}$, ou un atome d'halogène;

$R^1$, $R^2$, $R^3$, $R^4$, signifient indépendamment un atome d'hydrogène, groupe alcoxy (-OR'''), groupe sulfide (-SR'''), groupe cyano (-CN), groupe sulfoxyde (-S(O)R'''), groupe sulfonamide (-$SO_2NR'''_2$), groupe sulfone (-$SO_2$R'''), groupe phosphonite (-P(O)$(OR''')_2$), groupe phosphinite (-P(O)R'''(OR''')), groupe phosphinate (-P$(OR''')_2$), groupe phosphine (-$PR'''_2$), groupe nitro (-$NO_2$), groupe nitroso (-NO), groupe carboxyle (-COOH), groupe ester (-COOR'''), groupe formyle (-CHO), groupe cétone (-COR'''), amide (-$CONR'''_2$), imide (-CONR'''COR'''), amine (-$NR'''_2$), ammonium (-$N^+R'''_3$), amide (-NR'''COR'''), où R''' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$,

et en plus $R^1$, $R^2$, $R^3$ et $R^4$ peuvent être liés entre eux en formant un système cyclique;
réagit avec carbène de formule **8aa**

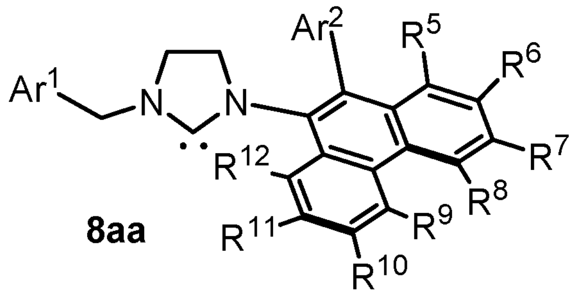

**8aa**

où:

$Ar_1$ et $Ar_2$ signifient indépendamment groupe aryle en $C_5$-$C_{20}$, aryloxy en $C_5$-$C_{24}$, hétéroaryle en $C_4$-$C_{20}$, hétéroaryloxy en $C_5$-$C_{20}$, perfluoroaryle en $C_5$-$C_{24}$, qui est substitué par les atomes d'hydrogène ou éventuellement est substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;
$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui éventuellement sont substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique.

**6.** Le procédé de préparation d'un composé de formule **9** selon la revendication 5, **caractérisé en ce que** le complexe alkylidène ruthénium de formule **10**

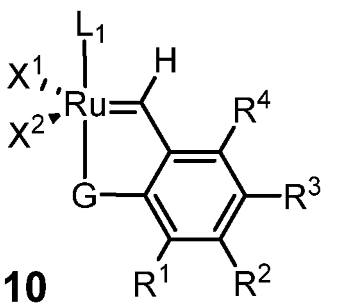

**10**

$L^1$ signifie un ligand neutre choisi dans le groupe constitant en pyridine ou pyridine substituée, $P(R^b)_3$, $P(OR^b)_3$, $O(R^b)_2$, $N(R^b)_3$, où chaque $R^b$ signifie indépendamment alkyle en $C_1$-$C_{12}$, cycloalkyle en $C_3$-$C_{12}$, aryle en $C_5$-$C_{20}$, aralkyle en $C_7$-$C_{24}$, perfluoroaryle en $C_5$-$C_{24}$, hétéroaryle à 5-12 chaînons;
$X^1$ et $X^2$ signifient indépendamment un ligand anionique, $X^1$ et $X^2$ peuvent être liés entre eux en formant un système cyclique;
G est un atome d'halogène ou un substituant choisi dans le groupe OR', SR', S(O)R', $S(O)_2R'$ N(R')(R"), P(R')(R")(R"'), où R', R" et R"' signifient le groupe alkyle en $C_1$-$C_{25}$ identique ou différent, groupe cycloalkyle en $C_3$-$C_{12}$, groupe alcoxy en $C_1$-$C_{25}$, groupe alcényle en $C_2$-$C_{25}$, perfluoroalkyle en $C_1$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, ou qui peuvent être liés entre eux en formant un système cyclique $C_4$-$C_{10}$ substitué ou non substitué ou un système polycyclique en $C_4$-$C_{12}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, peuvent être également substitués par un groupe ester (-COOR'), amide (-CONR'$_2$), formyle (-CHO), cétone (-COR'), hydroxamique (-CON(OR')(R')) où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryle en $C_5$-$C_{20}$, aryloxy en $C_5$-$C_{24}$, aralkyle en $C_7$-$C_{24}$, hétérocycle en $C_2$-$C_{20}$,

hétéroaryle en $C_4$-$C_{20}$, hétéroaryloxy en $C_5$-$C_{20}$, ou un atome d'halogène;
$R^1$, $R^2$, $R^3$, $R^4$ signifient indépendamment un atome d'hydrogène, groupe alcoxy (-OR'''), groupe sulfide (-SR'''), groupe cyano (-CN), groupe sulfoxyde (-S(O)R'''), groupe sulfonamide ($-SO_2NR'''_2$), groupe sulfone ($-SO_2R'''$), groupe phosphonite ($-P(O)(OR''')_2$), groupe phosphinite (-P(O)R'''(OR''')), groupe phosphinate ($-P(OR''')_2$), groupe phosphine ($-PR'''_2$), groupe nitro ($-NO_2$), groupe nitroso (-NO), groupe carboxyle (-COOH), groupe ester (-COOR'''), groupe formyle (-CHO), groupe cétone (-COR'''), amide ($-CONR'''_2$), imide (-CONR'''COR'''), amine ($-NR'''_2$), ammonium ($-N^+R'''_3$), amide (-NR'''COR'''), où R''' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$, hétéroaryle en $C_4$-$C_{20}$, hétéroaryloxy en $C_5$-$C_{20}$, et en plus $R^1$, $R^2$, $R^3$ et $R^4$ peuvent être liés entre eux en formant un système cyclique;
réagit avec carbène formé *in situ* par l'action d'une base choisie parmi telles que *tert*-amylate de potassium, *tert*-butylate de potassium, *N,N'*-bis (triméthylsilyl) amide de potassium, hydrure de sodium, sur le précurseur de carbène de formule **8a**

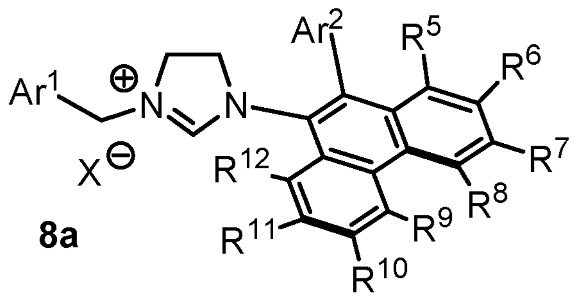

**8a**

où:

$Ar_1$ et $Ar^2$ signifient indépendamment groupe aryle en $C_5$-$C_{20}$, aryloxy en $C_5$-$C_{24}$, hétéroaryle en $C_4$-$C_{20}$, hétéroaryloxy en $C_5$-$C_{20}$, perfluoroaryle en $C_5$-$C_{24}$, qui est substitué par les atomes d'hydrogène ou est éventuellement substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;
$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui éventuellement sont substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique.
$X^-$ signifie un anion halogénure ou $BF_4^-$, $PF_6^-$, $ClO_4^-$.

7. Le procédé de préparation d'un composé de formule **9** selon la revendication 5, **caractérisé en ce que** les carbènes de formule **8aa** sont apportés au milieu réactionnel par leur génération thermique *in situ* à partir des précurseurs des carbènes correspondants de formule **8b** qui sont des adduits avec chloroforme, dioxyde de carbone ou alcool, ,

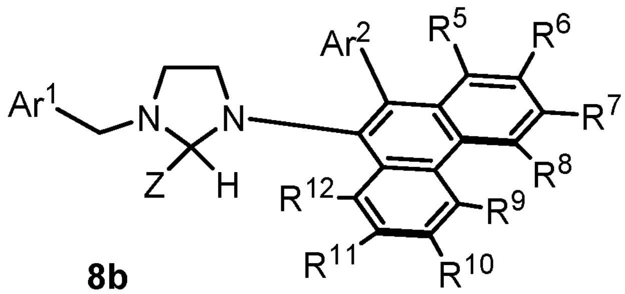

**8b**

où:

Z signifie $CCl_3$, $CO_2$ ou OR' où R' signifie alkyle en $C_1$-$C_{12}$, cycloalkyel en $C_3$-$C_{12}$, alcényle en $C_2$-$C_{12}$, ou aryle en $C_5$-$C_{20}$, qui est éventuellement substitué par au moins un alkyle en $C_1$-$C_{12}$, perfluoroalkyle en $C_1$-$C_{12}$, alcoxy

en $C_1$-$C_{12}$, aryloxy en $C_5$-$C_{24}$, hétéroaryloxy en $C_5$-$C_{20}$ ou un atome d'halogène;

$Ar_1$ et $Ar^2$ signifient indépendamment groupe aryle en $C_5$-$C_{20}$, aryloxy en $C_5$-$C_{24}$, hétéroaryle en $C_4$-$C_{20}$, hétéroaryloxy en $C_5$-$C_{20}$, perfluoroaryle en $C_5$-$C_{24}$, qui est substitué par les atomes d'hydrogène ou est éventuellement substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;

$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique.

8. Le procédé de préparation d'un composé de formule **9** selon la revendication 5, **caractérisé en ce que** le complexe alkylidène ruthénium de formule **10,** est en contact avec le composé de formule **8c,** qui agit comme donneur du ligand carbène NHC de formule **8aa,**

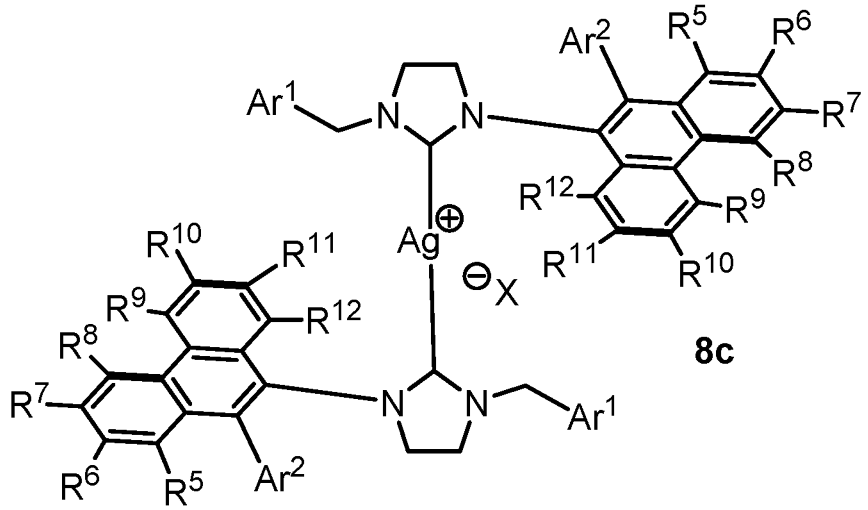

**8c**

où:

$Ar_1$ et $Ar^2$ signifient indépendamment groupe aryle en $C_5$-$C_{20}$, aryloxy en $C_5$-$C_{24}$, hétéroaryle en $C_4$-$C_{20}$, hétéroaryloxy en $C_5$-$C_{20}$, perfluoroaryle en $C_5$-$C_{24}$, qui est substitué par les atomes d'hydrogène ou est éventuellement substitué par au moins un groupe alkyle en $C_1$-$C_{12}$, perfluoroalkyle en $C_1$-$C_{12}$, alcoxy en $C_1$-$C_{12}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, ou un atome d'halogène;

$R^5$, $R^6$, $R^7$, $R^8$, $R^9$, $R^{10}$, $R^{11}$ et $R^{12}$ signifient indépendamment un atome d'hydrogène ou un groupe alkyle en $C_1$-$C_{25}$, cycloalkyle en $C_3$-$C_{12}$, perfluoroalkyle en $C_1$-$C_5$, alcényle en $C_2$-$C_{12}$, aryle en $C_5$-$C_{20}$, aryloxy en $C_5$-$C_{24}$, hétérocycle en $C_2$-$C_{20}$, hétéroaryle en $C_4$-$C_{20}$, hétéroaryloxy en $C_5$-$C_{20}$, aralkyle en $C_7$-$C_{24}$, perfluoroaryle en $C_5$-$C_{24}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryle en $C_4$-$C_{20}$, hétéroaryloxy en $C_5$-$C_{20}$, ou un atome d'halogène; en plus $R^5$ et $R^6$ et/ou $R^7$ et $R^8$ peuvent être liés entre eux en formant un système cyclique. $X^-$ signifie un anion halogénure ou $BF_4^-$, $PF_6^-$, $ClO_4^-$.

9. L'utilisation d'un composé selon l'une quelconque des revendications 1 à 4, de formule **9** comme précatalyseur et / ou catalyseur dans les réactions de métathèse des oléfines.

10. L'utilisation selon la revendiction 9, où le composé de formule **9** est utilisé comme précatalyseur et / ou catalyseur dans les réactions de métathèse de fermeture de cycle (RCM), homométhatèse, méthatèse croisée (CM), éthénolyse, isomérisation, dans la réaction de métathèse diastéréosélective de réarrangement de cycle (DRRM), métathèse type "alcène-alcyne" (ène-yne) ou dans les réactions de polymérisation type ROMP.

**11.** L'utilisation selon la revendiction 9 ou 10, où le composé de formule **9** est utilisé comme précatalyseur et / ou catalyseur dans la réaction de métathèse de polymérisation par ouverture de cycle (ROMP) du dicyclopentadiène ou du norbornène.

**12.** L'utilisation selon l'une quelconque des revendications 9 à 11, où le composé de formule **9** est utilisé comme précatalyseur et / ou catalyseur dans le mélange réactionnel pendant la durée d'une minute à 24 heures, et/ou la réaction est réalisée dans des solvants non polaires ou sans solvant, et / ou la réaction est réalisée dans un solvant organique tel que le toluène, le benzène, le mésitylène, le dichlorométhane, l'acétate d'éthyle, l'acétate de méthyle, le tert-butyl méthyl éther, le cyclopentyl méthyl éther, ou sans solvant, et / ou la réaction est réalisée à une température de 20 à 150 ° C.

**13.** L'utilisation selon l'une quelconque des revendications 9 à 12, où le composé de formule **9** est utilisé en une quantité ne dépassant pas 1 mole%, et/ou le composé **9** est ajouté au mélange réactionnel par portions et / ou en continu à l'aide d'une pompe, et/ou le composé **9** est ajouté au mélange réactionnel sous forme solide et / ou en solution dans un solvant organique, et / ou au moins une oléfine est ajoutée au mélange réactionnel par portions et / ou en continu à l'aide d'une pompe.

**14.** L'utilisation selon l'une quelconque des revendications 9 à 13, où le sous-produit gazeux de la réaction (éthylène, propylène, butylène) est activement éliminé du mélange réactionnel au moyen d'un gaz inerte ou d'un vide.

**15.** L'utilisation du composé de formule **9,** comme substrat pour la synthèse d'autres composés complexes du ruthénium qui sont des précatalyseurs et / ou des catalyseurs pour la métathèse des oléfines.

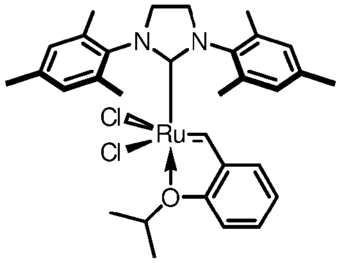

Ru-1

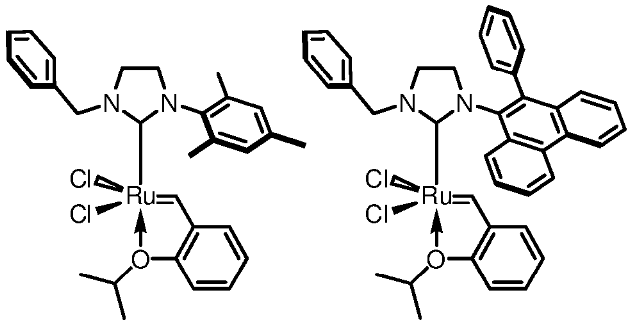

Ru-2

Ru-3

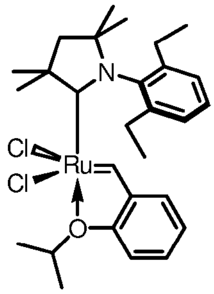

Ru-4

Fig. 1

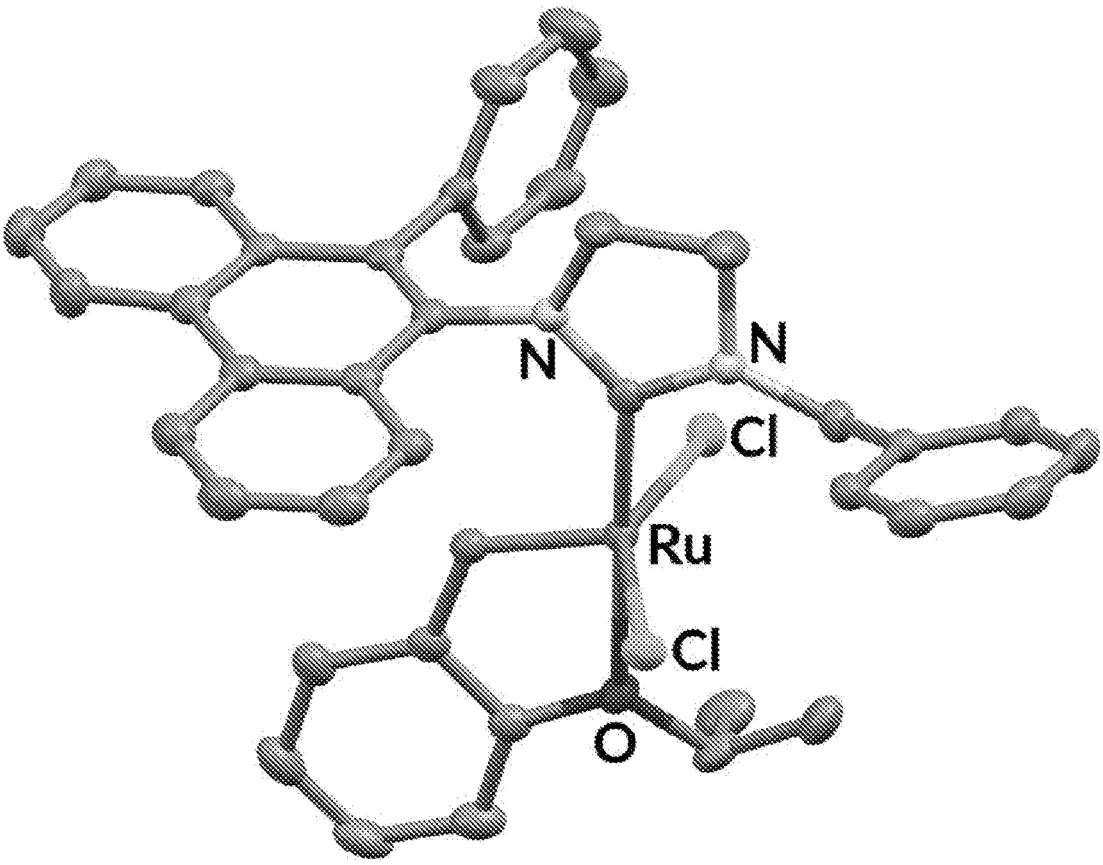

Fig. 2

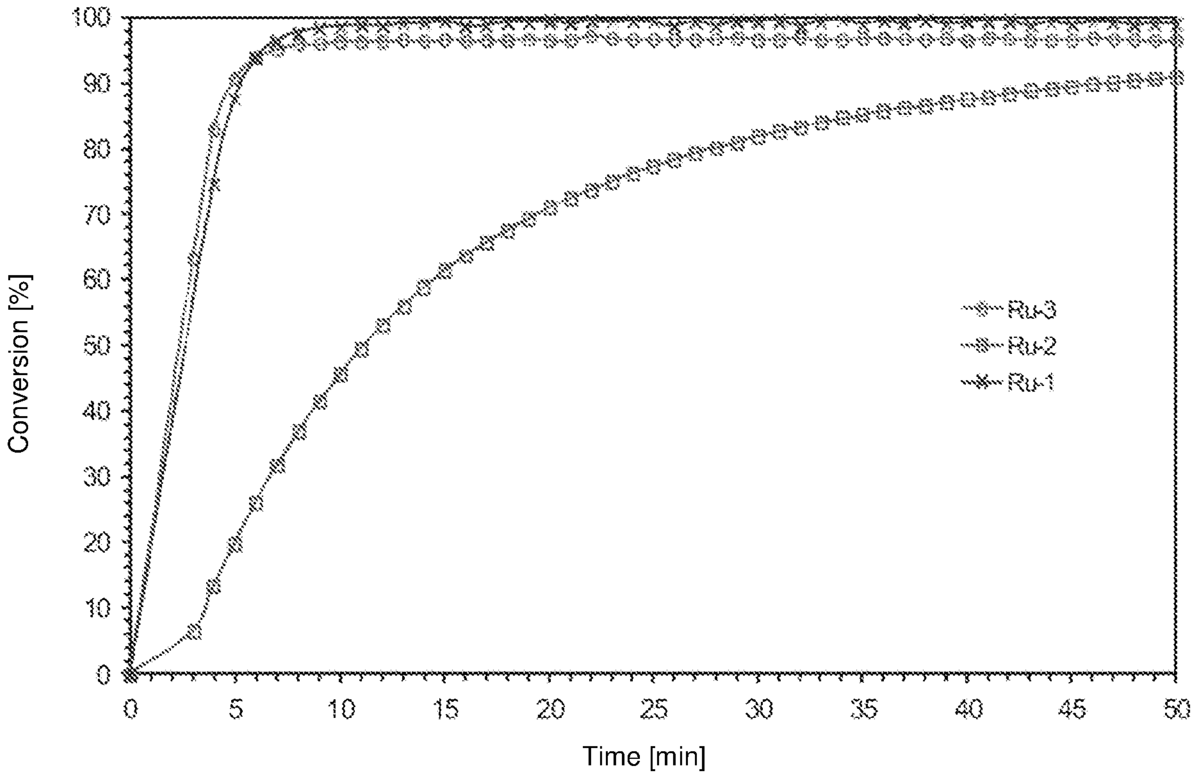
100
90
80
70
60
50
40
30
20
10
0
Conversion [%]
0
5
10
15
20
25
30
35
40
45
50
Time [min]
Ru-3
Ru-2
Ru-1

Fig. 3

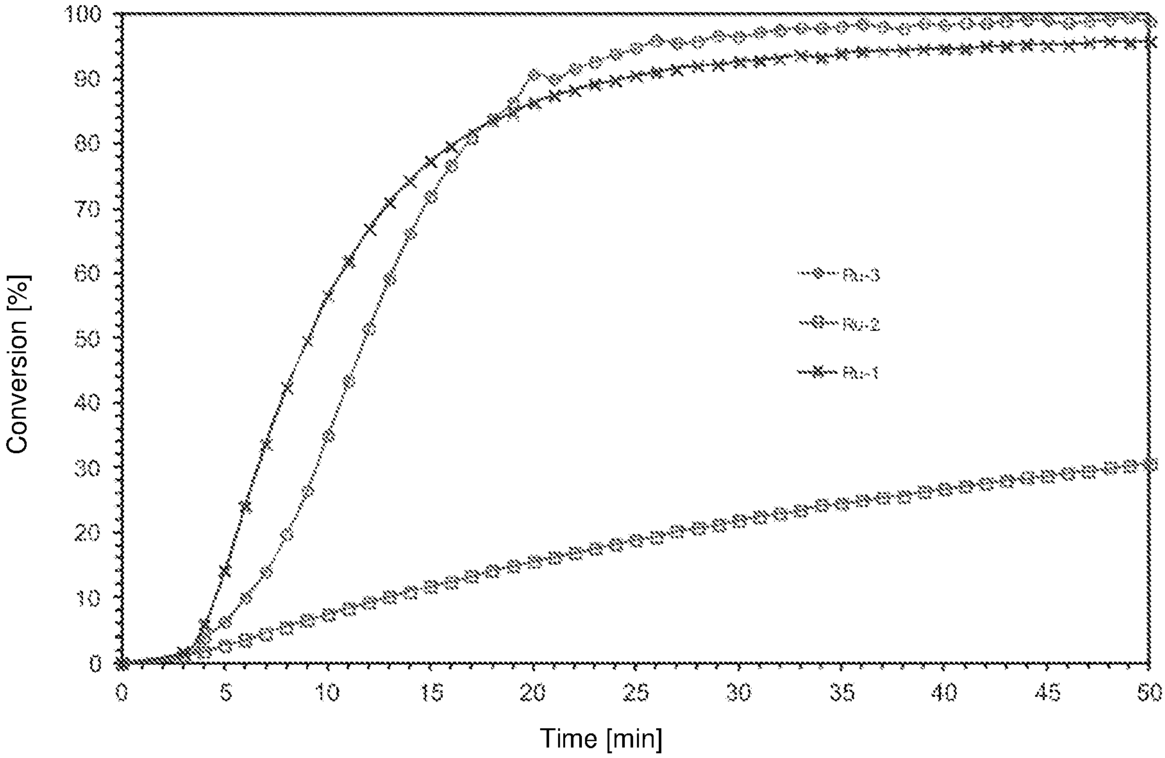
100
90
80
70
60
50
40
30
20
10
0
Conversion [%]
0
5
10
15
20
25
30
35
40
45
50
Time [min]
Ru-3
Ru-2
Ru-1

Fig. 4

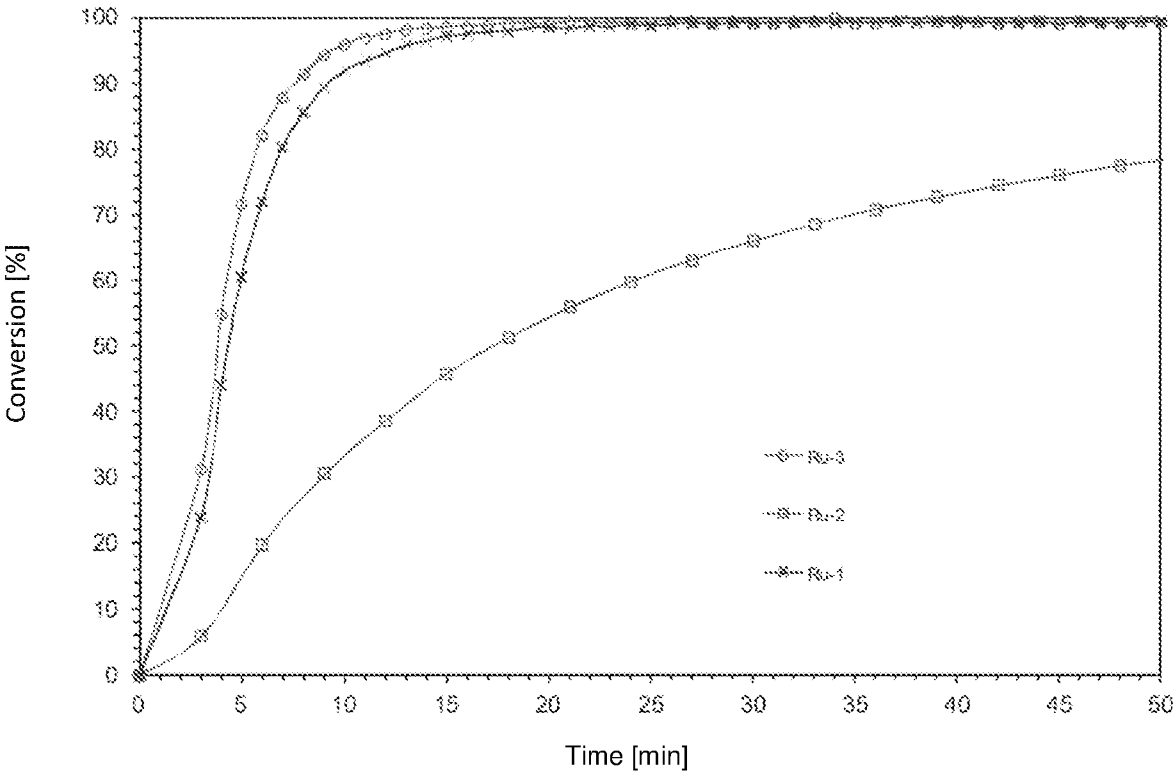
100
90
80
70
60
50
40
30
20
10
0
Conversion [%]
Ru-3
Ru-2
Ru-1
0
5
10
15
20
25
30
35
40
45
50
Time [min]

Fig. 5

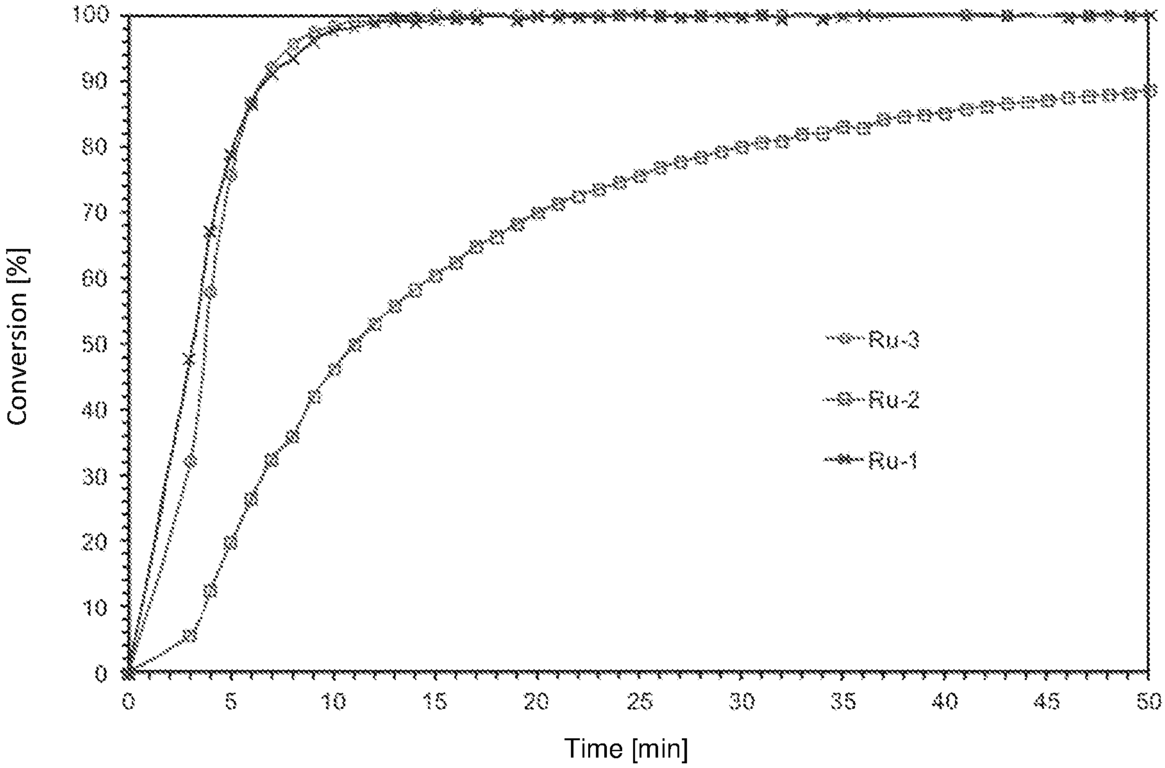
100
90
80
70
60
50
40
30
20
10
0
Conversion [%]
0
5
10
15
20
25
30
35
40
45
50
Time [min]
Ru-3
Ru-2
Ru-1

Fig. 6

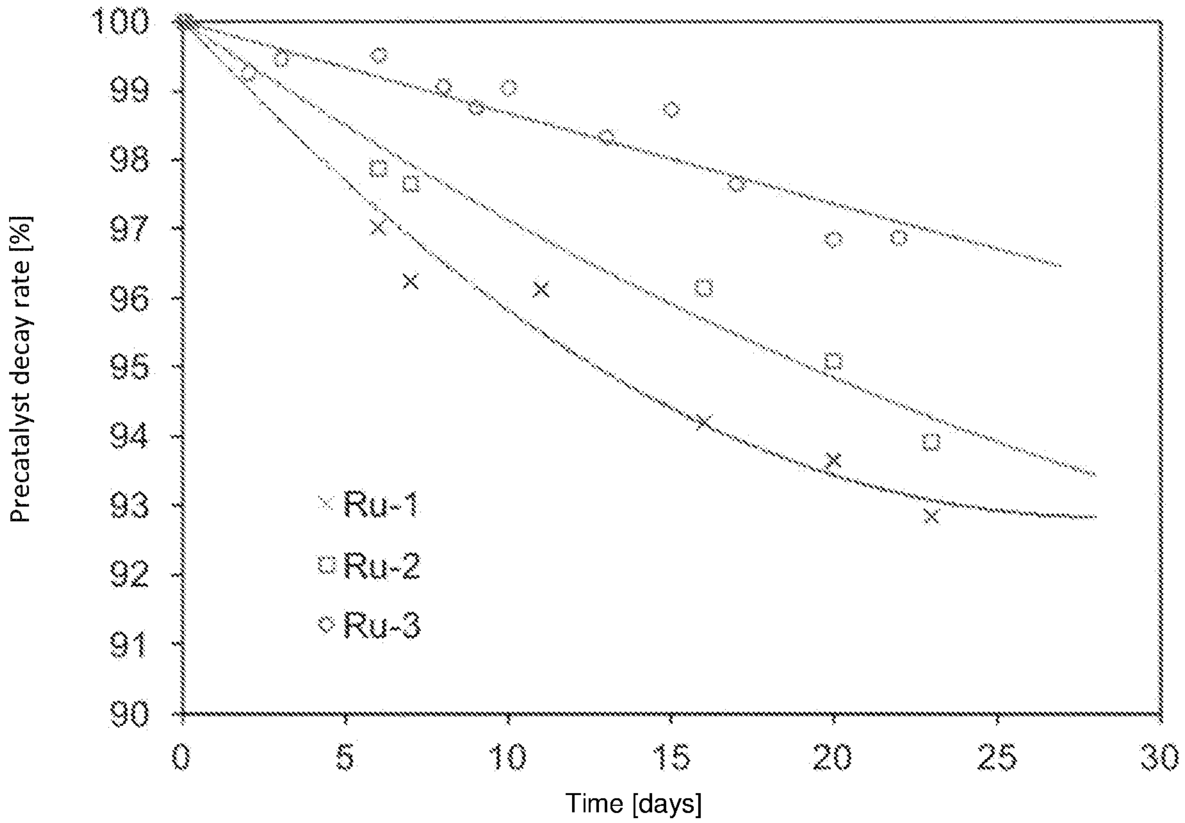
Precatalyst decay rate [%]
100
99
98
97
96
95
94
93
92
91
90
0
5
10
15
20
25
30
Time [days]
Ru-1
Ru-2
Ru-3

Fig. 7

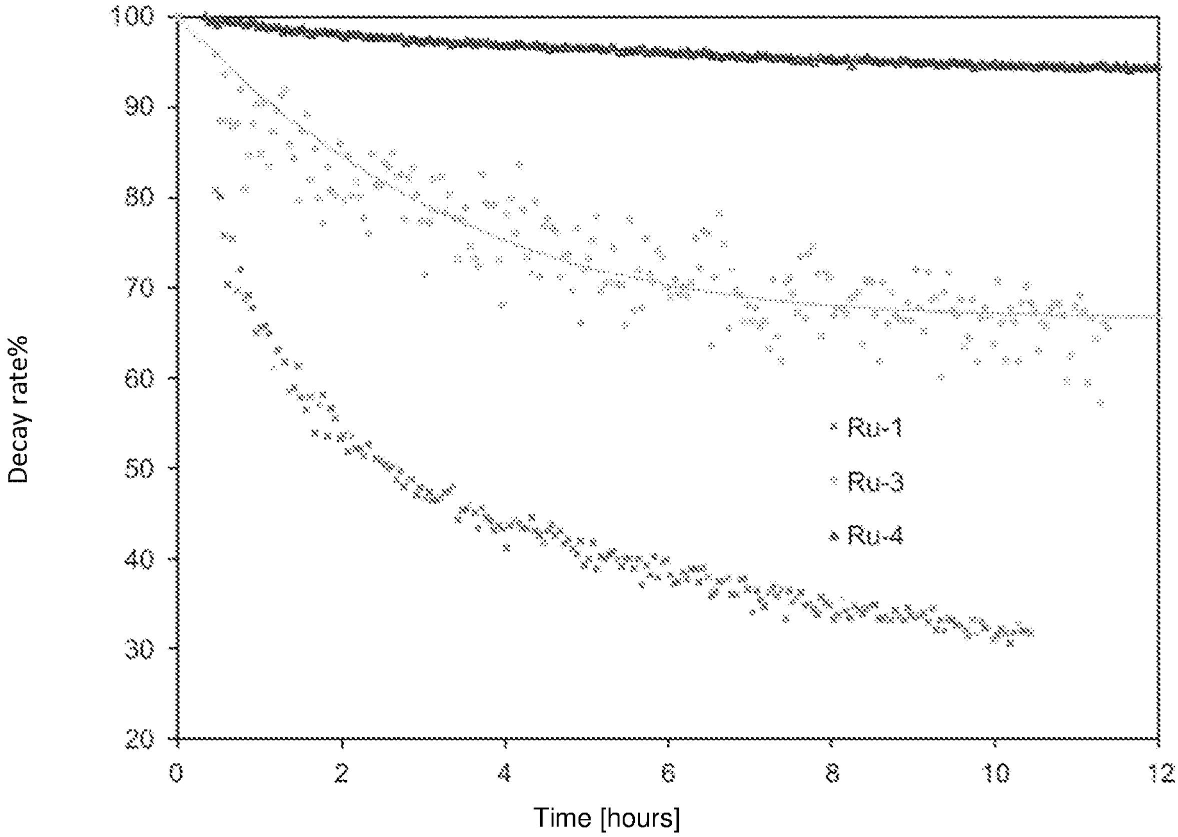

Decay rate%
Time [hours]
100
90
80
70
60
50
40
30
20
0
2
4
6
8
10
12
Ru-1
Ru-3
Ru-4


Fig. 8

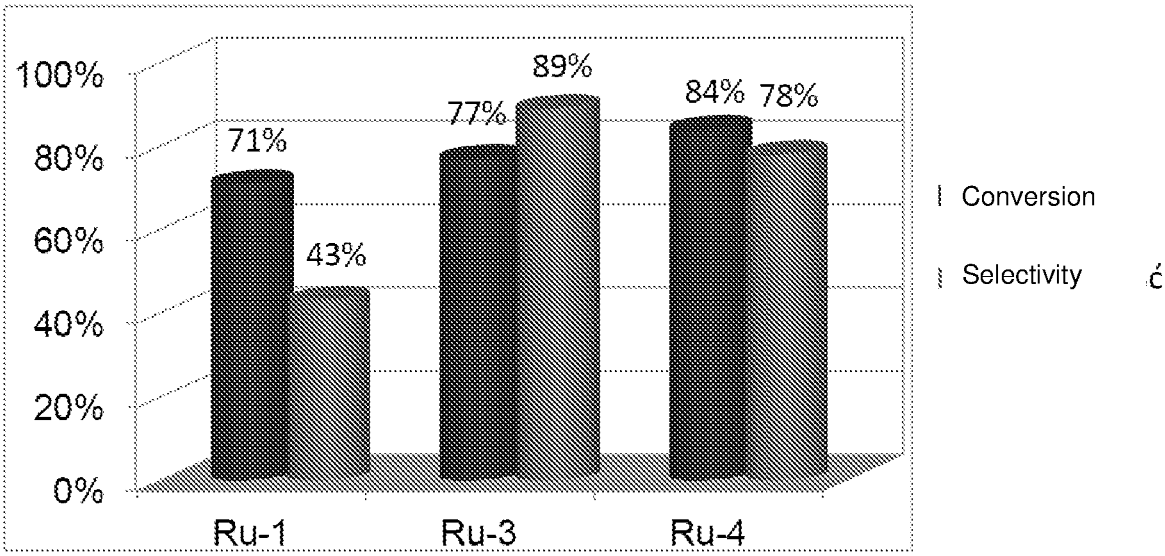
100%
80%
60%
40%
20%
0%
71%
43%
77%
89%
84%
78%
Ru-1
Ru-3
Ru-4
Conversion
Selectivity

Fig. 9a

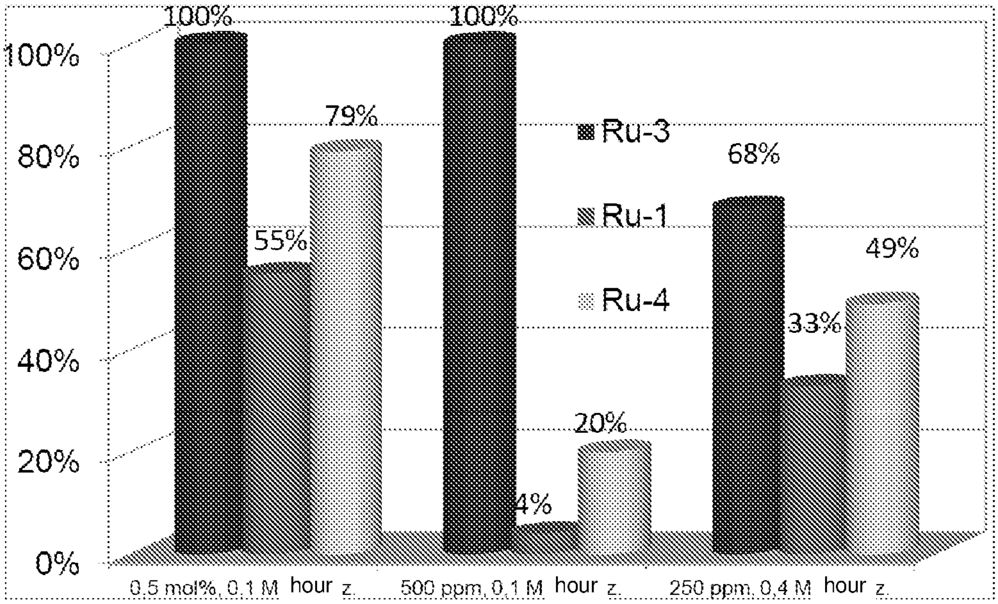
100%
80%
60%
40%
20%
0%
100%
55%
79%
100%
4%
20%
68%
33%
49%
Ru-3
Ru-1
Ru-4
0.5 mol%, 0.1 M hour
500 ppm, 0.1 M hour
250 ppm, 0.4 M hour

Fig. 9b

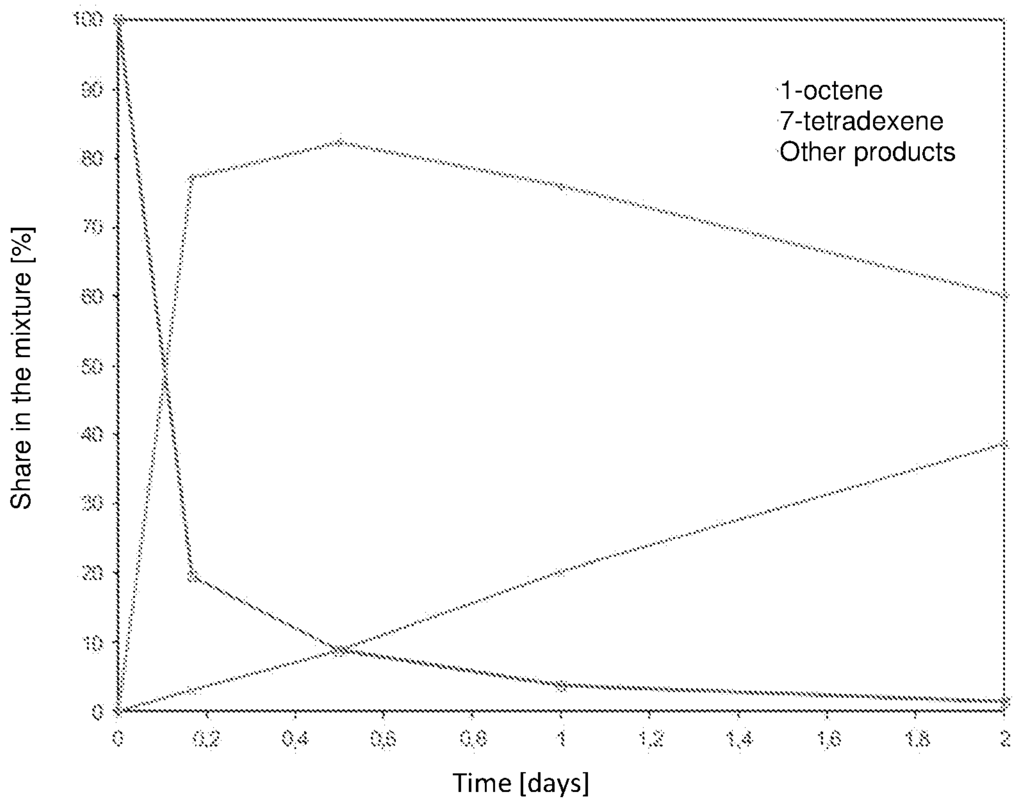


Fig. 10a

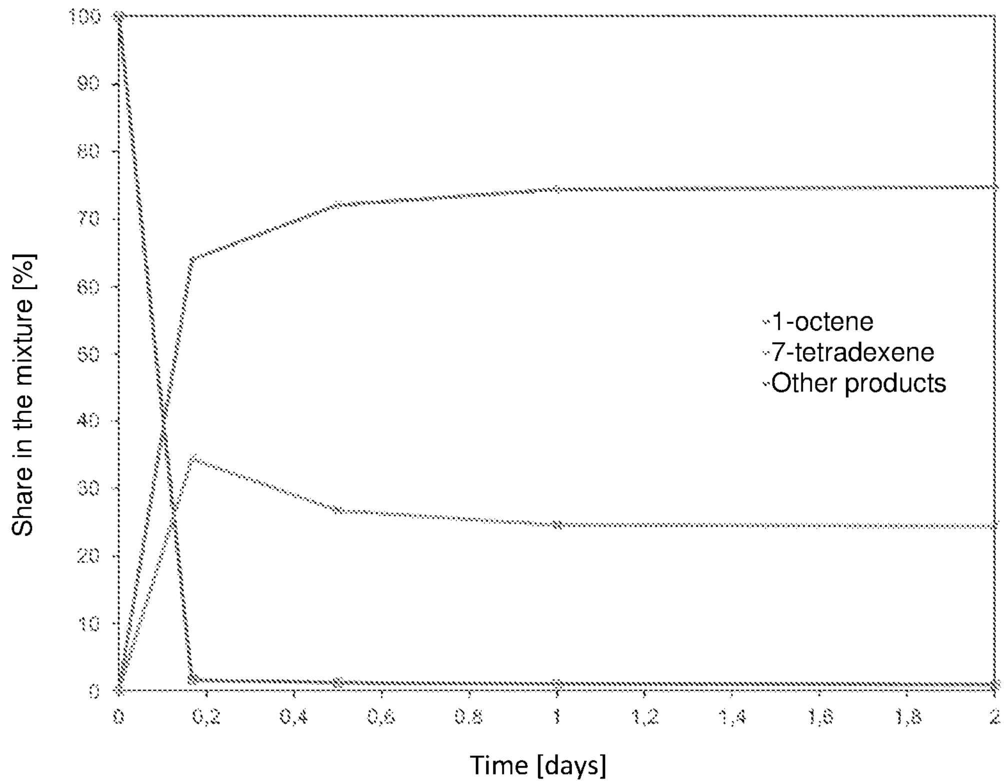


Fig. 10b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014027040 A1
- WO 2016092424 A1
- WO 2008010961 A2


### Non-patent literature cited in the description


- Handbook of Olefin Metathesis. John Wiley & Sons, Inc, 2015, vol. 3, 1608
- **GRUBBS et al.** *Chem. Rev.,* 2010, vol. 110, 1746-1787
- **NOLAN et al.** *Chem. Commun.,* 2014, vol. 50, 10355-10375
- **L. BENHAMOU ; E. CHARDON ; GUY LAVIGNE ; S. BELLEMIN-LAPONNAZ ; V. CÉSAR.** *Chem. Rev.,* 2011, vol. 111, 2705-2733
- **GRUBBS et al.** *Org. Lett.,* 2007, vol. 9, 1589-1592
- **GRUBBS et al.** *Org. Lett.,* 2008, vol. 10
- **GRELA et al.** *Chem. Commun.,* 2013, vol. 49, 3188-3190
- **GRUBBS et al.** *J. Am Chem. Soc.,* 2006, vol. 128, 11768-11769
- **MOL et al.** *Adv. Synth. Catal.,* 2002, vol. 344, 671-677
- **GRUBBS et al.** *Organometallics,* 2006, vol. 25, 5740-5745
- *Org. Lett.,* 2008, vol. 10, 441-444
- **GRUBBS et al.** *Org. Lett.,* 2007, vol. 9, 1339-1342
- **GRUBBS et al.** *J. Am. Chem. Soc.,* 2006, vol. 128, 1840-1846
- *Chem. Eur. J.,* 2008, vol. 14, 7545-7556
- **VERPOORT et al.** *Chem. Eur. J.,* 2006, vol. 12, 4654-4661
- **VERPOORT et al.** *Adv. Synth. Catal.,* 2007, vol. 349, 1692-1700
- **MAUDUIT et al.** *ACS Catalysis,* 2016, vol. 6, 7970-7976
- **GRELA et al.** *Organometallics,* 2012, vol. 31, 7316-7319
- **GRELA et al.** *Organometallics,* 2014, vol. 33, 2160-2171
- **GRELA et al.** *RSC Adv.,* 2016, vol. 6, 77013-77019
- **HOVEYDY et al.** *J. Am. Chem. Soc.,* 2002, vol. 124, 4954-4955
- **GRUBBS et al.** *J. Am. Chem. Soc.,* 2011, vol. 133, 8525-8527
- **GRUBBS et al.** *J. Am. Chem. Soc.,* 2012, vol. 134, 693-699
- **GRUBBS et al.** *Organometallics,* 2015, vol. 34, 2858-2869
- **Y. KOBAYASHI et al.** Highly Activated Second-Generation Grubbs-Hoveyda Catalyst Driven by Intramolecular Steric Strain. *Synlett,* 13 July 2016, vol. 27 (16), 2352-2356
- **MA et al.** *Org. Lett.,* 2003, vol. 5, 3317-3319
- *Org. Synth.,* 1960, vol. 40, 5
- **POWELL et al.** *Org. Lett.,* 2004, vol. 6, 4069-4072
- *Organometallics,* 2006, vol. 25, 5740-5745